(19) Europäisches Patentamt
European Patent Office
Office européen des brevets

(11) EP 2 229 935 A1

(12) **EUROPEAN PATENT APPLICATION**

(43) Date of publication:
**22.09.2010 Bulletin 2010/38**

(21) Application number: **08006502.2**

(22) Date of filing: **03.07.1998**

(51) Int Cl.:
***A61K 9/00*** *(2006.01)* ***C07D 233/24*** *(2006.01)*
***C07D 233/54*** *(2006.01)* ***C07D 209/16*** *(2006.01)*
***C07D 211/88*** *(2006.01)* ***C07D 401/06*** *(2006.01)*
***C07D 209/26*** *(2006.01)* ***C07D 209/18*** *(2006.01)*
***C07D 403/06*** *(2006.01)*

(84) Designated Contracting States:
**AT BE CH CY DE DK ES FI FR GB GR IE IT LI LU MC NL PT SE**

(30) Priority: **04.07.1997 RU 97111091**

(62) Document number(s) of the earlier application(s) in accordance with Art. 76 EPC:
**98935447.7 / 1 020 179**

(27) Previously filed application:
**03.07.1998 PCT/RU98/00215**

(71) Applicant: **Nebolsin, Vladimir Evgenievich**
**Sarov, 607190 (RU)**

(72) Inventors:
- **Nebolsin, Vladimir Evgenievich**
  **Sarov 607190 (RU)**
- **Zheltukhina, Galina Alexadrovna**
  **Moscow, 129344 (RU)**
- **Estigneeva, Rima Porfirievna**
  **Moscow, 11921 (RU)**

(74) Representative: **Srinivasan, Ravi Chandran**
**J.A. Kemp & Co.**
**14 South Square**
**Gray's Inn**
**London WC1R 5JJ (GB)**

Remarks:
This application was filed on 31-03-2008 as a divisional application to the application mentioned under INID code 62.

(54) **Peptide derivatives or pharmaceutically acceptable salts thereof, method for producing the same, use of said derivatives and pharmaceutical composition**

(57) Derivatives of peptides of general formula

$$R_1\text{-}(CH_2)_n\text{-}CONH\text{-}\underset{\displaystyle R_2}{\underset{|}{CH}}\text{-}(CH_2)_m\text{-}R_3 \text{ (I)},$$

or pharmaceutically acceptable salts thereof, their use as the agents possessing antioxidant, antiasthmatic, antihypoxic, antiinflammatory, antiviral, antibacterial, lipid-regulating, antitumor, antimetastatic, glucose lowering, adaptogenic and the other kinds of therapeutic effects, a method to produce them, a pharmaceutical composition or a cosmetic agent comprising as an active agent a peptide derivative of formula (I) or pharmaceutically or cosmetically acceptable salts thereof as well as a method of therapy or prevention of diseases.

EP 2 229 935 A1

## Description

The present invention relates to the field of bioorganic chemistry and, in particular, it concerns novel dipeptide and pseudopeptide compounds comprising a heterocyclic, for instance, imidazole or indole group, a method to produce these and the known compounds of similar structure, as well as their use in medicine as potential therapeutic agents.

Prior art

The substances of peptide nature are known to possess a high biological activity. Different action aspects of these compounds are disclosed in the literature: on the immune system, on histamine release from rat peritoneal mast cells and human basophils [Marone G., Columbo N., Soppeisa L. et al., J. Immunol. (1984) Vol. 133, No 3, pp. 1542-1546; Wise J., Wojtecka-Lukasik E., Maslinski S., Agents Actions (1986) Vol. 18, No 1-2, pp. 262-265], on production and catabolism of prostaglandin $E_2$ [Duchateau J., Bolla K., Med. Oncol. and Tumor Pharmacoter. (1989) Vol. 6, No 6, pp. 19-23], on the immediate reaction development [8. Muller E., Sonnenschein B., Naturamed. (1989), Vol. 4, No 4, pp. 34-38].

Hypoxia is a common link in the pathogenesis of a broad circle of pathological conditions (stress, physical load, radiation, allergic diseases, atherosclerosis and accompanying diseases., hepatic lesions of different etiology, diabetes mellitus and others). It is known that in hypoxia, lipid peroxidation (LP) rises and cytochrome P-450 content and the activity of P-450-dependent enzymes drop [Proulx M., Dusouich P., J. Pharm. Pharmacol. (1995), Vol. 47, Iss 5, pp. 392-397; Barakat M., du Souich P., J. Pharm. Pharmacol. (1996), Vol. 48, pp. 906-910]. At the same time, the activity of superoxide-desmutase rises and hepatic glutathione content and the activity of glutathione-peroxidase drop. These changes result in increased amount of the reactive oxygen radicals which can cause damage of the membrane-bound enzymes, in particular, the P-450 cytochrome system enzymes [Proulx M., Dusouich P., J. Pharm. Pharmacol. (1995), Vol. 47, Iss 5, pp. 392-397]. LP is an important physiological process continuously occuring in the cell membrane and normally playing an important role in the life activity of cells, e.g. during the synthesis of prostaglandins as well as in phagocytosis {Sokolov E.I. Diabetes mellitus and atherosclerosis. Moscow, Nauka publishers (1996), 405 pp.]. However, at the background of the developed tissue hypoxia, this process becomes poorly controllable and there is formed a great amount of free-radical compounds which have no time for neutralization. Acidosis development is accompanied by the induction of cytochrome P-450 2E1 which exhibits a pronounced activity when lipid peroxides are formed that still to a greater extent promotes rise in LP [Bestervelt L.L., Vaz A.D.N., Coon M.J., Proc. Natl. Acad. Sci. U.S.A. (1995), Vol. 92, Iss. 9, pp. 3764-3768]. Normalization of LP can serve as an important criterion in therapy of said diseases.

It is known that change in the hepatic cytochrome P-450 system condition is closely related to its antioxidant function. Study of this problem has an applied character since in many diseases (atherosclerosis, liver cirrhosis, hepatitis, alcohol abuse and others) disbalance of lipid metabolism occurs with sequent rise in lipid peroxidation (LP).

The development of experimental allergic reactions was shown to occur against the background of decrease in terminal oxigenase content and destabilized condition of the hepatic monooxigenase system components assessed by the renewal ability of $B_5$ and P-450 cytochromes [Krzhechkovskaya V.V., Meltsev G.Yu., Marokko I.N., The 4th All-Union Symposium on Medical Enzymology (1983), p. 137]. Change in the content and ratio of hepatic P450B and P450L cytochrome groups, correlates with change in Hexenal sleep duration, in hormonal status and in the severety of anaphylactic shock in guinea-pigs [Marokko I.N., Krzhechkovskaya V.V., Malikova N.A., Izotov M.V., Benediktova S.A., Spiridonova S.M., Bulletin of Experimental Biology and Medicine (1991) No 8, pp. 200-202]. Activation of the P-450 cytochrome system enzymes with Phenobarbital type inductors, results in the severety alleviation of experimental allergic reaction signs [Marokko I.N., Krzhechkovskaya V.V., Malikova N.A., Izotov M.V., Benediktova S.A., Spiridonova S.M., Bulletin of Experimental Biology and Medicine (1991) No 8, pp. 200-202]. At the same time, when sensitized animals are administered Methyrapone, an inhibitor of the P-450 cytochrome system and cortisol synthesis, increase in bronchospasm is observed [Fornhem C., Kumlin M., Lundberg J.M., Alving K., Eur. Resp. J. (1995), Vol. 8, Iss. 7, pp. 1100-1109; Fornhem C., Lundberg J.M., Alving K., Eur. Resp. J. (1995), Vol. 8, Iss. 6, pp. 928-937]. Sensitization state in humans and experimental animals is accompanied by drop in blood and urine level of glucocorticoids. Normalization of adrenocortical hormone levels results in improvement of clinical disease picture in humans and in decreased severety of allergic reaction signs in animals [Marokko I.N., Krzhechkovskaya V.V., Malikova N.A., Proceedings of the All-Union Conference "P-450 Cytochrome and Modification of Macromolecules", Yalta (1989) p. 339; Macharadze D.Sh, Marokko I.N., Balabolkin I.I., Yukhtina N.V., Malikova N.A., Pediatry (1994) No 3, pp. 9-12], and decrease in their level results in aggravation of allergic reaction signs [Fornhem C., Kumlin M., Lundberg J.M., Alving K., Eur. Resp. J. (1995) Vol. 8, Iss. 7, pp. 1100-1109; Fornhem C., Lundberg J.M., Alving K., Eur. Resp. J. (1995) Vol. 8, Iss. 6, pp. 928-937].

Arachidonic acid (AA) metabolites being formed in the P-450 cytochrome system, possess a number of important biological effects, in particular, vasodilating and bronchiolytic effects [Knickle L.C., Bend J.R., J. Moll. Pharmacol 91994), Vol. 45, Iss. 6, pp. 1273-1280; Quiroga J., Prieto J., Phamacol. Therap. (1993), Vol. 58, Iss. 1, pp. 67-91; Ma Y.H., Gebremedich D., Schwartzman M.L., et al., Circulation Research (1993), Vol. 72, Iss. 1, pp. 126-136], cytoprotective

action [Quiroga J., Prieto J., Pharmacol. Therap. (1993), Vol. 58, Iss. 1, pp. 67-91], they potentiate prostaglandin $E_2$ synthesis [Caroll M.A., Balazy M., Margiotta P., Falck J.R., Mcgiff J.C., J. Biol. Chem. (1993), Vol. 268, Iss. 17, pp. 12260-12266; Sakairi Y., Jacobson H.R., Noland T.D., Capdevilla J.H., Falck J.R., Breyer M.D., Amer. J. Physiol-Renal. Fl. Elect. (1995), Vol. 37, Iss. 5, pp. F931-F939] and other effects, vasodilating effect of AA being potentiated by administration of phenobarbital, which is a known hepatic monoamine oxygenase system inductor [Oyekan A.O., Eur. J. Pharmacol. (1995), Vol. 277, Iss. 2-3, pp. 123-132]. At the same time, the P-450-dependent AA metabolite 5,6-epoxyeucozatriene acid (5,6-EET) potentiates $PGE_2$ synthesis and secretion [Sakairi Y., Jacobson H.R., Noland T.D., Capdevila J.H., Falck J.R., Breyer M.D., Amer. J. Physiol-Renal. Fl. Elect. (1995), Vol. 37, Iss. 5. pp. F931-F939]. On one hand, $PGE_2$ inhibits anaphylactogenic surge of histamine and the other allergy and inflammation mediators from the mast cells. On the other hand, histamine potentiates $PGE_2$ synthesis that is considered as one of the feedback mechanisms in allergic and inflammatory reactions.

Of a special importance in the development of allergic diseases is a change in the pathochemical stage course of allergic reactions which to a great extent is determined by the condition of the first order target cells of allergy (basophils and mast cells), the important feature of which is their ability to accumulate and to synthesize biologically active compounds, in particular, histamine. In IgE and/or IgG-mediated response to antigen, just these cells participate in the secretion of active substances and determine the pathochemical phase course and manifestation degree of allergy clinical picture [Parker Ch.V. Mediators: Release and Functions. In: Immunology ed. by U. Pole, Moscow, Mir publishers (1989) Vol. 3, pp. 170-247; Chakravarty N.K. In: The mast cell: Its role in health and disease. Ed. J Pepys (1979) pp. 38-46]. The importance of examining histamine secretion by mast cells is also determined by the fact that in mast cells transglutaminase (EC 2.3.2.13) is present, i.e. the enzyme synthesizing protein-bound gamma-glutamyl histamine, which is a close analogue of the suggested compounds. Stimulation of mast cells was shown to rise the activity of transglutaminase and the content of protein-bound gamma-glutamyl histamine in mast cells [Fesus L., Szucs E., Barrett K. et al., J. Biol. C. Chem. (1995),No 25, pp. 13771-13778].

It was demonstrated that in hepatic diseases, e.g. hepatitis and cirrhosis, the hepatic P-450 cytochrome system functional activity decreases and LP rises [Imaoka S., Sugiyama T., Taniguchi N., Funae Y., Carcinogenesis (1993), Vol. 14, Iss. 1, pp. 117-121; Debinski H.S., Lee C.S., Danks J.A., Mackenzie P.I., Desmond P.V., Gastroenterology (1995), Vol. 108, Iss. 5, pp. 1464-1469]. Changes in hepatic structure and function which are characteristic of the given pathology, are noted in experimental animals administered carbon quadrichloride ($CCl_4$). The effect of this substance is connected with the damage of the cellular membrane structures caused by elevated LP that is one of the hepatic P450 cytochrome system enzyme inactivation [Kapil A., Koul I.B., Suri O.P. Phytother. Res. (1995), Vol. 9, Iss. 3, pp. 189-193].

Change in lipid metabolism in atherosclerosis, is interconnected with change in the P450 cytochrome system condition [Wolfgang GHI, Robertson DG, Welty DF, Metz AL. Fund. Appl. Toxicol. (1995), Vol. 26, Iss. 2, pp. 272-281; Remaley A.T., Schumacher U.K., Amouzadeh H.R., Brever H.B., Hoeg J.M. J. Lipid Res. (1995), Vol. 36, Iss. 2, pp. 308-314; Stegeman J.J., Hahn M.E., Weisbrod R., Woodin B.R., Joy J.S., Najibi S., Cohen R.A. Mol. Pharmacol. (?), Vol.47, Iss. 2, pp. 296-306]. It is known that ischemic heart disease that occupies the first place among the planet adult population mortality rate, is the most often occuring atherosclerosis manifestation. One of the leading disorders in this disease is lipid metabolism disorder manifested by elevated levels of plasma low density lipoprotein (LDL) and very low density lipoprotein (VLDL) cholesterol which are called "atherogenic" lipoproteins, with simultaneous decrease in "antiatherogenic" high density lipoproteins (HDL).

Change in plasma lipid levels and ratio was shown to reflect their changes in the membrane structures of parenchymatous organs. Membrane composition of cells, e.g. microsomal ones, is directly dependent on the ration of experimental animals [Wade A., Harred W. Feder. Proct. (1976), Vol. 55, pp. 2475-2479]. Cholesterol administration to animals causes its accumulation in cellular memranes decreasing their fluidity that, in its turn, results in change in the functional state of enzymes [Buters J.T.M., Zysset T., Reichen J. Biochem. Pharmacol. (1993), Vol. 46, Iss. 6, pp. 983-991; Reichen J., Buters J.T.M., Sojcic Z., Roos F.J., Experentia (1992), Vol. 48, Iss. 5, pp. 482-486; Tatony Ya.N. Obesety. Pathophysiology, diagnostics, therapy., Warsaw, the Polish Medical publishers (1981) p. 364].

In addition to carbohydrate metabolism disorders in insulin-dependent diabetes mellitus, change in ketone and fatty acid metabolism was demonstrated that results in disbalance of the hepatic microsomal monooxygenase system and still more promotes disorder of the body hormonal status [Shimojo N., Ishizaki T., Imaoka S., Funae Y., Fujii S., Okuda K., Biochem. Pharmacol. (1993), Vol. 46, Iss. 4, pp. 621-627]. In this case, there is observed change in lipid metabolism which is manifested by cholesterol level elevation in the LDL and VLDL fractions and cholesterol decrease in the HDL fraction as well as by a significant rise in lipid peroxidation [Sokolov E.I. Diabetes mellitus and atherosclerosis, Moscow, Nauka publishers (1996), 450 pp.]. Changes in lipid metabolism in diabetes mellitus practically completely coincide with changes in atherosclerosis. Blood glucose level is one of the most important parameters of the pathological process stabilization in diabetes mellitus. Practically all antidiabetic drugs are known to lower blood glucose level not only in diabetes patients but in healthy humans as well.

Phagocytosis is an important link in maintaining the body internal medium. Phagocyting cells are in a large

amount represented in the body. To them in particular belong peripheral blood neutrophils and macrophags (MP). Activation of MP is one of the homeostasis adaptive mechanisms promoting elimination of pathogenes, and antitumor resistance [Immunology. Edited by Pole U., Moscow, Mir publishers (1989), Vol. 3, pp. 202-228; Paltsev M.A., Ivanov A.A. Intercellular ineractions. Moscow, Meditsina publishers (1995), 224 pp.]. These cells are known to carry glucocorticoid receptors on the external membrane [Mayansky A.N., Pikuza O.I. Clinical aspects of phagocytosis. Kazan, MAGARIF publishers (1993), 192 pp.]. Elevation in glucocorticoid hormone levels, is one of neutrophil and MP activation mechanisms.

Surge into blood of the adrenocortical hormones, serves as a signal for neutrophil mobilization from the marrow that is the mechanism providing concordance between the marrow and stress-limiting reactions [Mayansky A.N., Pikuza O.I. Clinical aspects of phagocytosis. Kazan, MAGARIF publishers (1993), 192 pp.].

Active forms of neutrophils and basophils were shown to synthesize and release into extracellular medium such biologically active substances as hydrogen peroxide, peroxidase and others which inactivate leukotrienes that cause bronchospasm in allergic diseases and play an important role in defence against infection and maintaining the body homeostasis [Immunology. Edited by Pole U., Moscow, Mir publishers (1989), Vol. 3, pp. 202-228; Henderson W.R., Jorg A., Klebanoff S.J., J. Immunol. (1982), Vol. 128, No 6, pp. 2609-2613]. Moreover, tranformation of macrophags in consumption of increased cholesterol amount by animals which results in functional activity disorder of macrophags, in particular Kupfer cells [Remaley A.T., Schumacher U.K., Amouzadeh H.R., Brever H.B., Hoeg J.M., J. Lipid Res. (1995), Vol. 36, Iss. 2, pp. 308-314], is considered to be one of the key links in atherosclerotic plaque formation [Sokolov E.I. Diabetes mellitus and atherosclerosis, Moscow, Nauka publishers (1996), 405 pp.].

The formation and development process of metastases is known to be to a great extent determined by intravascular blood coagulation state, and as a result of tumor disease development and the effect of a number of accopmanying factors, blood coagulation system is activated. Administration of anticoagulants was shown to be able to restore the disbalance of blood coagulation and anticoagulation systems [Burov Yu.V., Syrkin A.B., Kinzirsky A.S., Koroleva A.M., Chemical-pharmaceutical production. Review information (1992), issue 11, 42 pp.].

Blood neutrophil activity in severe infections sharply drops that can cause generalization of infection, and rise in the number of activated phagocytes to which perypheral blood neutrophils and peritoneal macrophages belong, and increases the probability of an uncomplicated course of bacterial infection [Mayansky A.N., Pikuza O.I. Clinical aspects of phagocytosis. Kazan, MAGARIF publishers (1993), 192 pp.]. Antimicrobic, antiviral and antitumor defence of the body is directly related to the neutrophil and macrophage activation effects.

There are known the compounds produced using the classical peptide chemistry methods - β-alanylhistamine and γ-aminobutyrylhistamine - which correspond to general formula (I) and possess antioxidant activity in vitro as well as wound healing and anticataract activities [Evstigneeva R.P., Zheltukhina G.A., Ogrel' S.A., Nebol'sin V.E. Synthesis of pseudopeptides based on biogenic amines. Reports of the Russian Sciences Academy (1995), Vol. 345, No 4, pp. 493-495; Mc Caman M.W., Stetzler J., Clark B. Synthesis of γ-Glutamyl dopamine and Other Peptidoamines in the Nervous System of Aplysia californica. J. Neurochem. (1985), Vol. 45, No 6, pp. 1828-1835; Evstigneeva R.P., Zheltukhina G.A., Ageeva E.A., Babizhaev M.A. Lipoperoxidase activity of carnozine and carcinine. Reports of the Academy of Sciences of the USSR (1993), Vol. 333, No 1, pp. 104-106], as well as using enzymatic method - by combining amino acid and histamine in the presence of an enzyme of hydrolase type [Patent Er.-2,701,947 - September 2nd, 1994].

The ethers of N-acyl derivatives of γ-Glutamyl dipeptides produced in the patent [US Patent No 4,568,489 of February 4th, 1986] using DCC-method, are the closest ones by structure to the claimed compounds of dipeptide nature; however, it is known that the DCC-method used to produce said compounds, can result in side reactions by imidazole and indole groups [Schreder E., Lubke K. Peptides. Moscow, Mir publishers (1967), p. 249]. In the application [WO 95/12581. - May 11th, 1995. - Cl CO7D 233/64. - A61K 31/415. - 7/42] pseudopeptide products are disclosed having imidazole group and possessing antioxidant properties. However, no one of the above indicated works discloses the suggested novel imidazol derivatives of dipeptides, the processes of their synthesis and a broad spectrum of their action.

Description of the invention

The present invention provides novel presudopeptides of general formula (I):

$$\begin{array}{c} R_1\text{-}(CH_2)_n\text{-}CONH\text{-}CH\text{-}(CH_2)_m\text{-}R_3 \ (I), \\ | \\ R_2 \end{array}$$

or pharmaceutically acceptable salts thereof, where $R_1$ is a hydrogen atom or $C_1$ - $C_3$ - hydrocarbon radical substituted

for a functional group selected from amino-, $C_1$ - $C_5$ - amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; or $C_1$ - $C_3$ - hydrocarbon radical substituted for indole residue or 5-6 membered saturated or unsaturated cyclic or heterocyclic group, hydrocarbon radical possibly simultaneously comprising amino group, free or substituted for an acyl substitute or ether of carbonic acid; $R_2$ is hydrogen atom or a functional group selected from carboxyl which can be etherified; $R_3$ is indole or methyl and/or hydroxyl derivative thereof, hydroxyl group possibly being acylated, alcylated or aralcylated; 5-6 membered saturated or unsaturated cyclic or heterocyclic groups comprising oxygen, sulfur and/or 1-3 nitrogen atoms or methyl derivatives thereof; hydrogen atom or $C_1$ - $C_3$ - hydrocarbon radical substituted for a functional group selected from amino-, $C_1$ - $C_5$ - amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; n=0-4, m=1-5 provided that when $R_1$ = $-NH_2$, n = 2-3, m=1, $R_2$ =H, then $R_3$ does not signify -4-imidazolyl, -3-(5-Ome-indolyl), -3-(5-OH-indolyl); when $R_1$ =$NH_2$, n=4-5, $R_2$ =-H, then $R_3$ does not signify -4-imidazolyl; when $R_1$ = $-NH_2$, n = 4-5, m=1, $R_2$ =-H, then $R_3$ does not signify -4-imidazolyl; when $R_1$ = $-NH_2$, n = 2-3, m=1, $R_2$ =COOH, then $R_3$ does not signify -4-imidazolyl; when $R_1$ = - $NHCOCH_3$, n = 2, m=1, $R_2$ =H, -COOH, then $R_3$ does not signify -4-imidazolyl; when $R_1$ = -HOOC-CH ($NH_2$) -, n = 2, m=1, $R_2$ =H, -COOH, then $R_3$ does not signify -4-imidazolyl, -3-indolyl, -3-(5-OH-indolyl); when $R_1$ = HOOC-CH ($NH_2$)-, n = 1, m=1, $R_2$ =-COOH, then $R_3$ does not signify -4-imidazolyl; when $R_1$ = $-NH_2$-CH ($[CH_2]_k$ COOH)- n = 0, k=1-2, m=1, $R_2$ =COOH, then $R_3$ does not signify -4-imidazolyl; when $R_1$ = $NH_2$-CH ($[CH_2]_2$ COOH)-, n = 0, m=1, $R_2$ =H, then $R_3$ does not signify -4-imidazolyl; when $R_1$ = $-CH_3$-CONH-CH (COOH)-, n = 1, m=1, $R_2$ =H, then $R_3$ does not signify -4-imidazolyl, -3-indolyl, -3-(5-OH-indolyl); when $R_1$ = $-CH_3$-CONH-CH (COOH)-, n = 2, m=1, $R_2$ =-COOH, then $R_3$ does not signify -4-imidazolyl; when $R_1$ = - $CH_3$CONH-CH ($CH_2$COOH)-, n = 0, m=1, $R_2$ =H, then $R_3$ does not signify -4-imidazolyl, -3-indolyl, -3-(5-OH-indolyl); when $R_1$ =Ry-NH-CH (Rx-$CH_2$-)-, n = o, m=1, $R_2$ =-COOH, where Rx = -4-imidazolyl, -3-indolyl, Ry= Boc-, Z-, H-, then $R_3$ does not signify -4-imidazolyl, -3-indolyl; when $R_1$ = o-, m, -π-$C_5H_4N$-, n = 0, m=1, $R_2$ =H, then $R_3$ does not signify -3-indolyl, -3-(5-Ome-indolyl); when $R_1$ = - COOH, n = 1-2, m=1, $R_2$ =-COOH, then $R_3$ does not signify -3-indolyl; when $R_1$ -CO- = pGlu-, n = 0, m=1, $R_2$ =H, -COOH, $-COOCH_3$, then $R_3$ does not signify -4-imidazolyl; when $R_1$ -CO-= pGlu-, n = 0, m=1, $R_2$ =COOH, then $R_3$ does not signify -3-indolyl; when $R_1$ -CO- = Pro-, n = 0, m=1, $R_2$ =H, then $R_3$ does not signify -4-imidazolyl; when $R_1$ -CO- = Pro-, n = 0, m=1, $R_2$ =COOH, then $R_3$ does not signify -4-imidazolyl, -3-indolyl, possessing antioxidant, antiradical, lipid regulating, hypoglycemic, antiinflammatory, antiaggregate, immunomodulating, antiallergic, antihypoxic, antiatherosclerotic effects as well as ability to induce the P-450 cytochrome system, to modulate the metabolism of arachidonic acid and adrenocortical hormones, to lower the level and antigen-dependent histamine secretion by peritoneal mast cells, to modulate the activity of macrophages, natural killers, the interferon system (of cytokines), as well as activity related to controlling the signs and preventing asthma and pulmonary emphysema, wound healing properties, activity related to controlling the signs of skin lesion, e.g. psoriasis, exzema, varicose veins, activity related to preventing dysfunctional disorders including imminent abortion, dysfunctional uterine bleedings, amenorrhea as well as activity related to controlling the signs of ischemic disease, obesety, diabetes mellitus, hepatoprotective properties, activity related to controlling radiation damages, hepatic lesions including toxic ones, hepatitis, cirrhosis, alcohol abuse, capability to prevent the development and to eliminate the signs of herontological changes including cataract, changes in cutaneous teguments, senile psychoses, Alzheimer and Parkinson diseases, antibacterial and antivirus activity including the activity against HIV infection, antitumor and antimetastatic activity including their combined administration with cytostatic agents and radiotherapy, as well as useful as an adaptogen to overcome stress conditions including hard physical load.

The preferred compounds of present invention are pseudopeptides of general formula

$$R_1\text{-}(CH_2)_n\text{-}CONH\text{-}\underset{\displaystyle R_2}{\underset{|}{CH}}\text{-}(CH_2)_m\text{-}R_3 \quad (I),$$

where $R_1$ =$NH_2$ -, n=2÷5; $R_1$ =HOOC -, n=1÷4; $R_1$ =Rz-OCO -, n=1÷4; Rz= -H or $C_1$-$C_3$ - hydrocarbon radical; $R_1$ =HOOC-$CH_2$ - ($CH_3$) C (Rv) -, Rv= H, OH, $CH_3$; $R_1$ =$C_6H_5CH_2$-OCO-NH-, n=2÷3; $R_1$ =Rx-CONH-, n=2÷5, Rx= $C_1$ - $C_3$ - hydrocarbon radical;; $R_1$ = $CH_3$CONH-CH(COOH) -, n=1÷2; $R_1$ = $CH_3$CONH-CH($[CH_2]_k$ COOH)-, n=0, k=1÷2; $R_1$ = $NH_2$ -CH($[CH_2]_k$ COOH)-, n=0, k=1÷2; $R_1$ = HOOC-CH($NH_2$) -, n=0, k=1÷2; $R_1$ = HOOC-CH ($NH_2$)-, n=1÷2; $R_1$

= $CH_3$ OOC-CH ($NH_2$)-, n=1÷2; $R_1$ = $(CH_3)_3$C-OCONH-CH ($COOCH_2$-$C_6H_5$)-, n=1÷2; $R_1$ = -4-imidazolyl, -3-indolyl, n=1÷2; $R_1$ = Rb-$CH_2$-CH (NHRy)-, Rb= -4-imidazolyl, -3-indolyl, Ry= Boc-, Z-, H-, n=0; $R_1$ = -$CH_3$, n=3-5; $R_1$ = cyclo-$C_6H_{11}$, n=0; $R_1$ = o,m,π-$C_5H_4$N-, n=0; $R_1$ -CO-= pGlu-, n=0; $R_1$ -CO-= Pro-, homo-Pro-, n=0; $R_2$ =-H, -COOH, -COORz, Rz= -H or $C_1$ - $C_3$ - hydrocarbon radical,

$R_3$= 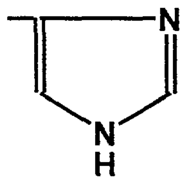 , 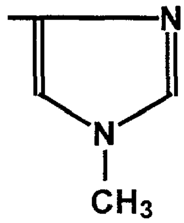 , 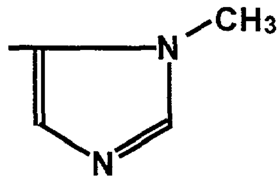 , 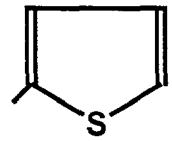

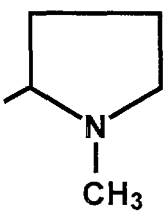 , 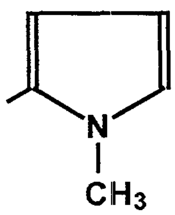 , 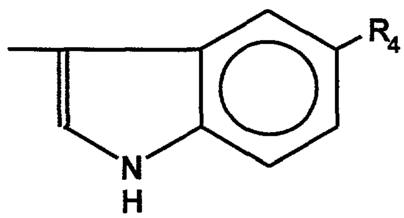 , 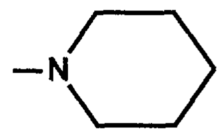 ,

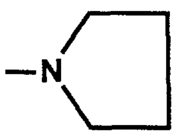 , 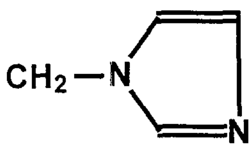 , 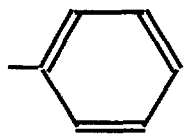 , 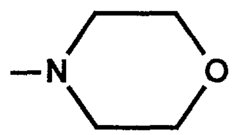 , 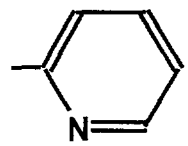

m=1; $R_3$ = -$CH_3$, m=1-5; $R_3$ = $NH_2$, m=1-3; $R_3$ = -COOH, m=0-3; $R_3$ = -CH($NH_2$)-COOH, m=0-2, where $R_4$ = -H, -OH, -$OCH_3$, -$OCH_2$ $C_6H_5$.

Characteristic representatives of the novel dipeptides and pseudopeptides corresponding to general formula (I) are those presented below:

Glutaryl histamine (n=3), Succinyl histamine (n=2)

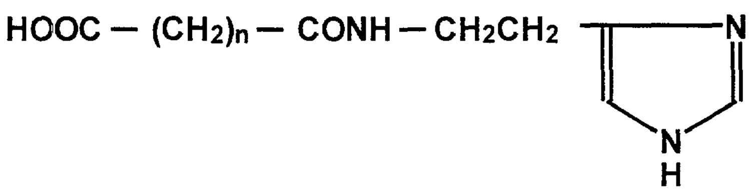

$N^{\alpha}$ -acethyl-L-γ-glutamyl histamine

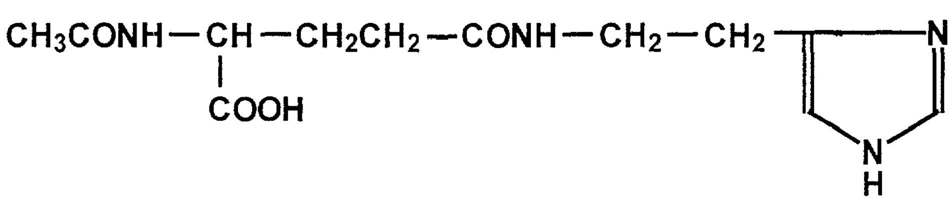

Methyl ether of glutaryl-L-histidine

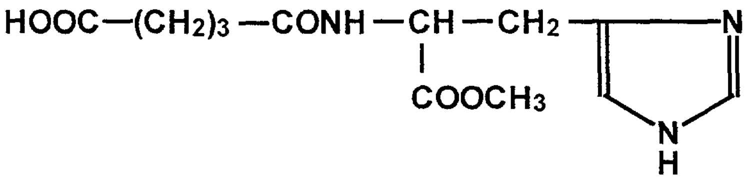

Methyl ether γ-aminobutyryl-L-histidine

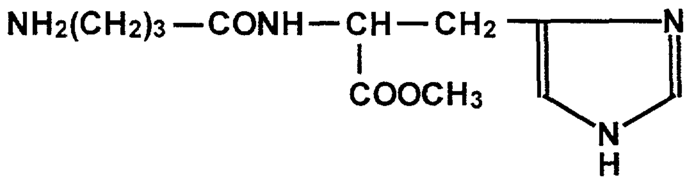

γ -Aminobutyryl tryptamine

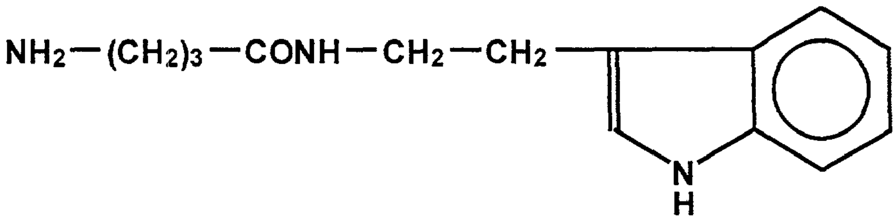

Glutaryl tryptamine

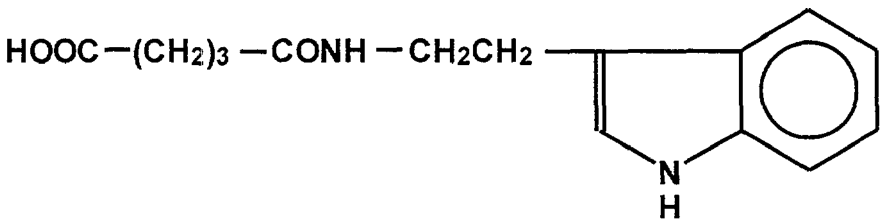

Glutaryl-4-(2-aminoethyl) morpholine

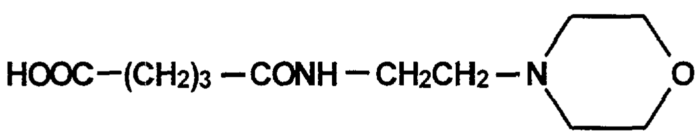

Examples of the compounds of general formula (I) include

$$R_1\text{-}(CH_2)_n\text{-}CONH\text{-}CH\text{-}(CH_2)_m\text{-}R_3\ (I)$$

$$\begin{array}{c} | \\ R_2 \end{array}$$

| № соед. | $R_1$ | n | $R_2$ | m | $R_3$ |
|---|---|---|---|---|---|
| II | **HOOC-** | **2** | **H** | **1** | 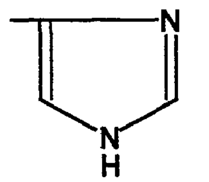 (-4-Im*) |
| III | **HOOC-** | **3** | **H** | **1** | **-4-Im** |
| IV | **HOOC-** | **4** | **H** | **1** | **-4-Im** |
| V | **$HOOCCH_2\text{-}(CH_3)CH\text{-}$** | **1** | **H** | **1** | **-4-Im** |
| VI[a] | **$(CH_3)_3COCONH\text{-}CH\text{-}$**<br>**$COOCH_2C_6H_5$** | **2** | **H** | **1** | **-4-Im** |
| VI | **$CH_3CONHCH\text{-}$**<br>**COOH** | **2** | **H** | **1** | **-4-Im** |
| VII | **HOOC-CH-**<br>**$NH_2$** | **2** | **-COOCH3** | **1** | **-4-Im** |
| VIII | **HOOC-CH-**<br>**$NH_2$** | **2** | **-COOCH3** | **1** | 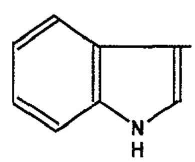 (-3-Ind** |
| IX | HOOC- | **3** | **-COOCH3** | **1** | **-4-Im** |
| X[b] | **$C_6H_5CH_2OCONH\text{-}$** | **3** | **-COOCH3** | 1 | **-4-Im** |
| X | **$NH_2\text{-}$** | **3** | **-COOCH3** | 1 | **-4-Im** |

(continued)

| № соед . | $R_1$ | n | $R_2$ | m | $R_3$ |
|---|---|---|---|---|---|
| XI | $CH_3OOC-CH-$<br>\|<br>$NH_2$ | **2** | **-COOH** | 1 | **-4-Im** |
| XII[a] | **$C_6H_5CH_2OCONH-$** | **3** | **H** | 1 | **-3-Ind** |
| XII | **$NH_2-$** | **3** | **H** | 1 | **-3-Ind** |
| XIII | **$NH_2-$** | **2** | **H** | 1 | **-3-Ind** |
| | **HOOC-** | **3** | **H** | 1 | 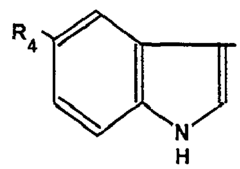 , где |
| XIV | **HOOC-** | **3** | **H** | 1 | **$R_4$=$-OCH_2C_6H_5$** |
| XV | **HOOC-** | **3** | **H** | 1 | **$R_4$=-OH** |
| XVI | **HOOC-** | **3** | **H** | 1 | **$R_4$=$-OCH_3$** |
| XVII | **HOOC-** | **3** | **H** | 1 | **$R_4$=-H** |
| XVIII | **HOOC-** | **3** | **H** | 1 | **$R_4$=$-COCH_3$** |
| XIX | **HOOC-** | **3** | **H** | 1 | 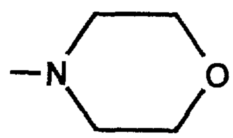 |
| XX | **HOOC-** | **3** | **H** | **1** | 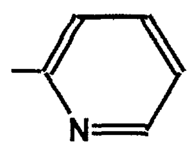 |
| XXI | **HOOC-** | **3** | **H** | **1** | 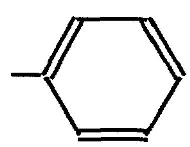 |
| XXII | **HOOC-** | **2** | **H** | **1** | 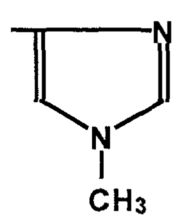 |

(continued)

| № соед. | $R_1$ | n | $R_2$ | m | $R_3$ |
|---|---|---|---|---|---|
| XXIII | **HOOC-** | **3** | **-COOCH3** | **1** | 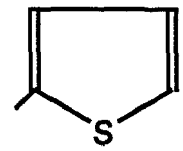 |
| XXIV | **HOOC-** | **3** | **H** | **1** | 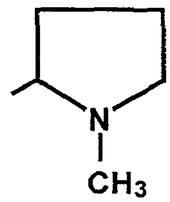 |
| XXV | **HOOC-** | **3** | **H** | **1** | 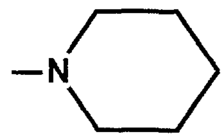 |
| XXVI | **HOOC-** | **3** | **H** | **1** | 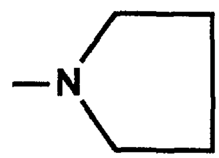 |
| XXVII | **HOOC-** | **2** | **H** | **2** | 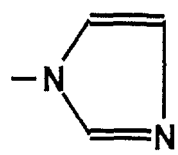 |
| XXVIII | **$C_2H_5OCO$-** | **1** | **H** | **1** | **-4-Im** |
| | | **0** | **H** | **1** | **-4-Im** |
| XXIX | 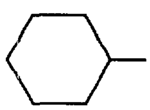 | | | | |
| XXX | 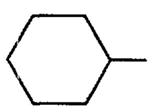 | **0** | **H** | **1** | **-3-Ind** |
| XXXI | **CH3-** | **4** | **H** | **1** | **-3-Ind** |
| XXXII | **CH3-** | **4** | **-COOCH3** | **1** | **-3-Ind** |
| XXXIII | **-4-Im** | **1** | **H** | **5** | **CH3-** |
| XXXIV | **-3-Ind** | **1** | **H** | **2** | **-(NH2) CH- COOH** |
| XXXV | **-3-Ind** | **2** | **H** | **2** | **-NH2** |
| XXXVI | **-CH (NH2)-CH2- 4- Im** | **0** | **H** | **1** | **-COOH** |

(continued)

| № соед . | $R_1$ | n | $R_2$ | m | $R_3$ |
|---|---|---|---|---|---|
| XXXVII | **-NHCO- CH3** | **3** | **H** | **1** | **-4-Im** |
| XXXVIII | **$NH_2$-CH(COOH)-** | **1** | **H** | **1** | **-4-Im** |
| XXXIX | **NH2-CH($CH_2$COOH)-** | **0** | **H** | **1** | **-4-Im** |
| XL [a,b,c] | 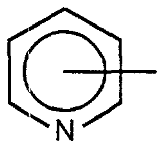 | **0** | **H** | **1** | **-4-Im** |
| XLI | | **0** | **H** | **1** | **-3-Ind** |
| | 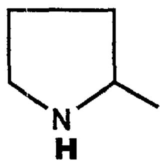 | | | | |
| XLII | 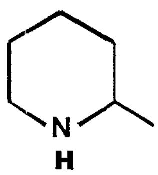 | **0** | **H** | **1** | **-4-Ind** |
| XLIII | 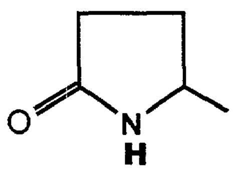 | **0** | **H** | **1** | **-3-Ind** |
| *Im - imidazolyl, **Ind - indolyl. | | | | | |

Further, the present invention relates to the use of the known compounds of formula I, where when $R_1$ = $NH_2$ -, n=2-3, $R_1$ = $CH_3$CONH-CH (COOH)-, n=1, $R_1$ = $CH_3$CONH-CH ($CH_2$ COOH)-, n=0, $R_1$ -CO =pGlu-, n=0, $R_2$ = -H, when $R_1$ = $NH_2$-CH (COOH)-, n=2, $R_2$ = -COOH,

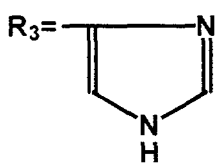
,

m=1, when $R_1$ = $NH_2$ -, n=2-3, $R_1$ = $NH_2$-CH (COOH)-, n=2, $R_2$ =H,

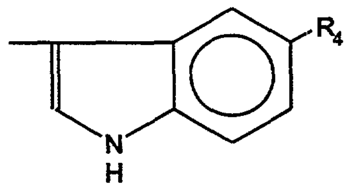

,

m=1, where $R_4$= -H, -OH, $-OCH_3$, $-CH_2C_6H_5$ as the agents possessing antiallergic effect, except for the compound γ-L-glutamylhistamine, wound healing properties, except for the compounds β-alanylhistamine, γ- aminobutyrylhistamine and γ-L-glutamylhistamine, immonomodulating and antiradical effect, except for the compounds β-alanylhistamine and γ- aminobutyrylhistamine, antihypoxic, in vivo antioxidant, lipid regulating, hypoglycemic, antiinflammatory, antiaggregate, antihypoxic, antiatherosclerotic effect as well as the ability to induce the P-450 cytochrome system, to modulate the metabolism of arachidonic acid and adrenocortical hormones, to lower the level and antigen-dependent histamine secretion by peritoneal mast cells, to modulate the activity of macrophages, natural killers, the interferon system (cytokines), as well as the activity related to the control and prevention of asthma and pulmonary emphysema, the activity related to the control of cutaneous lesion signs, e.g., psoriasis, eczema, varicose veins, the activity related to the prevention of dysfunctional disorders including imminent abortion, dysfunctional uterine bleedings, amenorrhea as well as the activity related to the control of ischemic disease signs, obesety, diabetes mellitus, hepatoprotective properties, the activity related to the control of radiation lesions, hepatic lesions including toxic ones, hepatitis, cirrhosis, alcohol abuse, the ability of preventing the development of and to control the signs of herontological changes, including cataract, changes in cutaneous tegumants, senile psychoses, Alzheimer and Parkinson diseases, antibacterial and antiviral activity including activity against HIV infection, antitumor and antimetastatic activity including their combined administration with cytostatic agents and radiotherapy, as well as they are useful as adaptogens to overcome stress conditions including hard physical load.

The most preferred are the known pseudopeptides corresponding to general formula (I), where when $R_1$= $NH_2$ -, n=2-3, $R_1$=$NH_2$ CH-(COOH)-, n=2, $R_1$= $CH_3CONH$-CH (COOH)-, n=1, R1= $CH_3CONH$-CH ($CH_2COOH$)-, n=0, $R_1$,-CO-=pGlu-, n=0, $R_2$=-H -, when $R_1$= $NH_2CH$ (COOH) -, n=1-2, $R_2$= -COOH,

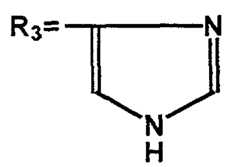

m=1, when $R_1$ =$NH_2$ -, n=2-3, $R_1$ =$NH_2$ -CH (COOH)-, n=2, $R_2$ =H,

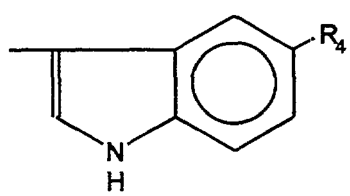

m=1, where $R_4$ = -H, -OH.

The characteristic examples of the known pseudopeptides corresponding to general formula (I), are presented below:

γ -L-, D-glutamylhistamine (XLIV, XLV)

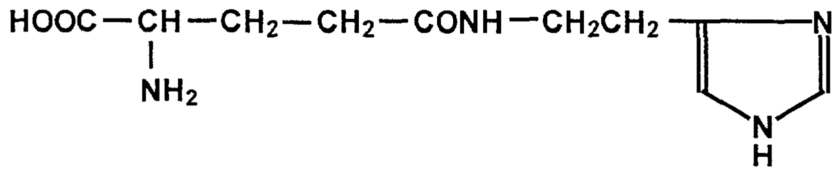

β -Alanylhistamine, n=2; γ-Aminobutyrylhistamine, n=3 (XLVI, XLVII)

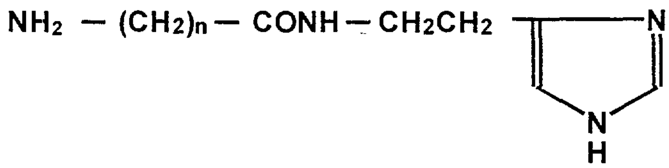

γ -L-glutamyltryptamine (XLVIII)

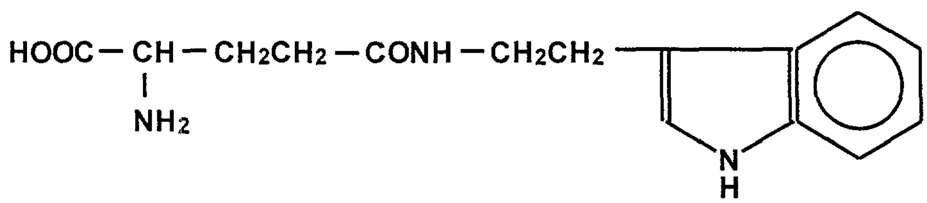

γ -L-glutamylserotonine (XLIX)

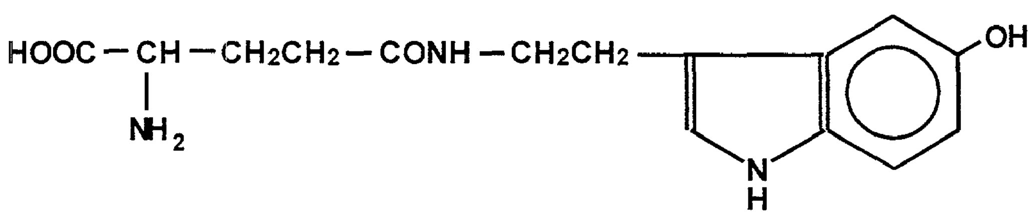

N-Acethyl-β -Aspartylhistamine (L)

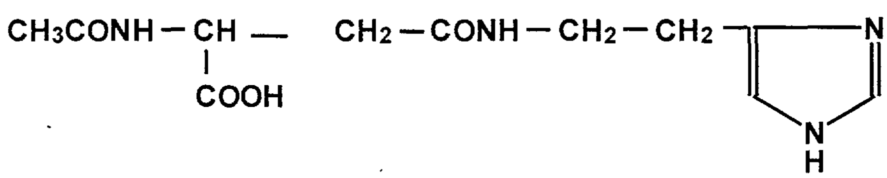

N-Acethyl- α-Aspartylhistamine (LI)

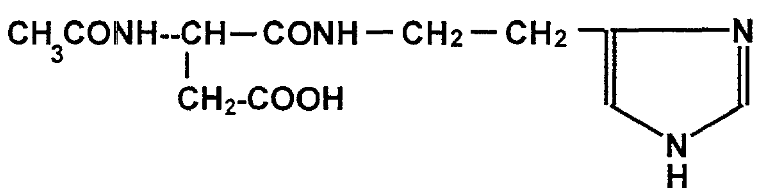

Pyroglutamylhistamine (LII)

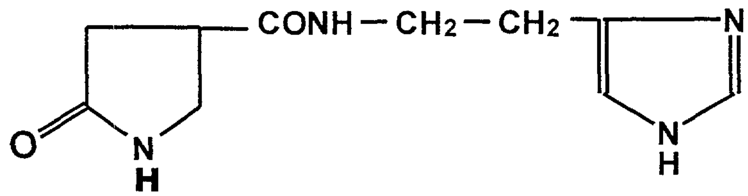

One more subject matter of this invention is a method to produce novel and the known pseudopeptides of general formula I,

$$R_1\text{-}(CH_2)_n\text{-}CONH\text{-}\underset{\displaystyle R_2}{\underset{|}{CH}}\text{-}(CH_2)_m\text{-}R_3 \text{ (I)},$$

or pharmaceutically acceptable salts thereof, where $R_1$ is a hydrogen atom or $C_1$ - $C_3$ - hydrocarbon radical substituted for a functional group selected from amino-, $C_1$ - $C_5$ - amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; or $C_1$ - $C_3$ - hydrocarbon radical substituted for indole residue or 5-6 membered saturated or unsaturated cyclic or heterocyclic group, hydrocarbon radical possibly simultaneously comprising amino group, free or substituted for an acyl substitute or ether of carbonic acid; $R_2$ is hydrogen atom or a functional group selected from carboxyl which can be etherified; $R_3$ is indole or methyl and/or hydroxyl derivative thereof, hydroxyl group possibly being acylated, alkylated or aralkylated; 5-6 membered saturated or unsaturated cyclic or heterocyclic groups comprising oxygen, sulfur and/or 1-3 nitrogen atoms or methyl derivatives thereof; hydrogen atom or $C_1$ - $C_3$ - hydrocarbon radical substituted for a functional group selected from amino-, $C_1$ - $C_5$ - amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; n=0-4, m=1-5 including amino group acylation of the amino compound of general formula $NH_2$ -CH ($R_2$) - $(CH_2)_m$ - $R_3$, activated by carboxylic group with the compound of general formula $R_1$ - $(CH_2)_n$ -COX, where X is the activating group.

In more details, the compounds of general formula (I) are produced by acylation of amine or amino acid amino group witn carbonic, dicarbonic or N-protected amino acid derevative activated by carboxylic group.

Synthesis of dipeptides and pseudopeptides comprising N-amino acylic substitute, is performed according to the classical methods of peptide chemistry advantageously using activated, for instance, N-oxysuccinimide ethers. Preferable embodiment includes the use of pentafluorphenyl ethers as the most active ones from the known ethers. As the activated derivatives of dicarbonic acids, cyclic internal anhydrides therof, are as a rule used.

α -Amino group of the carboxylic or amino component, are protected by different conventionally used groups, preferably tert.-butyloxycarbonylic (Boc-) or benzyloxycarbonylic (Z) protective groups.

α-Carboxylic functions of glutamic and asparagic acids are preferrably protected by benzyl (Bzl-) group and omitine by tert.-butyl group.

In the compounds of general formula (I), carboxylic group of the amino component histidine is in the form of methyl ether or remains unprotected.

Synthesis of the compounds comprising N-amino acylic substitute, is shown on the following scheme 1:

Stage 1. **Ry-NH-A-COO-X+$NH_2$-B-Ry-NH-A-CONH-B**

Stage 2. **$R_y$-NH-A-CONH-B→$NH_2$-A-CONH-B**

Stage 3.

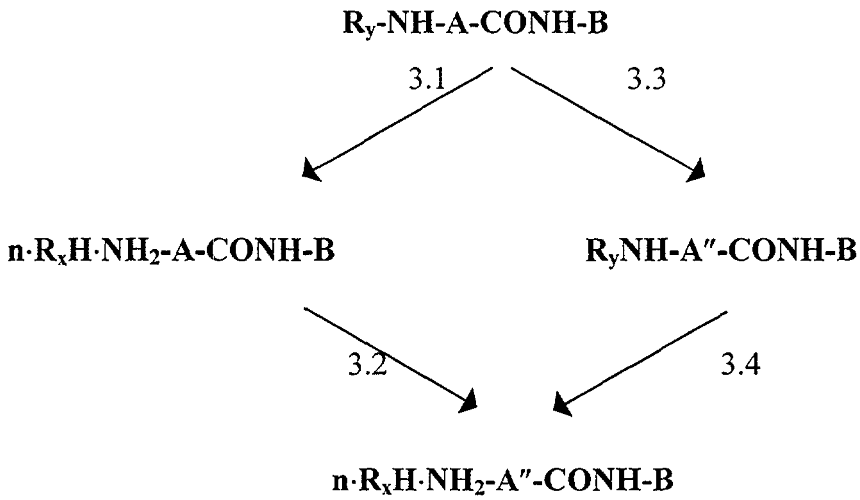


Stage 4. n·$R_x$H·$NH_2$-A"-CONH-B→$NH_2$-A"-CO-NH-B,

where $R_y$ =Boc-, Z-; $NH_2$ -A-CO- = β-Ala-, γ-Abu-, H-L-Glu-OBzl, H-L-Asp-Obzl, H-L-Asp )Obzl)-; $NH_2$ -B=HA, H-His-OH, H-His-OMe, TrpA, H-Trp-OMe, 5-(OMe)TrpA, 5-)OBzl)TrpA; $NH_2$ -A"-CO=H-L-Glu-, H-L-Glu-OMe, n•$R_x$H = HHal (HCl), $CF_3COOH$, n = 1,2.

Stage 1 is as a rule carried out in the medium of dehydrated aprotonic solvent, preferrably dimethyl formamide (DMF) for 18-48 hours at room temperature, except for producing dipeptide Boc-L-Glu(L-His)-OBzl. The latter is produced by action of a three-fold excess of Boc-L-Glu (ONSu)-OBzl on unprotected L-histidine in hydrous-dioxanic medium (1: 1). The advantage of this method consists in simplification of the process due to reduced number of stages (absence of need in introduction and removal of histidine C-protection) and possibility of producing the dipeptide selectively protected by one of two carboxylic groups.

In case of need, cleaving off the protective groups of the intermediate compound Ry -NH-A-CONH-B in accordance with stages 2 and 3.

Stage 2 is carried out by way of catalytic hydrogenolysis only in case if Ry =Z-, $NH_2$-A-CO- = β -Ala-, γ-Abu-.

In cases of need, stage 3 is accomplished in two different modifications when groups Ry -A-CONH-B N -Boc- and Bzl are present in the intermediate compound, namely, for the derivatives of glutamic and aspartic acids. Method 3.1 consists in acidolytic cleaving off $N^\alpha$ -Boc-protection, for instance, by the effect of hydrochloride in organic solvent, advantageously dioxan, methanol or a mixture thereof; or trifluoroacetic acid with subsequent removal of Bzl-group using catalytic hydrogenolysis (3.2). According to method 3.3., hydrogenolysis is first carried out and then, acidolytic cleaving off $N^\alpha$-protection (3.4). As a result of stage, 3 products in the form of respective salts are produced.

In case of need in producing target compounds in the form of free bases, stage 4 is carried out.

In case if a compound does not comprise unprotected carboxylic groups, it can be produced in the form of free base by adding organic ($Et_3N$) or inorganic (NaOH) base with subsequent isolation of this base salt from the target product. In addition, ion-exchange chromatography in accordance with the known technique [Evstigneeva R.P., Zheltukhina G.A., Ogrel' S.A., Nebol'sin V.E. Synthesis of pseudopeptides based on biogenic amines., Reports of the Academy of Sciences of the USSR (1995), Vol. 345, No 4, pp. 493-495], can be used to achieve this goal.

In addition, when it is needed, the compound in the form of a base can be produced in the form of a transition metal salt with formation of a chelate.

The known compound γ-L-Glu-HA [Konishi H., Kakimoto Y. Formation of γ-Glutamylhistamine from histamine in rat brain, J.Neurochem. (1976), Vol. 27, pp. 1461-1463], being an initial one for producing a novel derivative (V), can be produced using the method described in the literature [Mc Caman M.W., Stetzler J., Clark B. Synthesis of γ-Glutamyldopamine and Other Peptidoamines in the Nervous System of Aplysia california, J. Neurochem. (1985), Vol. 45, No 6, pp. 1828-1835]. N-acetyl derivative γ-L-Glu-HA-Ac-γ-L-Glu-HA (V), can be produced in accordance with the method suggested in this invention and presented on scheme 2.

Scheme 2

$$AcONp + H\text{-}\gamma\text{-}L\text{-}Glu\text{-}HA \xrightarrow{DIOXAN/H_2O} Ac\text{-}\gamma\text{-}L\text{-}Glu\text{-}HA$$

Scheme 2 of introducing acetyl group into dipeptide, is advantageous over using N-acetylic derivative of glutamic acid as an initial product for creating a peptide bond, since it reduces the possibilty of racemization. The use of p-nitrophenylacetate is prefferable as compared with acetic anhydride, since its use is not accompanied by side reactions by imidazole group which consist n the formation of an acetylic derivative by imidazole and the respective salt of the latter with the molecule release by acetic acid.

Synthesis of the compounds of general formula (I) comprising a dicarbonic acid residue, can be accomplished using different methods, preferrably, in accordance with scheme 3, where as a C-activated carboxylic compound, its internal cyclic anhydride is used.

Scheme 3

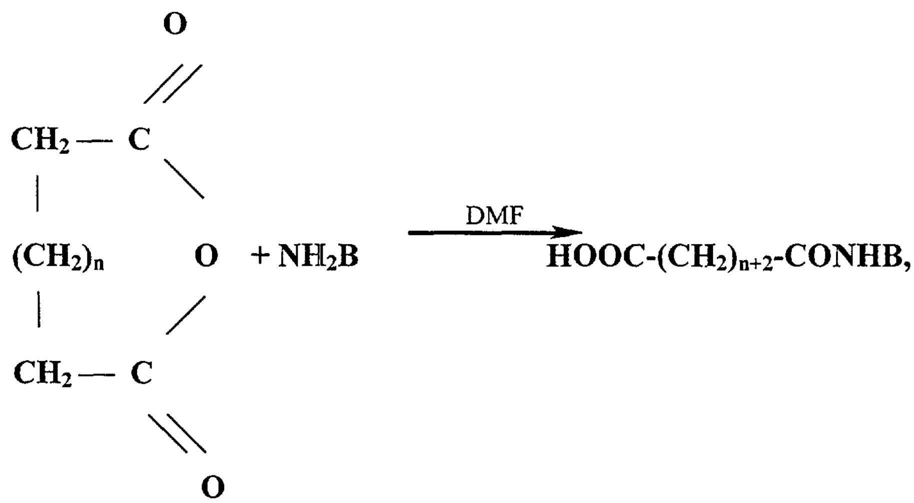


where **n=0÷1, $NH_2B$=HA, H-His-OMe,**

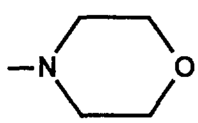
,
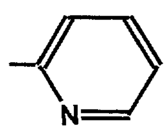
,
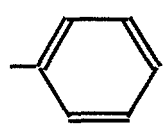
,

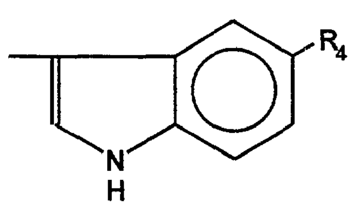
,

where **$R_4$=-H, -OH, $-OCH_3$, $-OCH_2C_6H_5$.**

In case if similar anhydride is not available, e.g. for adipinic acid, synthesis of pseudopeptide can be accoplished

using the DCC-method. In this case, the reaction between dicarbonic acid and DCC is first carried out, the ratio being 2:1 M/M, and then aminocomponent is added (amine or an amino acid derivative).

Synthesis of the compounds of general formula (I) comprising N-acylic residue of fatty acid, is accoplished using chloranhydride method.

Present invention also relates to a pharmaceutic or cosmetic composition possessing antihypoxic, antioxidant, antiradical, lipid-regulating, hypoglycemic, antiaggregate, immunomodulating, wound healing, antiallergic, antiasthmatic, antiviral, antibacterial, antitumor, antimetastatic, adaptogenic effect, the ability to induce the hepatic P-450 cytochrome system, to modulate the metabolism of arachidonic acid and adrenocortical hormones, to lower the level and antigen-dependent histamine secretion by mast cells, to modulate the activity of macrophages, natural killers, the interferon system (of cytokines), as well as to prevent abortions and dysfunctional uterine bleedings, the signs of diabetes mellitus, obesety, ischemic heart disease, stress conditions, hepatitis, cirrhosis, toxic liver lesions, alcohol abuse, radiation injuries, herontological changes, comprising as an active agent the both novel and the known peptide derivatives of general formula (I) or pharmaceutically acceptable salts thereof

$$R_1\text{-}(CH_2)_n\text{-}CONH\text{-}\underset{\displaystyle R_2}{\underset{|}{CH}}\text{-}(CH_2)_m\text{-}R_3 \text{ (I)},$$

where $R_1$ is a hydrogen atom or $C_1$ - $C_3$ - hydrocarbon radical substituted for a functional group selected from amino-, $C_1$ - $C_5$ -amido-, $C_1$ - $C_7$ -urethano- or carboxylic group, carboxylic group possibly being etherified, and amino group can be substituted for an acylic substitute or ether of carbonic acid; or $C_1$ - $C_3$ - hydrocarbonic radical simultaneously substituted for amino- or carboxylic group, carboxylic group possibly being etherified and amino group can be substituted for an acylic substitute or ether of carbonic acid; or $C_1$ - $C_3$ - hydrocarbonic radical substituted for indole residue or 5-6 membered saturated or unsaturated cyclic or heterocyclic group, hydrocarbonic radical possibly simultaneously comprising amino group, free or substituted for an acylic substitute or ether of carbonic acid; $R_2$ is a hydrogen atom or a functional group selected from carboxyl which can be etherified; $R_3$ is indole or methyl and/or hydroxyl derivative thereof, hydroxyl group possibly being acylated, alkylated or aralkylated; 5-6 membered saturated or unsaturated cyclic and heterocyclic groups comprising oxygen, sulfur and/or 1-3 nitrogen atoms or methyl derivatives thereof; hydrogen atom or $C_1$ - $C_3$ - hydrocarbon radical substituted for a functional group selected from amino-, $C_1$ - $C_5$ -amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- and carboxylic group, carboxylic group possibly being etherified and amino group can be substituted for an acyl substitute or ether of carbonic acid; n=0-4, m=1-5, in an efficient amount and pharmaceutically acceptable supplements.

Pharmaceutical compositions of the present invention can be used in the form of pharmaceutical preparations (for instance, in a solid, semisolid and liquid forms), comprising the compounds of present invention as the active ingredients in a mixture with organic or inorganic carrier or excipient, suitable for intramuscular, intravenous, intranasal, oral, sublingual, inhalation and intrarectal administration. The active ingredient can be included into the pharmaceutical composition together with conventionally used non-toxic, pharmaceutically acceptable carriers, suitable for producing solutions, tablets, pellets, capsules, suppositoria, emulsions, suspensions, ointments and any other dosage forms. Water, glucose, lactose, gum arabic, gelatin, starch, magnesium trixylitol, maize starch, urea and other carriers suitable for preparing solid, soft or liquid preparations, can be used as carriers. In this case, stabilizers, thickeners, stains and flavors can be used as supplements.

The active target compound is introduced into a pharmaceutical composition in an amount sufficient to achieve needed effect depending on nosology, course and severety of disease.

In producing a single dosage form, the active ingredient amount used in combination with a carrier, can vary depending on the recipient subjected to therapy, on a particular administration method of a therapeutic agent.

Thus, for instance, in using the compound of present invention in the form of solutions for injections, the active ingredient content in them is 0,01-0,03%. As the substance diluents, 0,9% sodium chloride solution, distilled water, novocaine solution for injections, Ringer solution, glucose solution, can be used. In using the compounds of general formula (I) in the form of tablets and suppositoria, the amount is 3,0-30,0 mg per a single dosage form. For tablets and suppositoria, any pharmaceutically suitable base is used as a pharmaceutical excipient.

To treat and prevent diseases and conditions connected with allergy, inflammation, bronchial asthma, pulmonary emphysema, psoriasis, eczema, varicose veins, dysfunctional disorders, immitent abotrion, uterine bleedings, atherosclerosis, ischemic disease, obesety, diabetes mellitus, infections (viral and bacterial ones), oncology, hepatic lesions (hepatites, cirrhoses), alcohol abuse, toxic, radiation, herontological lesions as well as in cases of need to speed

up wound healing, to exert adaptogenic effect, to induce the hepatic P-450 cytochrome system, to modulate the metabolism of arachidonic acid and adrenocortical hormones, to reduce the level and antigen-dependent histamine secretion by mast cells, to modulate the activity of macrophages, natural killers, interferon system (cytokines), the compounds of formula (I) can be administered orally, locally, parenterally, by inhalations and rectally in the form of single dosage forms comprising standard, non-toxic pharmaceutically acceptable carriers. The term "parenteral administration" as used herein, means subcutaneous, intravenous, intramuscular or intrathoracic injections and infusions.

The compounds of the present invention can be administered once daily at doses 0,03 to 0,5 mg per 1 kg of body weight per a day for 5 to 10 days.

It should be noted that a particular dose for every individual patient will depend on many factors including the activity of the given compound being used, age, body weight, gender, health condition and nutrition regimen of the patient, time and method of the therapeutic agent administration, its excretion rate from the body, a particular combination of therapeutic agents being used as well as severety of disease being treated.

Dosage forms of the present invention are produced using the standard techniques.

It should be noted that the compounds of present invention manifest biological activity at doses being by two to three orders lower than those of the known drugs used for comparison, in practically similar efficacy, and for them, no side effects were detected and they have no contraindications for their use. At the same time, in toxicity testing, $LD_{50}$ was not found since even in administering to animals, e.g. of one of the tested compounds at an oral dose 5000 $\mu$g/kg, and 10,000 $\mu$g /kg parenterally, death of experimental animals was not observed.

Antihypoxic activity of the claimed compounds allows one to use them as antiischemic agents, since it is known that lowered blood supply and hence, oxygen delivery to the specific cardiac region, is the most important factor in the necrosis focus development.

The ability of the compounds of general formula (I) to modulate biochemical and physiological systems, i.e. the hepatic P-450 cytochrome system, hormonal background, lipid peroxidation system (LP) and antiradical activity, arachidonic acid metabolism, spontaneous and antigen-stimulated histamine secretion by peritoneal mast cells, explains their pronounced antiallergic and antiinflammatory activity.

In addition, endogenic rise in the level of precursors and glucocorticoid hormones as affected by the test compounds, can be used in therapy of diseases associated with reduced synthesis of these hormones in the body. Exogenic adminstration of hormonal drugs is known as a rule to result in systemic complications and it is a risk factor of the development of cancer diseases [Parker K.L., Schimmer B.P. The Role of Nuclear Receptors in Steroid-Hormones Production. Seminars in Cancer Biology (1994), Vol. 5, Iss. 5, pp. 317-325] as well as it inhibits the synthesis of respective hormones in the body. In particular, a positive effect of using the compunds of general formula (I) can be predicted in pathological conditions accompanied by decrease in the level of corticosteroids: allergic and inflammatory diseases, hepatic lesions of different etiology, gynecological diseases. The detected elevation in the amount of blood oxyprogesterone, can be of importance in therapy of a number of gynecological diseases, such as infertility, imminent abortion, dysfunctional uterine bleeding etc. Since glucocorticoids and vasodilators are known to be used in therapy of cutaneous manifestations of allergic diseases, as well as a number of cutaneous diseases, including psoriasis, then the test compounds can be used for therapy of these diseases.

Since the suggested compounds possess the ability to alleviate the signs of allergic and inflammatory reactions in local application as well as to increase the synthesis of endogenous antiaggregate agents - prostaglandin $E_2$ and prostacycline (6-keto-$PGF_1$), then their use is possible in the form of ointment in varicose veins development, in the pathogenesis of which such components are present as inflammatory ones, rise in blood coagulation etc.

Aging processes are also known to be associated with oxydative stress accompanied by elevated LP and as a sequence of this, MDA production which interacting with lysine of proteins, forms a lipofuscine-like pigment. This probably speeds up lipofuscinosis that causes irreversible morphological shifts. Lowering MDA level by the compounds of given invention, decreases the probability of the given pigment production that can result in decreased probability of age-dependent disease development. In addition, the results obtained in the work by Emanuel with collegues [Emanuel N.M., Obukhova L.K., Hajdich V.I., Murza L.I., Bunto T.V. Aging Inhibition through Activation of the System of Microsomal Oxidases with Phenobarbital. Reports of the USSR Academy of Sciences (1977(, Vol. 235, No 4, pp. 957-960], increase in avarage lifetime of experimental animals, can serve as confirmation of possibility to use the tested compounds in age-dependent diseases when low doses of Phenobarbital are used. The compounds of general formula (I) cause changes in the hepatic P-450 cytochrome system, similar to those caused by phenobarbital, and their effect on the body can result in decrease in aging rate. The complex of herontological disorders includes such pathological conditions as senile psychoses, cataract, senile changes in cutaneous teguments. The compounds of formula (I) can also be used for therapy of Parkinson disease, cataract, senile changes in cutaneous teguments, one of the pathogenesis link of which is oxidative stress in the nervous tissue (increase in LP).

The compounds of general formula (I) possess a pronounced hepatoprotective effect. This effect of the compounds is explained by the fact that in their administration into the body, a functional reconstruction of many central body systems participating in maintaining homeostasis occurs that allows also to recommend them for treating disorders

caused by the effect on the body of such factors as stress, physical load, ionizing radiation, in preventing side radiotherapy events, for activating reparation processes; in treating hepatic diseases (cirrhoses, hepatites of different etiology); in treating the conditions associated with intoxication with the substances activating lipid peroxidation, for instance, in alcohol abuse; as prophylactic drugs in industries connected with production of chemical reagents and in exposures to toxic substances.

The activity of the compounds of general formula (I) normalizing the lipid metabolism parameters as well as their antihypoxic and antiischemic efficacy allows one to propose them for prophylaxis and therapy of the diseases connected with lipid metabolism disorders characterized by elevation in the level of triglycerides, total cholesterol (CS), low and very low density lipoprotein cholesterol (LDL and VLDL) and in decrease in the level of high density lipoprotein cholesterol, such as atherosclerosis, obesety, ischemic heart and cerebral disease, myocardial infarction, stroke, which serve as risk factor of diabetes mellitus manifestation and thrombogenesis.

The homeostasis stabilizing effect of the compounds of general formula (I) serves as the basis for recommending them for therapy of diabetes mellitus.

The compounds of given invention significantly inhibit the growth of a transplantable tumor and the process of its metastasizing, they elevate resistance of animals to microbial and viral infections.

Inhibition of tumor growth and metastasizing processes is connected with a number of bioigical effects of the compounds of general formula (I): with change in the P-450 cytochrome system condition, with change in hormonal status, with their antioxidant and antiradical properties, with increase in the activity of phagocytes (neutrophils and peritoneal macrophages), natural killer cells and production of interferons (cytokines) under stress effect, that allows to use them in therapy of cancer diseases, since the all the systems listed above, are known to participate in maintaining the body homeostasis and to undergo significant changes in given diseases. Decrease in 12-HETE production can be one more explanation of antimetastatic effect of the tested compounds, since this arachidonic acid metabolite is known to potentiate the process of metastasizing [Honn K.V., Tang D.G., Gao X., Butovich I.A., Liu B., Timar J., Hagmann W. Cancer and Metastasis Reviews (1994), Vol. 13, Iss. 3-4, pp. 265-396, Ref: 195]. Increase in prostacycline/thromboxane ratio also can play a certain role in the antimetastatic effect of the compounds. In addition, the compounds rise antitumor activity of cyclophosphane, which is widely used in the therapy practice of cancer patients. An important characteristics of the compounds is that they exhibit therapeutic properties in oral administration. In administering a dosage form, therapeutic activity is comparable with the use of the substance. The fact of decrease in the number of metastases is of a great practical importance since the rate of this process course is extremely important and its inhibition results in a more favorable prognosis in cancerogenesis.

It was established that normalization of the above mentioned parameters and especially rise in the number of active peritoneal macrophages in animals with administering the compounds of general formula (I), as well as increase in the number of active NK cells actively participating in the immune body state maintenance under stress effect and rise in γ-interferon production which inhibits in vitro proliferation of the cells transformed by virus, is an explanation of increased resistance of animals to microbial and viral infections. The protective effect of the compounds in cytodestructive effect of the human immunodefficiency virus, should be especially emphasized. Antiviral effect of the compounds can also be connected with lipid peroxidation normalization.

The ability of the compounds of general formula (I) to potentiate mitogen-stimulated blasttransformation of human lymphocytes, can serve as the basis for using the tested compoundes in lesions of cutaneous teguments, in particular, to speed up wound healing [Koyama, Masayoshi, Takahashi, Mikiko, Yanagawa, Masayoshi EP 95-107298 950513; JP 94-115161 940527. Producation of L-lysyl-glycyl-L-histidine and therapeutic agent for wound healing containing the same. Eur. Pat. Appl., 5 pp.].

Thus, the tested compounds of general formula (I) can be recommended for therapy of cancer diseases, bacterial and viral infections and that should be especially emphasized, for therapy of HIV infection.

A broad action spectrum of the compounds of general formula (I), can be partially explained by the fact that these compounds act on the central body systems which stabilize homeostasis, for instance, on the P-450 cytochrome system, LP and phagocytosis system. Change in the activity of one system in the live organism is known to be followed by changes in the other systems, and these changes attain a cascade character. With adminitering the substances of general formula (I) into the body, normalization of many its functions occurs that allows one to atribute them to adaptogens.

The subject matter of the present invention is also a method of therapy and prevention of diseases of warm-blooded animals, the diseases including: stress conditions, allergic diseases of immediate and delayed type - bronchial asthma, food allergy, anaphylactic shock, allergic eczemas as well as psoriasis, atherosclerosis, ischemic disease (of the heart and brain), obesety, type 1 and 2 diabetes mellitus, alcohol abuse, alcohol intoxication, varicose veins and prevention of thrombogenesis; hepatitis, hepatic cirrhosis; toxic hepatic lesions; nervous system diseases, Parkinson disease; herontological diseases: cataract, senile change in cutaneous teguments; radiation injury, elimination of radiotherapy consequences; viral and bacterial infections, in particular, HIV infection; cancer diseases; hard physical load; to speed up wound healing, comprising administration of novel or known compounds of general formula (I) in an efficient amount. The compounds of general formula (I) can be used in medicine and veterinary for therapy and prevention of

the above listed diseases.

Further, specific examples are given to illustrate the present invention which should not be considered as any limitation of the scope of the given invention.

The following abbreviations are used in the examples presented below:

| | |
|---|---|
| AAS - | active anaphylactic shock |
| AC - | arachidonic acid |
| ALT - | alanine aminotransferase |
| AN - | activated neutrophils |
| AsA - | ascorbinic acid |
| AST - | aspartate aminotransferase |
| AOF - | active oxygen forms |
| HPLC - | high performance liquid chromatography |
| DTH - | delayed type hypersensitivity |
| ITH - | immediate type hypersensitivity |
| HS - | hexenal sleep |
| DMF - | dimethyl formamide |
| MII - | metastasizing inhibiton index |
| LD - | lethal dose |
| LA - | linolic acid |
| LO - | lipoxygenase route |
| HDL - | high density lipoproteins |
| LDL - | low density lipoproteins |
| VLDL - | very low density lipoproteins |
| LT - | leukotrienes |
| MDA - | malonic dialdehyde |
| MP - | macrophages |
| NR - | neutral red |
| NK - | natural killer cells |
| NBT - | nitroblue tetrazolium (formazan) |
| OVA - | chicken ovalbumin |
| PG - | prostaglandins |
| PCA - | passive cutaneous anaphylaxis |
| PM - | peritoneal macrophages |
| LP - | lipid peroxidation |
| MLT - | mean lifetime |
| FC - | free cholesterol |
| TBA - | thiobarbituric acid |
| TG - | triglycerides |
| TGlu - | transglutaminase |
| MC - | mast cells |
| TMS - | tetramethylsilane |
| TCA - | trichloracetic acid |
| TX - | thromboxane |
| TLC - | thin layer chromatography |
| TCD - | tissue cytopathic dose |
| P - | phospholipids |
| PB - | phenobarbital |
| PG - | phagocytes |
| NS - | normal saline |
| CL - | chemiluminescence |
| CS - | cholesterol |
| CNS - | central nervous system |
| CO - | cyclooxygenase route |
| EDTA - | ethylenediamide-tetraacetate |
| NMR - | nuclear magnetic resonance |
| $Bu^tOH$ - | tert-butanol |
| But - | tert-butyl |

| | |
|---|---|
| Bzl - | benzyl |
| Boc - | tert-butyloxycarbonyl |
| DCC - N, N' - | dicyclohexylcarbodiimide |
| DCU - N, N' - | dicyclohexylurea |
| DMF - N, N' - | dimethylformamide |
| $Et_3N$ - | triethylamine |
| EtOAc - | ethylacetate |
| EtOH - | ethanol |
| EET - | epoxyeucozatrienic acid |
| GA - | glutaric acid |
| Glu - | glutamic acid |
| F - | cortisol |
| HA - | histamine |
| HETE - | (S)-hydroxy-6,8,11,14-eucozatrienic acid |
| 5-HT - | 5-hydroxytryptamine (serotonin) |
| Ind - | indol |
| Im - | imidazol |
| MeCN - | acetonitryl |
| MeOH - | methanol |
| $OBu^t$ - | tert-butylic ether |
| ONSu - | N-oxysuccinimidyl |
| OPfp - | pentafluor phenyl |
| Py - | pyridine |
| P-450B - | a group of P-450 cytochromes with active center, oriented into cytosol |
| P-450L - | a group of P-450 cytochromes with active center, oriented into phospholipid membrane |
| TrpA - | tryptamine |
| TFA - | trifluoracetic acid |
| Z - | benzyloxycarbonyl |

The derivatives of glutamic, aspargic acid, histidine, tryptophane, ornitine, used in synthesis, are the L-row derivatives and use of D-derivatives is especially indicated. Individuality of the produced compounds is checked using TLC method on the Silufol plates of the Kavalier company, UV-254 (Czechoslovakia) in the systems of solvents : chorophorm-methanol 9:1 (1), chloroform-methanol 8:2 (2), n-butanol-acetic acid-water 4:1:5 (3); on the Kieselgel plates of the Merck company in the systems of solvents chloroform-methanol-25% hydrous ammonia 5:3:1 (4); on the Silufol plates: chloroform-methanol 9,3:0,7 (5); chloroform-methanol-25% hydrous ammonia 5:3:0,5 (6), chloroform-methanol 8,5:1,5 (7); isopropanol-water-25% hydrous ammonia 6:3:1 (8), chloroform (9), on the Kieselgel plates of the Merck company in the systems of solvents isopropanol-water-25% hydrous ammonia 6:1:3 (10).

Chromatograms are developed with chlorotolidine solution, Pauly and Erlich reagents, ninhydrine and in UV light.

The melting temperature of compounds is determined using the Boetius instrument (Germany).

The $^1$H-NMR spectra are taken on the Brucker WM-250 apparatus (Germany) and the Varian XL-400 (Japan) with TMS as an internal standard.

Mass-spectrometry is carried out on the MSBCh instrument (Sumy, Ukraine) using the plasma-desorption ionization method with nuclear fragments of californium 252.

Analytical reversed phase HPLC is carried out under the conditions (1): the MPS-270 C-18 column (4,0 x 250 mm), 10 μm, elution with 4% acetonitryl in water comprising 0,1% TFA; (2): the same column, elution with gradient from 10% to 50% of B phase in A phase for 20 minutes; A phase - 0,1% TFA in water, B phase - 0,09% TFA in the mixture of acetonitryl and water 60:40; (3): the MPS-300 C-18T column (4,0 x 250 mm), 10 μm, elution with gradient from 0% to 40% of B phase for 20 minutes. A phase - 0,1% TFA in water, B phase - 0,09% TFA in the mixture of acetonitryl and water 60:40; (4) the MPS-270 C-18 column (4,0 x 250 mm), 10 μm, elution with 0,1M $Na_2HPO_4$, pH 2,3; (5) : the same column, elution with 0,1M $Na_2HPO_4$, pH 2,7; (6) the Diasorb 130C18T column (4,0 x 150 mm), 7 μm, elution with gradient from 0% to 42% acetonitryl in 0,1% TFA; (7) : the MPS-300 C-18T column (4,0 x 250 mm), 10 μm, elution with gradient from 0% to 18% acetonitryl in 0.1% TFA for 20 minutes; (8) : the Lichrosorb RP-18 column (4,6 x 250 mm), 5 μm, elution with gradient from 6% to 24% acetonitryl in 0,1% TFA for 20 minutes; (9): the Diasorb 130 C 16T column (4,0 x 150 mm), elution 0,1% TFA; (10) : the Diasorb 130 C 16T column (4,0 x 150 mm), 7 μm, elution with gradient from 0% to 24% acetonitryl in 0,1% TFA for 30 minutes; (11) : the same column, elution with gradient from 3% to 54% acetonitryl in 0,1% TFA for 30 minutes; (12) : the same column, elution with gradient from 0% to 30% acetonitryl in 0,1% TFA for 30 minutes; (13) : the MPS C18T column (4,6 x 250 mm), elution with gradient from 12% to 60% acetonitryl in 0,1%

TFA for 20 minutes; (14) : the MPS-270 column (4,0 x 250 mm), 10 μm, elution with gradient from 0% to 60% acetonitryl in 0,1% TFA for 20 minutes; (15) : the Diasorb 130 C 16T column (4,0 x 150 mm), 7 μm, elution with gradient from 0% to 60% acetonitryl in 0,1% TFA for 30 minutes; (16) : the Diasorb 130 C 16T column (4,0 x 150 mm), 7 μm, elution with gradient from 60% to 100% acetonitryl in 0,1% TFA for 15 minutes.

In all cases, HPLC is carried out at elution rate 1 ml/min with detection at 214 nm. EXAMPLE 1

N-SUCCINYLHISTAMINE (II)

To 0,080 g (0,72 M) histamine solution in 4 ml DMF 0,072 g (0,72 M) succinic anhydride are added while stirring. The reaction mixture is stirred for 1 hour and left for 20 hours at 20˚C. Solvent is removed under vacuum. Oily residue is dissolved in 1,5 ml ethanol, 4 ml dry ether are added, ground and kept for 30 minutes at $4^0$ C. The residue is separated and three times recrystallized from methanol. Yield is 0,075 g (49,2%). $R_f$ 0,41 (4). Melting temperature 153-$155^0$C. HPLC under the conditions (6): one peak, retention time 7,77 minutes. $^1$H-NMR spectrum, ($CD_3OD$), δ, ppm : 2,42 (t, 2H, -$CH_2$CONH), 2.55 (t, 2H, HOOC-$CH_2$), 2.8 (t, 2H, β-$CH_2$-HA), 3.4 (t, 2H, α-$CH_2$-HA), 7.0 (s, 1H, CH-5-Im), 8.0 (s, 1H, CH-Im). Mass-spectr; m/z: $[M+1H]^+$ 212.2.

EXAMPLE 2

N-GLUTARYLHISTAMINE

To 0,366 g (3.3 M) histamine solution in 5 ml DMF 0,376 g (3.3 M) glutaric anhydride are added. The reaction mixture is stirred for 3 hours and left for 20 hours at $20^0$C. White sediment is separated, dried under vacuum and recrystallized. Yield is 0,510 g (70,0%). $R_f$ 0.36 (6), 0.34 (4). Melting temperature 187-$189^0$C. HPLC under the conditions (7): one peak, retention time 14.36 minutes. $^1$H-NMR spectrum, ($D_2O$), δ, ppm : 1,72 (m, 2H, β-$CH_2$), 2.18 (m, 4H, α,γ-$CH_2$), 2.85 (t, 2H, β-$CH_2$-HA), 3.5 (t, 2H, α-$CH_2$-HA), 7.25 (s, 1H, CH-5-Im), 8.5 (s, 1H, CH-2-Im). Mass-spectr.; m/z: $[M+1H]^+$ 226.1.

EXAMPLE 3

N-ADIPINYLHISTAMINE CHLOROHYDRATE (IV).

To the solution of 0,197 g (1.35 M) adipinic acid in 2.5 ml DMF at $0^0$C 0.278 g (1.35 M) DCC are added. The reaction mixture is stirred at $0^0$C for 30 minutes and the solution of 0.150 g (1.35 M) histamine in 1 ml DMF is added and left at $20^0$C for 20 hours. DCU sediment is separated by filtration. To the reaction mixture 10 ml dry ether are added and left for 1 hour at $0^0$C. Oily residue is dissolved in ethanol, and 0.2 ml 4N HCl in dioxane are added. Solvent is removed under vacuum. The residue is washed with ether, dissolved in the chloroform-ethanol mixture 1.5:1 and purified on a column with silicagel 40/100 (22x175 mm). Elution is done with the chloroform-ethanol mixture from 7:3 to 2:8, ethanol, methanol and the methanol-AcOH-$H_2O$ mixture 8:1:0,5. The fractions containing the target substance with $R_f$ 0,25 (6), are united, the solvent is removed under vacuum and dried over $P_2O_5$. Yield is 0,129 g (40%). $R_f$ 0,25 (6). HPLC under the conditions (11): one peak, retention time 3.6 minutes. $^1$H-NMR spectrum, ($CD_3OD$), δ, ppm : 1.6 (m, 4H, β,γ-$CH_2$), 2.3 (m, 4H, α,γ-$CH_2$), 3.0 (t, 2H, β-$CH_2$-HA), 3.55 (t, 2H, α-$CH_2$-HA), 7.25 (s, 1H, CH-5-Im), 8.65 (s, 1H, CH-Im).

EXAMPLE 4

N -ACETHYL-γ-L-GLUTAMYLHISTAMINE (V)

To 0.10 (0.405 M) γ-L- glutamylhistamine 5 ml water are added and stirred to dissolution of basic substance mass. To the reaction mixture 2.5 ml dioxane and 0.073 g (0.405 mM) p-nitrophenyl acetate are added, stirred for 2 hours and left for 18 hours at $20^0$C. The solvent is removed under vacuum at $40^0$C. The residue is dissolved in the minimum amount of methanol and purified using column chromatography on Kieselgel 60, eluted with methanol. The fractions containing the target substance with $R_f$ 0.3 (4), are united, the solvent is removed under vacuum. Colorless glassy substance is produced. Yield is 0.046 g (40.0%). $R_f$ 0.3 (4). HPLC under the conditions (3): one peak, retention time 10.77minutes. $^1$H-NMR spectrum, ($CD_3OD$), δ, ppm : 2.0-2.3 (m, 2H, β,-$CH_2$), 2.19 (s, 3H, $CH_3$CO), 2.45 (t, 2H, γ-$CH_2$), 3.07 (t, 2H, β-$CH_2$-HA), 3.64 (t, 1H, α-$CH_AH_B$-HA), 3.65 (t, 1H, α-$CH_AH_B$-HA), 4.42 (t, 1H, α-CH), 7.42 (d, 1H, CH-5-Im), 8.69 (d, 1H, CH-2-Im).

EXAMPLE 5

5.1. N$^{\alpha}$-TERT-BUTYLOXYCARBONYL-$\alpha$-BENZYL-L-GLUTAMYL-L-HISTIDINE METHYL ETHER (VI$^{a}$)

To the solution of 0,30 g (1.16 M) L-histidine methyl ether dihydrochloride produced by heating to 40$^{0}$C with 4 ml anhydrous methanol, with subsequent cooling to 0$^{0}$C, cold sodium methylate solution produced from 0.053 g mettalic sodium (2.32 M) and 1 ml methanol, is added and left for 20 minutes at 0$^{0}$C, then equal volume of dry ether is added and left for 20 minutes at 0$^{0}$C. Sodium chloride sediment is separated, the solvent from filtrate is removed under vacuum. Residue is dissolved in 3.5 ml DMF and 0.604 g (1.16 mM) Boc-L-Glu (Opfp) - OBzl. The reaction mixture is stirred for 2 hours and left for 20 hours. DMF is removed under vacuum. Oily residue is purified on a column 30x1.6 cm ith silicagel 100/160, eluted with chloroform:methanol mixture (9:1). The fractions containing the target substance, are united, the solvent is removed under vacuum. The target substance is dried over $P_2O_5$. Yield is 0.334 g (55.0%). $R_f$ 0.35 (1). $^{1}$H-NMR spectrum, ($CD_3OD$) : 1.45 (s, 9H, $(CH_3)_3$ C), 2.0 (m, 2H, $\beta$-$CH_2$), 2.3 (t, 2H, $\gamma$ -$CH_2$), 3.0 (d, 2H, $\beta$-$CH_2$-His), 3.7 (s, 3H, $CH_3O$), 4.1 (t, 1H, $\alpha$-CH), 4.55 (t, 1H, $\alpha$-CH-His), 5.15 (m, 2H, $CH_2$ -Bzl), 6.85 (s, 1H, CH-5-Im), 7.35 (m, 5H, $C_6H_5$ -Bzl), 7.6 (s, 1H, CH-2-Im).

5.2. $\gamma$ -L-GLUTAMYL-HISTIDINE METHYL ETHER DIHYDROCHLORIDE (VI)

To the solution of 0,30 g Boc-L-Glu (HisOMe) - Obzl (Vi$^{a}$) in 1 ml MeOH 3 ml 4 n hydrochloride in dioxane. In 30 minutes 5 ml dry ether are added. The solvents are removed under vacuum, dry ether is added and also removed under vacuum. Residue is ground with dry ether. Ether is separated by decantation. White solid substance is dried over $P_2O_5$ under vacuum. 0.25 g 2HCl• L-Glu (HisOMe) - Obzl are produced.

To the solution of 0.140 g of the produced substance in 4.5 ml anhydrous methanol, 0.10 g 10% palladium on activated carbon are added and stirred for 2.5 hours, periodically passing air flow through it. Catalyzer is separated, washed on MeOH filter. Solvent from the united filtrate is removed under vacuum. To residue dry ether is added and also removed under vacuum. The substance is dried under vacuum over $P_2O_5$. Yield is 0,103 g (90,3%). $R_f$ 0.35 (6). HPLC under the conditions (9): one peak, retention time 6.16 minutes. $^{1}$H-NMR spectrum, ($D_2O$), $\delta$, ppm : 2.15 (m, 4H, $\beta$-$CH_2$), 2.55 (t, 2H, $\gamma$-$CH_2$), 3.15 (t, 1H, $\alpha$-CH), 3.75 (s, 3H, $CH_3O$), 4.0 (t, 1H, $\alpha$-CH-His), 4.8 (d, 2H, $\beta$-$CH_2$-His), 7.4 (s, 1H, CH-5-Im), 8.81 (s, 1H, CH-2-Im). Mass-spectr; m/z: 299.1.

EXAMPLE 6

N-GLUTARYL-L-HISTIDINE METHYL ETHER (VII)

To the solution of 0,30 g (1.16 m) L-histidine methyl ether dihydrochloride produced by heating to 40$^{0}$C in 4 ml anhydrous methanol with subsequent cooling to 0$^{0}$C, cold sodium methylate solution produced from 0.053 g (2.32 mM) mettalic sodium and 1 ml methanol are added. The reaction mixture is left for 30 minutes at 0$^{0}$C, then equal volume of dry ether is added and left for 20 hours at 20$^{0}$C. Sodium chloride sediment is separated under vacuum. Residue is dissolved in 3.5 ml DMF and 0.132 g (1.16 mM) glutaraldehyde are added. The reaction mixture is stirred for 2 hours and left for 20 minutes at 20$^{0}$C. The solvent is removed under vacuum. Oily residue is purified on a column (30x1.6 cm) with silicagel 100/160, eluted with methanol. The fractions containing the target substance, are united. The solvent is removed under vacuum, dried over $P_2O_5$ under vacuum. Yield is 0,095 g (28%). $R_f$ 0.43 (10). HPLC under the conditions (8): one peak, retention time 9.95 minutes. $^{1}$H-NMR spectrum, ($D_2O$), $\delta$, ppm : 1.85 (m, 2H, $\beta$-$CH_2$), 2.25 (t, 4H, $\alpha$, $\gamma$-$CH_2$), 3.05 (d, 2H, $\beta$-$CH_2$-His), 3.7 (s, 3H, $CH_3O$), 4.67 (t, 1H, $\alpha$-CH-His), 6.92 (s, 1H, CH-5-Im), 7.72 (s, 1H, CH-2-Im). Mass-spectr; m/z: 284.4.

EXAMPLE 7

7.1. N-BENZYLOXYCARBONYL-$\gamma$-AMINOBUTYRIC ACID PENTAFLUORPHENYL ETHER (VIII$^{a}$)

To 0.60 g (2.53 mM) N-benzyl oxycarbonyl-$\gamma$-aminobutyric acid 9 ml anhydrous ethylacetate are added, stirred and cooled to 0$^{0}$C. 0.52 g (2.53 mM) DCC and 0.465 (2.53 mM) pentafluor phenol are added. The reaction mixture is stirred for 2 hours while stirring, then left for 20 hours at 20$^{0}$C. DCU residue is separated, washed with anhydrous ethylacetate. Filtrates are united, solvent is removed under vacuum. White, slightly yellowish Z-$\gamma$-Abu-OPfp crystalls are produced which are dried under vacuum. Yield is 1.05 g (98.0%). $R_f$ 0.85 (1).

7.2. N-(BENZYLOXYCARBONYL-γ-AMINOBUTYRYL)-L-HISTIDINE METHYL ETHER ($VIII^b$)

To 0.30 g (1.16 mM) L-histidine methyl ether dichlorohydrate 4 ml anhydrous histidine are added and heated to dissolution. The solution is cooled to $0^0$C and cooled sodium methylate solution produced from 0.053 g (2.32 mM) metallic sodium and 1 ml anhydrous methanol, is added. The reaction mixture is left at $0^0$C for 20 minutes, equal volume of dry ether is added and left for 20 minutes at $20^0$C. Sodium chloride residue is separated. Solvent from the filtrate is removed under vacuum. To oily residuel 5 ml anhydrous DMF and 0.50 g (1.24 mM) Z-γ-Abu-OPfp are added and left for 20 hours at $20^0$C. Solvent is removed under vacuum. Sodium chloride residue is separated. Solvent is removed from filtrate under vacuum. To oily residue 5 ml DMF, 0.50 g (1.24 mM) Z-γ-Abu-OPfp are added and left for 20 hours at $20^0$C. Solvent is removed under vacuum. Residue is purified using column chromatography on silicagel L 40/100, eluted with chloroform and methanol gradient in chloroform to the ratio of 2:8. The fractions containing the target substance with $R_f$ 0,46 (2), are united, the solvent is removed under vacuum. To the produced residue, dry ether excess is added with 1 drop of triethylamine and ground. White solid substance is separated, washed with dry ether, dried under vacuum. Yield is 0,243 g (65.0%). $R_f$ 0,46 (2). Mass-spectrum, m/z: 375.

7.3. γ-AMINOBUTYRYL-L-HISTIDrNE METHYL ETHER (VIII).

To 0.04 g (0.124 mM) Z-γ-Abu-L-His-OMe 7 ml anhydrous methanol, 0.04 g 10% palladium on activated carbon are added and hydrated for 1 hour while stirring. Following a complete conversion of original substance with $R_f$ 0.46 (2), into the target product with $R_f$ 0 (2), catalyst is separated, washed with methanol. Filtrates are united, solvent is removed under vacuum. Colorless viscous oil is produced. Yield is 0,030 g (90.0%). $R_f$ 0.05 (3), $R_f$ 0.1 (4). HPLC under the conditions (1): one peak, retention time 4.8 minutes. $^1$H-NMR spectrum, ($D_2O$), δ ppm : 1.85 (m, 2H, β-$CH_2$), 2.23 (t, 2H, γ-$CH_2$), 2.95 (d, 2H, β-$CH_2$-His), 3.1 (t, 2H, α-$CH_2$), 3.7 (s, 3H, $CH_3O$), 4.65 (t, 1H, α-CH-His), 6.85 (s, 1H, CH-5-Im), 7.6 (s, 1H, CH-2-Im).

8.1. $N^\alpha$ -TERT-BUTYLOXYCARBONYL-α-BENZYL-L-GLUTAMYL HISTIDINE ($IX^a$)

To solution of 0.100 g (0.645 mM) L-histidine in 3 ml water 0.75 ml dioxane are added while stirring and then for 2 hours 0.560 g (1.29 mM) Boc-L-Glu (ONSu) Obzl and 2.25 ml dioxane are added in portions (to dioxane to water ratio 1:1). The formed suspension is stirred for 2 hours and left for 20 hours at 20˚C and then suspension is transformed into solution. Solvent is removed under vacuum. Oily residue is purified on a column 30 x 1.6 cm with silicagel 100/160, eluted with chloroform-methanol mixture 5:1, graduslly increasing MeOH content up to 50%. The fractions containing the target substance, are united. The solvent is removed under vacuum. White solid substance is produced. Yield is 0,150 g (45.7%). $R_f$ 0.48 (3). $^1$H-NMR spectrum, ($CD_3OD$), δ, ppm : 1.4 (s, 9H, $(CH_3)_3C$), 1.98 (m, 2H, β-$CH_2$), 2.3 (t, 2H, γ-$CH_2$-Glu), 3.1 (m, 2H, β-$CH_2$-His), 4.07 (t, 1H, α-CH), 4.5 (t, 1H, α-CH-His), 5.18 (m, 2H, $CH_2$ -Bzl),7.17 (s, 1H, CH-5-Im), 7.35 (m, 5H, $C_6H_5$), 8.4 (s, 1H, CH-2-Im).

8.2. γ-L-GLUTAMYL HISTIDINE α-METHYL ETHER DICHLOROHYDRATE (IX)

To solution of 0.140 g Boc-L-Glu (His)-OBzl ($Ix^a$) in 1 ml anhydrous methanol 3 ml 4 n hydrochloride in dioxane are added and kept for 40 minutes at $20^0$C. To reaction mixture ether is added and solvents are removed under vacuum. Sediment is ground with ether until white solid substance is formed. Ether is decanted, residue is dried under vacuum and purified with preparative paper chromatography in the butanol-acetic acis-water system 4:1:5, upper phase. 0.12 g (95.8%) dichlorohydrate dipeptide are produced, $R_f$ 0.26 (3). To solution of 0.117 g product obtained in 4.5 anhydrous methanol, 0.096 g 10% palladium on activated coal are added. Reaction mixture is stirred for 2.5 hours, periodically passing hydrogen flow through it. Catalyzer is separated, ashed on filter 5 ml MeOH. Filtrates are united, solvent is removed under vacuum. Residue is dried under vacuum over $P_2$ $O_5$ . Yield is 0,091 g (96,1%). $R_f$ 0,3 (6). HPLC under the conditions (10): one peak, retention time 5,40 minutes. Mass-spectr; m/z: 299.2. $^1$H-NMR spectrum, ($CD_3OD$), δ, ppm : 2,15 (m, 2H, β-$CH_2$), 2.55 (t, 2H, γ-$CH_2$), 3.16 (t, 1H, α-CH), 3.75 (s, 3H, $CH_3O$), 3.99 (t, 1H, α-CH-His), 4.8 (dd, 2H, β-$CH_2$ -His), 7.38 (s, H, CH-5-Im), 8.80 (s, H, CH-2-Im).

EXAMPLE 9

9.1. N-(BENZYLOXYCARBONYL-γ-AMINOBUTYRYL)TRYPTAMINE ($X^a$)

To solution of 0.252 g (-.625 mM) Z-γ-Abu-OPfp in 5 ml DMF 0.10 g (0.625 mM) tryptamine are added and stirred at $25^0$C for 2 hours. Reaction mixture is left for 16 hours at the same temperature, after which a 7-fold water excess (by volume) is added. White coagulated sediment is filterred off, washed on filter with water and dried. Yield is

0.22 g (93.0%). $R_f$ 0.6 (5), $R_f$ 0.54 (3). Mass-spectr, m/z: 380.6

9.2. γ-AMINOBUTYRYLTRYPTAMINE (X)

To 0.133 g (0.35 mM) Z-γ-Abu-TrpA 7 ml anhydrous methanol are added, stirred untill dissolution of the basic substance mass, 0.133 g 10% palladium on activated coal are added and hydrated for 1 hour. Following a complete convertion of the initial substance with $R_f$ 0.6 (5) into the target product with $R_f$ 0 (5), catalyzer is separated and the product is washed with methanol. Filtrates are united, solvent is removed under vacuum. Colorless viscous oil is produced. Yield is 0,086 g (99.0%). $R_f$ 0,43 (3). HPLC under the conditions (2): one peak, retention time 18.5 minutes. $^1$H-NMR spectrum, ($CD_3OD$), δ, ppm : 1,65 (m, 2H, β-$CH_2$), 2,05 (t, 2H, γ-$CH_2$), 2.85 (t, 2H, β-$CH_2$-TrpA), 3.0 (t, 2H, α-$CH_2$), 3.45 (m, 2H, α-$CH_2$-TrpA), 6,95 (s, 1H, CH-2-Ind), 6.99 (m, 1H, CH-5-Ind), 7.05 (m, 1H, CH-6-Ind), 7.28 (d, 1H, 1H, CH-7-Ind), 7.48 (d, 1H, 1H, CH-4-Ind).

EXAMPLE 10

N-GLUTARYL-O-BENZYLSEROTONIN (XI)

To suspension of 0.20 g (0.66 mM) 0-benzyl serotonin chlorohydrate in 3 ml DMF 0.09 ml (0.66 mM) triethylamine are added while stirring and then 0.075 g (0.66 mM) glutaranhydride. Reaction mixture is stirred for 1 hour and left for 20 hours at 20$^0$C. Sediment of triethylamine chlorohydrate is separated, solvent is removed under vacuum. Oily residue is purified on a column 31 x 1.5 cm with silicagel Silica gel 60, 0.063-0.2 mm (Merck). Elution with chloroform-methanol mixture 9:1 is done. Fractions containing the target product are united, solvent is removed under vacuum. Colorless glassy product is obtained. Yield is 50 mg (20%). $R_f$ 0,48 (1). HPLC under the conditions (14): one peak, retention time 23.8 minutes. $^1$H-NMR spectrum, ($CD_3OD$), δ, ppm : 2.05 (m, 2H, β-$CH_2$-GA), 2,45 (m, 4H, α,γ-$CH_2$-GA), 3.1 (t, 2H, β-$CH_2$), 3.67 (t, 2H, α-$CH_2$), 5.3 (s, 2H, $CH_2$-Bzl), 7.0 (dd, 1H, CH-6-Ind), 7.1-7.7 (m, 8H, CH-7-Ind, CH-2-Ind, CH-4-Ind, $C_6H_5$).

EXAMPLE 11

N-GLUTARYLSEROTONIN (XII)

To 25 mg (0.065 mM) glutaryl-0-benzyl serotonin 4 ml anhydroyus methanol are added. To solution 30 mg catalyzer - palladium on activated coal are added and hydrated for 1 hour while stirring. Catalyzer is filterred out. Solvent is removed from filtrate under vacuum. Yield is 17 mg (90%). $R_f$ 0,31 (1). HPLC under the conditions (14): one peak, retention time 16.27 minutes. $^1$H-NMR spectrum, ($CD_3OD$), δ, ppm : 2.0 (m, 2H, β-$CH_2$-GA), 2.5 (m, 4H, α,γ-$CH_2$-GA), 3.0 (t, 2H, β-$CH_2$), 3.65 (t, 2H, α-$CH_2$), 6.85 (dd, 1H, CH-6-Ind), 7.15 (d, 1H, CH-4-Ind), 7.25 (s, 1H, CH-2-Ind) 7.4 (d, 1H, CH-7-Ind).

EXAMPLE 12

N-GLUTARYL-5-O-METHYLSEROTONIN (XIII)

To solution of 0.20 g (1.05 mM) 0-methyl serotonin in 3 ml DMF 0.12 g (1.05 mM) glutaranhydride are added while stirring. Reaction mixture is stirred for 1 hour and left for 20 hours at 20$^0$C. Solvent is removed under vacuum. Oily residue is purified on a column 31 x 1.5 cm with silicagel Silica gel 60, 0.063-0.2 mm (Merck). Elution with chloroform-methanol mixture 9:1 is done. Fractions containing the target product are united, solvent is removed under vacuum. Colorless glassy product is obtained. Yield is 0.095 g (29.8%). $R_f$ 0.51 (1). HPLC under the conditions (14): one peak, retention time 19.0 minutes. $^1$H-NMR spectrum, ($CDCl_3$+$CD_3OD$), δ, ppm : 1.95 (m, 2H, β-$CH_2$-GA), 2,37 (m, 4H, α,γ-$CH_2$-GA), 3.0 (t, 2H, β-$CH_2$), 3.56 (t, 2H, α-$CH_2$), 3.92 (s, 3H, $CH_3O$), 6.83 (dd, 1H, CH-6-Ind), 7.1 (d, 2H, CH-2,4-Ind), 7.31 (d, 1H, CH-7-Ind).

EXAMPLE 13

N-GLUTARYLTRYPTAMINE (XVII)

To solution of 0.15 g (0.94 mM) tryptamine in 3 ml DMF 0.107 g (0.94 mM) glutaranhydride are added while stirring. Reaction mixture is stirred for 1 hour and left for 20 hours at 20$^0$C. Solvent is removed under vacuum. Oily residue is purified on a column 31 x 1.5 cm ith silicagel Silica gel 60, 0.063-0.2 mm (Merck). Elution with chloroform-methanol mixture 9:1 is done. Fractions containing the target product are united, solvent is removed under vacuum. Colorless glassy product is obtained. Yield is 0.2 g (78%). $R_f$ 0.41 (1). HPLC under the conditions (14): one peak, retention time 18.95 minutes. 1H-NMR spectrum, ($CDCl_3$+$CD_3OD$), δ, ppm : 1.90 (m, 2H, β-$CH_2$), 2,27 (dt, 4H, α,γ-$CH_2$), 2.95 (t, 2H, β-$CH_2$-TrpA), 3.50 (t, 2H, α-$CH_2$-TrpA), 7.0 (d, 1H, CH-7-Ind), 7.55 (d, 1H, CH-4-Ind).

EXAMPLE 14

N-GLUTARYL-4-(2-AMINOETHYL)MORPHOLINE (XIV)

To solution of 0.5 g (4.39 mM) glutaranhydride in 1 ml DMF 0.57 ml (4.39 mM) 4-(2-aminoethyl) morpholine are added while stirring and cooling. Reaction mixture is stirred for 30 minutes and left for 20 hours at $20^0$C. Solvent is removed under vacuum. Oily residue is washed with ether, solvent is removed under vacuum, dried and left at +4˚C for 20 hours. The formed crystalline substance is washed and ground with ether (3 x 1.5 ml) and then acetone (4x1.5 ml), separated from solvents and dried under vacuum. Yield is 0.265 g (24.8%). $R_f$ 0.41 (6). HPLC under the conditions (11): one peak, retention time 3.93 minutes. $^1$H-NMR spectrum, ($CD_3OD$), δ, ppm : 0.5 (m, 2H, β-$CH_2$-GA), 0.9 (m, 4H, α,γ-$CH_2$-GA), 1.3 (m, 8H, $CH_2$-2,3,5,6-morpholinyl), 2.0 (t, 2H, β-$CH_2$), 2.38 (t, 2H, α-$CH_2$).

EXAMPLE 15

N-GLUTARYL-2-(2-AMINOETHYL)PYRIDINE (XV)

To solution of 0.3 g (2.63 mM) glutaranhydride in 1 ml DMF 0.315 ml (2.63 mM) 2-(2-aminoethyl) pyridine are added while stirring and cooling with water. Reaction mixture is stirred for 1 hour and left for 20 hours at 20˚C. White sediment is filterred off, washed many times with ether and acetone and dried. Yield is 0.29 g (46.7%). $R_f$ 0.27 (1). HPLC under the conditions (12): one peak, retention time 14.33 minutes. $^1$H-NMR spectrum, ($CD_3OD$) δ, ppm: 0.41 (m, 2H, β-$CH_2$-GA), 0.81 (m, 4H, α,γ-$CH_2$-GA), 1.6 (t, 2H, β-$CH_2$), 2.15 (t, 2H, α-$CH_2$), 5.9 (m, 2H, CH-4,5-pyridinyl), 6.4 (m, 1H, CH-3-pyridinyl), 7.05 (m, 1H, CH-6-Py).

EXAMPLE 16

N-GLUTARYLPHENYLETHYLAMINE (XVI)

To solution of 0.5 g (4.39 mM) glutaranhydride in 1 ml DMF 0.55 ml (4.39 mM) phenylethylamine are added while stirring and cooling with water. Reaction mixture is stirred for 30 minutes, left for 20 hours at 20˚C. Solvent is removed under vacuum, residue is purified on a column 25 x 230 mm with Kieselgel 40 for column chromatography ("Fluka"). Elution with chloroform ÷ chloroform - MeOH (9:1) is done. Fractions containing the target product with $R_f$ 0.57 (1) are united and dried following solvent removal under vacuum. Yield is 1.02 g (99%). HPLC under the conditions (13): one peak, retention time 15.12 minutes. $^1$H-NMR spectrum, ($CD_3OD$), δ, ppm: 1.9 (m, 2H, β-$CH_2$-GA), 2.15 (m, 4H, α,β-$CH_2$-GA), 2.8 (t, 4H, α,β-$CH_2$-GA), 7.2 (m, 5H, $C_6H_5$).

EXAMPLE 17

CYCLOHEXYLCARBONYLHISTAMINE (XXIX)

To 700 mg (3.85 mM) histamine dichlorohydrate 9 ml anhydrous methanol are added, heated up to 50˚C and stirred until dissolution, then cooled to 0˚C. To solution 1.42 ml (7.7 mM) sodium methylate solution in methanol are added. Mixtire is kept for 20 minutes at 0˚C and then equal volume of anhydrous ether is added and kept for 20 minutes at $0^0$C. NaCl sediment is separated. Solvent is removed from filtrate under vacuum. Oily residue is dissolved in 5 ml DMF and cooled to +5˚C. To solution 0.537 ml (3.85 mM) triethylamine are added while stirring and then 0.52 ml (3.85 mM) cyclohexane carbonic acid chloroanhydride. Reaction mixture is stirred for 1 hour White sediment is separated. Solvent from filtrate is removed under vacuum. Product is crystallized from 2 ml acetone with adding triethylamine. Sediment is separated, washed with 2 ml of ether and acetone (1:1) mixture, 2 ml methanol with acetone (1:1) with addition of triethylamine. $R_f$ 0.57 (7). Yield is 184 mg (52%). HPLC under the conditions (1): one peak, retention time 18.05 minutes. $^1$H-NMR spectrum, ($CD_3OD$) δ, ppm: 1.36-1.78 (m, 10H, $(CH_2)_5$ - cyclohexane), 2.18 (m, 1H, >CH-CO-, 2.8 (t, 2H, β-$CH_2$-HA), 3.41 (t, 2H, α-$CH_2$-HA), 6.85 (s, 1H, 5-CH-Im), 7.6 (s, 1H, 2-CH-Im).

EXAMPLE 18

N-CYCLOHEXYLCARBONYLTRYPTAMINE (XXX)

To solution of 0,20 g (1.25 mM) tryptamine in 2.5 ml dimethyl formamide 0.17 ml (1.25 mM) triethylamine and 0.17 ml (1.25 mM) cyclohexanoyl chloride are added while vigorously stirring. Reaction mixtire is stirred for 1.5 hours in darkness and then 10 ml 5% $Na_2CO_3$ solution are added and stirred for additional 30 minutes. The formed emulsion is extracted with 2 x 15 ml ethylacetate. Ethylacetate extract is united, washed with 30 ml water, dried over anhydrous $Na_2SO_4$. Solvent is removed under vacuum. Oily residue is purified on a column with silicagel Kieselgel 60 with particle size 0.035-0.07 mm (Fluka company) with elution by gradient of solvents chloroform-chloroform:methanol (9:1). Fractions containing the target product are united and solvent is removed under vacuum. Yield is 0.068 g (20.3%). $R_f$ 0.63 (1). HPLC under the conditions (15): one peak, retention time 32.7 minutes. $^1$H-NMR spectrum, ($CDCl_3$), δ, ppm: 1.1-1.85 (m, 10H, $(CH_2)_5$ - cyclohexane), 2.0 (m, 1H, CH-), 2.97 (t, 2H, β-$CH_2$-TA), 3.6 (t, 2H, α-$CH_2$-TA), 7.0 (s, 1H, 2-CH-Ind), 7.1 (t, 1H, 5-CH-Ind), 7.2 (t, 1H, 6-CH-Ind). 7.4 (d. 1H, 4-CH-Ind), 7.6 (d, 1H, 7-CH-Ind).

EXAMPLE 19

N-HEXANOYLTRYPTAMINE (XXXI)

To solution of 0,20 g (1.25 mM) tryptamine in 2.5 ml dimethyl formamide 0.18 ml (1.25 mM) triethylamine and 0.17 ml (1.25 mM) hexanoyl chloride are added while vigorously stirring. Reaction mass is stirred for 1.5 hours in darkness and then 10 ml 5% $Na_2CO_3$ solution are added and stirred for additional 30 minutes. White sediment is separated and washed on filter with 2 x 4 ml water, 2 x 4 ml hydrochloric acid, 3 x 4 ml water, dried under vacuum over $P_2O_5$. The obtained product is then purified on a column with silicagel Kieselgel 60 with particle size 0.063-0.2 mm (Merck company) with elution by gradient of solvents chloroform-chloroform:methanol (9:1). Fractions containing the target product are united and solvent is removed under vacuum. Yield is 0.108 g (33.4%). $R_f$0.54 (1). HPLC under the conditions (15): one peak, retention time 33.13 minutes. $^1$H-NMR spectrum, ($CDCl_3$), δ, ppm: 1.9 (t, 3H, $CH_3$ -), 1.25 (m, 4H, $(CH_2)_2$ -hexanoyl), 1.6 (m, 2H, β-$CH_2$- hexanoyl), 2.15 (t, 2H, α-$CH_2$- hexanoyl), 3.0 (t, 2H, β-$CH_2$- hexanoyl ), 3.6 (t, 2H, α-$CH_2$-TA ), 7.0 (s, 1H, 2-CH-Ind), 7.1 (t, 1H, 5-CH-Ind), 7.2 (t, 1H, 6-CH-Ind), 7.4 (d, 1H, 7-CH-Ind), 7.6 (d, 1H, 4-CH-Ind).

EXAMPLE 20

N-HEXANOYL-L-TRYPTOPHANE METHYL ETHER (XXXII)

To solution of 0,40 g (1.57 mM) HCl • H-Trp-Ome in 2.5 ml dimethyl formamide 0.44 ml (3.14 mM) triethylamine and 0.216 ml (1.57 mM) hexanoyl chloride are added while stirring. In 2,5 hours to reaction mixtire at cooling with ice 20 ml, 5% $Na_2CO_3$ solution are added and stirred for 10 minutes. The product is extracted with 2 x 25 ml ethyl acetate. United ethyl acetate extracts are washed with equal water volume and dried under anhydrous $Na_2SO_4$. Solvent is removed under vacuum. Oily residue is purified on a column with silicagel Kieselgel 60 with particle size 0.063-0.2 mm (Fluka company) with elution by gradient of solvents chloroform-chloroform:methanol (9,5:0,5). Fractions containing the target product are united and solvent is removed under vacuum. Yield is 0.138 g (27.7%). $R_f$ 0.79 (1). HPLC under the conditions (16): one peak, retention time 6.92 minutes. $^1$H-NMR spectrum, ($CDCl_3$), δ, ppm: 0.9 (t, 3H, $CH_3$ -), 1.24 (m, 4H, γ,δ, -$CH_2$ -hexanoyl), 1.57 (m, 2H, β-$CH_2$-),2.13 (t, 2H, α-$CH_2$-), 3.31 (d, 2H, β-$CH_2$-Trp), 3.7 (s, 3H, $CH_3$O-) 4.96 (t, 1H, α-CH-Trp), 7.0 (s, 1H, 2-CH-Ind), 7.12 (m, 2H, 5-CH, 6-CH-Ind), 7.33-7.53 (dd, 1H, 4,7-CH-Ind).

EXAMPLE 21

N-(IMIDAZOLYL-4-ACETHYL)-HEPTHYLAMINE (XXXIII)

To solution of 0.30 g (1.84 mM) imidazolacetic acid chlorohydrate in 2.5 ml DMF while stirring and cooling with ice, 0.248 g (1.84 mM) 1-oxybenzotriazole and 0.38 g (1.84 mM) dicyclohexylcarboiimide are added. Reaction mixture is stirred for 1 hour and left for 20 hours at +4˚C. Dicyclohexylurea sediment is separated, solvent is removed from filtrate under vacuum. Fractions containing the target product are united, solvent is removed under vacuum. Yield is 0.12 g (29%). $R_f$ 0.39 (1). HPLC under the conditions (15): retention time of individual substance is 28.01 minutes. $^1$H-NMR spectrum, ($CDCl_3$), δ, ppm: 0.9 (t, 3H, $CH_3$ -), 1.3 (m, 10H, -$(CH_2)_5$ -), 1.8 (t, 2H, α-$CH_2$- ), 3.0 (s, 2H, - $CH_2$-), 7.6 (s, 1H, 5-CH-Im), 8.2 (s, 1H, 2-CH-Im) . Mass-spectrum: $[M+1H]^+$, m/z:224.

EXAMPLE 22

22.1 $N^{\alpha}$-TERT.-BUTYLOXYCARBONYL-β-ALANINE BENZYL ETHER

Solution of 0.8 g (5.29 mM) Boc-β-Ala in 4 ml ethanol is added to solution of 1.38 g (5.29 mM) cesium carbonate in water. Solvent is removed under vacuum. Oily residue is dried under vacuum until formation of 1.45 g glassy residue of cesium Boc-β-Ala salt which then is dissolved in 10 ml DMF. To solution 0.538 ml (4.53 mM) benzyl bromide are added while stirring and cooling and mixture is stirred for 1,5 hours. Cesium bromide sediment is separated and solvent is removed from filtrate under vacuum. Oily residue is purified with column chromatography on silicagel with elution by chloroform. 0.70 g (55.0%) Boc-β-Ala benzyl ether are produced. $R_f$ 0.27 (9).

22.2 N -BENZYLOXYCARBONYL-L-HYSTIDYL-β-ALANINE BENZYL ETHER

To 60 g (2.15 mM) Boc-β-Ala-OBzl 3.5 ml 4N HCl solution in dixane are added and left for 40 minutes. Then 3x5 ml anhydrous ether are added and solvent is removed under vacuum. Oily residue is dissolved in 3,5 ml DMF, triethylamine is added up to pH 8. 0.622 g (2.15 mM) Z-L-His and 1.63 g (2.15 mM) "F complex" are added while cooling with ice and stirring. Reaction mixture is stirred at 0° C for 1 hour and left for 20 hours at +4$^0$C. Dicyclohexylurea sediment is separated, solvent is removed from filtrate under vacuum. Oily residue is dissolved in acetone and triethylamine is added up to pH 9. Solvent is removed under vacuum, residue id purified on a column with silicagel in gradient of solvents chloroform-chloroform: methanol (9,5:0<5). 0.170 g of white solid substance are produced. $R_f$ 0.44 (1).

22.3 N-L-HISTIDYL-β-ALANINE (XXXVI)

To solution of 0.14 g Z-L-His-β-Ala-OBzl in 3 ml methanol 0.1 g 10% palladium on coal are added and stirred for 1,5 hours, periodically passing through it hydrogen flow. Catalyzer is separated, solvent is removed under vacuum, residue is dried. Yield is 0.085 g (98%). $R_f$ 0.69 (10). HPLC under the conditions (15): retention time of individual substance is 3.55 minutes.

EXAMPLE 23

23.1. ASPARTYL HISTAMINE $N^{\alpha}$ -TERT.-BUTYLOXYCARBONYL-β-BENZYL ETHER

To solution of 1.7 g (3.48 mM) Boc Asp (OBzl) OPfp in 5 ml DMF are added while stirring. Reaction mixture is stirred for 1,5 hours and left for 20 hours at +4°C. Solvent is removed under vacuum. Oily residue is dissolved in 2 ml chloroform, triethylamine is added up to pH 8 and purified on a column 30 x 1,5 cm with silicagel 40/100 in gradient of solvents chloroform-chloroform:methanol (9:1). Fractions containig the target product, are united and evaporated. Colorless substance is produced. Yield is 800 mg (55%), $R_f$ 0.29 (1).

23.2. $N^{\alpha}$-TERT.-BUTYLOXYCARBONYL- ASPARTYLHISTAMINE

85 mg Boc-Asp (OBzl)HA are dissolved in 2 ml methanol, 80 mg Pd/C are added and stirred for 1,5 hours, periodically passing through it hydrogen flow. Catalyzer is filterred off, filtrate is evaporated. 65 mg (95%) clear oil are produced. $R_f$ 0.35 (3).

23.3. α-L- ASPARTYLHISTAMINE (XXXVIII)

Solution of 65 mg Boc-Asp-HA in 2 ml 4N HCl in dioxane is kept for 40 minutes. Solvent is removed under vacuum, residue is washed with dry ether, solvent is removed under vacuum. Yield is 50 mg (83%) 2HCl • Asp-HA. $R_f$ 0.57 (10). $^1$H-NMR spectrum, ($CDCl_3$+$CD_3OD$), δ, ppm: 2.0 (d, 2H, β-$CH_2$ -Asp), 2.9 (t, 2H, β-$CH_2$ -HA), 3.5 (t, 2H, α-$CH_2$-HA), 4.2 (t, 1H, α-$CH_2$-Asp), 7.0 (s, 1H, 5-CH-Im), 8.15 (s, 1H, 2-CH-Im) . Mass-spectrum:, m/z: $[M+1H]^+$ 227.2.

According to the typical techniques, novel compounds of general formula (I) shown in table 2, are also produced.

Table 2 Structure and characteristics of compounds of general formula (I)

| № | $R_1$ | n | $R_2$ | m | $R_3$ | Physical-chemical characteristics |
|---|---|---|---|---|---|---|
| V | **$HOOC-CH_2CH-$** | 1 | H | 1 | -4-Im | $^1H$-NMR spectrum: 1.5 (d, 3H, $CH_3$), 1.75 (m, 1H, β-CH), 2.2 |
| | **$CH_3$** | | | | | (m, 4H, α,γ- $CH_2$), 2.8 (t, 2H, β-$CH_2$-HA), 3.4 (t, 2H, α-$CH_2$-HA), 7.3 (s, 1H, CH-5-Im), 8.6 (s, 1H, CH-2-Im). Mass-spectrum, m/z: $[M+1H]^+$ 240.2. |
| VIII | **HOOC-CH-**<br>\|<br>**$NH_2$** | 2 | $CH_3$ OOC | 1 | -3-Ind | $^1H$-NMR spectrum: 2.12 (m, 2H, β-$CH_2$), 2.43 (t, 2H, γ-$CH_2$), 3.12 (t, 2H, β-$CH_2$-Trp), 3.7 (s, 3H, $CH_3O$), 3.85 (t, 1H, α-CH), 3.92 (t, 1H, α-CH-Trp), 6.99 (t, 1H, 5-CH-Ind), 7.05 (s, 1H, 2-CH-Ind), 7.06 (t, 1H, 6-CH-Ind), 7.55 (d, 2H, 4,7-CH-Ind). Mass-spectrum, m/z: $[M+1H]^+$ 348.3. |
| XIII | **$NH_2$-** | 2 | H | 1 | -3-Ind | $^1H$-NMR spectrum: 2.35 (dt, 4H, α,β-$CH_2$), 3.05 (t, 2H, β-$CH_2$-TrpA), 3.63 (t, 2H, α-$CH_2$-TrpA), 7.07 (m, 3H, 2-CH-2,5,6-Ind), 7.43 (d, 1H, CH-7-Ind), 7.43 (d, 1H, CH-7-Ind), 7.65 (d, 1H, CH-4-Ind). Mass-spectrum, m/z: $[M+1H]^+$ 232.5. |
| XVIII | **HOOC-** | 3 | H | 1 | where $R_4$ =$CH_3CO$- | $^1H$-NMR spectrum,: 1.98 (m, 2H, β-$CH_2$ -GA), 2.25 (s, 3H, $CH_3CO$), 2.45 (m, 4H, α,γ-$CH_2$ -GA), 3.13 (t, 2H, β-$CH_2$), 3.60 (t, 2H, α-$CH_2$), 6.91 (dd, 1H, CH-6-Ind), 7.15 (d, 2H, CH-2,4-Ind), 7.36 (d, 1H, CH-7-Ind). Mass-spectrum, m/z: $[M+1H]^+$ 333.3 |
| XXII | **HOOC-** | 2 | H | 1 | | HPLC under the conditions (6): one peak, retention time 8,9 minutes. Mass-spectrum, m/z: $[M+1H]^+$ 226.2 |
| XXIII | **HOOC-** | 3 | $CH_3OOC$ | 1 | | Mass-spectrum:, m/z: $[M]^+$ 299.2 Calculated %: S 10.69. Found %: 10.85. |

(continued)

| № | $R_1$ | n | $R_2$ | m | $R_3$ | Physical-chemical characteristics |
|---|---|---|---|---|---|---|
| XXIV | **HOOC-** | 3 | H | 1 | | $^1$H-NMR spectrum: 0.62 (m, 2H, β-$CH_2$ -GA), 0.95 (m, 4H, α,γ- $CH_2$-GA), 1.15 (d, 3H, $CH_3$), 1.75 (m, 4H, $CH_2$-3,4-pyrrolidinyl), 2.5 )s, 3H, $CH_3$-), 2.74 (t, 2H, β- $CH_2$), 3.05 (t, 2H, α-$CH_2$), 3.21 (t, 2H, $CH_2$-5-pyrrolidinyl), 3.34 (m, 1H, CH-2-pyrrolidinyl) Mass-spectrum, m/z: $[M]^+$ 242.3. |
| XXV | **HOOC-** | 3 | H | 1 | | $^1$H-NMR spectrum: 0.5-65 (m, 2H, β-$CH_2$ -GA; $CH_2$-3,4,5-piperazinyl), 0.9 (m, 4H, α,γ- $CH_2$-GA), 1.25 (m, 4H, $CH_2$-2,6 -piperazinyl), 2.42 (t, 2H, α-$CH_2$). Mass-spectrum, m/z: $[M]^+$ 242.3. |
| XXVI | **HOOC-** | | H | 1 | | $^1$H-NMR spectrum: 0.6 (m, 2H, β-$CH_2$ -GA), 0.8-0.9 (m, 4H, $CH_2$-3,4 - pyrrolidinyl), 1.05 (m, 4H, α,γ- $CH_2$-GA), 1.35 (m, 4H, $CH_2$-2,6 -pyrrolidinyl), 2.05 (t, 2H, β-$CH_2$), 2.4 (t, 2H, α- $CH_2$). Mass-spectrum, m/z: $[M]^+$ 228.2. |
| XXVII | **HOOC-** | 2 | H | 2 | | HPLC under the conditions (7): one peak, retention time 13,8 minutes. Mass-spectrum, m/z: $[M+1H]^+$ 240.2 |
| XXVIII | **$C_2H_5OCO$-** | 1 | H | 1 | -4-Im | $^1$H-NMR spectrum,: 1.77 (m, 2H, β-$CH_2$), 2.22 (m, 4H, α,γ-$CH_2$), 2.9 (t, 2H, β-$CH_2$-HA), 3.49 (t, 2H, α-$CH_2$-HA), 3.6 (dd, 2H, -$CH_2$-O-CO), 3.75 (t, 3H, $CH_3$ -$CH_2$-), 7.26 (s, 1H, CH-5-Im), 8.6 (s, 1H, CH-2-Im). Mass-spectrum, m/z: $[M+1H]^+$ 226.2. |
| XXXVII | **$CH_3$-CONH-** | 3 | H | 1 | -4-Im | $^1$H-NMR spectrum,: 1.91 (m, 2H, β-$CH_2$), 2.18 (s, 3H, $CH_3$CO), 2,36 (t, 2H, γ-$CH_2$), 2.97 (t, 2H, β-CH2-HA), 3.32 (t, 2H, α-$CH_2$-HA), 3.53 (t, 2H, α-$CH_2$), 7.32 (s, 1H, CH-5-Im), 8.66 (s, 1H, CH-2-Im). Mass-spectrum, m/z: $[M+1H]^+$ 239.1. |

(continued)

| № | $R_1$ | n | $R_2$ | m | $R_3$ | Physical-chemical characteristics |
|---|---|---|---|---|---|---|
| XXXVIII | $NH_2$-CH-<br>\|<br>COOH | 1 | H | 1 | -4-Im | $^1$H-NMR spectrum: 1.8 (d, 2H, β-$CH_2$-Asp), 2.72 (t, 2H, β-$CH_2$-HA), 3.31 (t, 2H, α-$CH_2$-HA), 4.05 (t, 1H, α-CH-Asp), 6.9 (s, 1H, CH-5-Im), 7.95 (s, 1H, CH-2-Im). Mass-spectrum, m/z: $[M+1H]^+$ 227.2. |
| XXXIV | **-3-Ind** | 1 | H | 2 . | $NH_2$)CH-COOH | $^1$H-NMR spectrum: 1.85 (m, 4H, β,γ-$CH_2$), 2.1 (t, 2H, δ-$CH_2$), 3.1 (s, 2H, -$CH_2$-), 3.45 (t, 2H, α-CH), 6.95 (s, 1H, CH-2-Ind), 7.0-7.05 (m, 2H, CH-5,6-Ind), 7.4-7.45 (dd, 2H, CH-4,7-Ind). Mass-spectrum, m/z: $[M+1H]^+$ 290.2. |
| XXXV | **-3-Ind** | 2 | H | 2 | -$NH_2$ | $^1$H-NMR spectrum: 1.7 (m, 2H, β-$CH_2$), 2.1 (t, 2H. γ-$CH_2$), 2,9 (t, 2H, β-$CH_2$-indolyl-propion.), 3.05 (t, 2H, α-$CH_2$), 3.5 (m, 2H, α-$CH_2$-indolyl-propion.), 7.0 (s, 1H, CH-2-Ind), 7.05 (m, 1H, CH-5-Ind), 7.1 (m, 1H, CH-6-Ind), 7.32 (d, 1H, CH-7-Ind), 7.5 (d, 1H, CH-4-Ind). Mass-spectrum, m/z: $[M+1H]^+$ 245.2. |
| XL[6] | 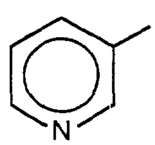 | 0 | H | 1 | -4-Im | $^1$H-NMR spectrum: 2.4 (t, 2H, β-$CH_2$HA), 3.3 (t, 2H, α-$CH_2$HA), 7.1-7.15 (m, 4H, 2,4,5,6-CH), 7.3 (s, 1H, CH-5-Im), 8.65 (s, 1H, CH-2-Im). Mass-spectrum, m/z: $[M+1H]^+$ 217.1. |
| XLI | 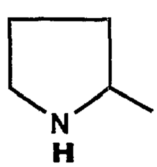 | 0 | H | 1 | -3-Ind | $^1$H-NMR spectrum: 2.01 (m, 2H, 4-$CH_2$), 2.3 (m, 2H, 3-$CH_2$ -), 2.42 (t, 2H, β-$CH_2$TrpA), 3.4 (t, 2H, 5-$CH_2$-), 3,4 (t, 2H, 5-$CH_2$-), 3.46 (t, 2H, α-$CH_2$-TrpA), 4.1 (m, 1H, 2-CH), 6.95 (m, 3H, 2,5,6-CH-Ind), 3.5 (m, 2H, α-$CH_2$-indolyl-propion.), 7.0 (s, 1H, CH-2-Ind), 7.05 (m, 1H, CH-5-Ind), 7.6 (dd, 2H, 4,7-CH-Ind). Mass-spectrum, m/z: $[M+1H]^+$ 257.2. |

(continued)

| № | $R_1$ | n | $R_2$ | m | $R_3$ | Physical-chemical characteristics |
|---|---|---|---|---|---|---|
| XLII | 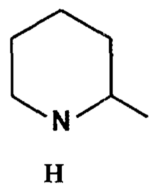 | 0 | H | 1 | -4-Im | $^1$H-NMR spectrum: 2.1-2.25 (m, 6H, 3,4,5-$CH_2$-), 2.4 (t, 2H, β-$CH_2$ -), 2.4 (t, 2H, β-$CH_2$-HA), 3.45 (t, 2H, 6-$CH_2$-), 3,48 (t, 2H, α-$CH_2$-HA), 4.15 (t, 1H, 2-CH), 7.2 (s, 1H, CH-5-Im), 8.5 (s, 1H, CH-2-Im). Mass-spectrum, m/z: $[M+1H]^+$ 223.2. |
| XLIII | 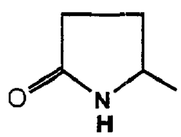 | 0 | H | 1 | -3-Ind | $^1$H-NMR spectrum: 2.1 (m, 2H, β-$CH_2$), 2.5 (t, 2H, β-$CH_2$ TrpA), 2.93 (t, 2H, γ-$CH_2$), 3.45 (t, 2H, α-$CH_2$-TrpA), 4.25 (t, 1H, α-CH), 3.46 (t, 2H, α-$CH_2$-TrpA), 7.03 (m, 1H, CH-5-Ind), 7.09 (s, 1H, CH-2-Ind), 7.1 (m, 1H, CH-6-Ind), 7.6 (d, 1H, CH-7-Ind), 7.65 (d, 1H CH-4-Ind). Mass-spectrum, m/z: $[M+1H]^+$ 271.2. |

Further, examples of biological trials of general formula (I) compounds are presented.

All experimental results were statistically processed {Gubler E.V. Computer analysis methods and recognition of pathological processes. Moscow, Nauka publishers (1978), 365 pp.].

EXAMPLE 24

ANTIHYPOXIC ACTIVITY STUDY OF THE COMPOUNDS OF GENERAL FORMULA (I)

The tests of determining average lifetime of animals in a closed volume and on the model of acute hypobaric hypoxia at the minimum atmospheric rarefaction (170-185 mm Hg) were used to study antihypoxic activity of the compunds of general formula (I).

A. Determining average life-span of animals in a closed volume

Mice were one by one placed into sealed glass jars and their lifetime was recorded.

The experiment was carried out on 90 mongrel male mice having initial body mass 20 ± 0,5 g housed in cages by 10 animals and given a standard vivarium ration. Average lifetime of mice in closed vessels having volume 250 ml was preliminarily determined and it was 22 to 25 minutes. All the groups included 10 animals each.

Experimental protocol:

a) - preliminary hypoxia for 12 minutes;
b) - first administration of a compound immediately following preliminary hypoxia;
c) - second administration of a compound in 24 hours after the first one, then daily for three days;
d) - recording lifetime of animals under hypoxic conditions in a sealed space 2 hours after the last administration of a compound.

The test compounds were dissolved in distilled water and orally administered to animals for 5 days.

Experimental groups:

Group 1 -(control) - without preliminary hipoxia with oral administration of distilled water for 5 days;

Groups 2 and 3 - administration of the compound XLIV using two doses 50 and 500 μg respectively;
Group 4 - oral administration of sodium oxybutyrate at a dose 300 μg/kg 2 hours before hypoxia;
Group 5 -(control)- with preliminary hypoxia for 12 minutes with subsequent administration of distilled water for 5 days;
Groups 6 and 7 - administration of the compound XLIV using two doses 50 and 500 μg respectively;
Groups 8 and 9 - administration of the compound III using two doses 50 and 500 μg respectively.

The data presented in table 3, give evidence of the fact that the compounds of general formula (I) possess a pronounced antihypoxic activity, significantly increasing lifetime of experimental animals by 20-50 percent. It should be noted that the tested compounds rised lifetime of mice in a way comparable with the effect of the reference drug (sodium oxybutyrate) in their administration at doses that are lower by three-four orders.

Table 3

| Effect of test compounds on lifetime of mice uder hypoxic conditions | | |
|---|---|---|
| Groups of animals | Drug dose (mg/kg) | Lifetime (seconds) |
| Group 1 - without preliminary hypoxia | - | 1440,3±86,2 |
| Group 2 - compound XLIV | 0,05 | 2110,6±123,6**[1] |
| Group 3 - compound XLIV | 0,50 | 1742,8±68,1*[1] |
| Group 4 - sodium oxybutyrate | 300 | 2103,9±100,5**[1] |
| Group 5 - control with hypoxia | - | 1437,2±87,3 |
| Group 6 - compound XLIV | 0,05 | 1415,5±47,2 |
| Group 7 - compound XLIV | 0,50 | 1844,8±93,2*[5] |
| Group 8 - compound III | 0,05 | 1670,6±25,3*[5] |
| Group 9 - compound III | 0,50 | 1532,6±96,0 |
| *- significant difference relative to the respective control group (figures 1 and 5); * - p<0,05; ** - p<0,01. | | |

B. Acute hypobaric hypoxia model (AHBH)

Simulation of AHBH was done in a flow-through chamber at temperature 22°C. Experiments were carried out on mongrel mice with initial body mass 20 g at the minimum rarefaction 170-185 mm Hg, that corresponds to the altitude of 10500-11000 m. Lift rate was 35 m/second. Each group included 15 animals. Compounds were administered orally, lift on to altitude was performed 2 hours after the last administration.
Groups of animals:

Group 1 - intact control -administration of normal saline four times.
Groups 2 and 3 - administration of the compound III at doses 50 and 500 μg/kg for 4 days.
Group 4 - single administration of sodium oxybutyrate at a dose 300 μg/kg.

Results ere statistically processed.
The results presented in table 4, give evidence of the fact that administration of the compound at a dose 500 μg/kg, increases lifetime of animals under hypoxic conditions.

Table 4

| Effect of the compounds on lifetime of mice under hypoxic conditions | | | |
|---|---|---|---|
| Groups of animals | Dose in μg/kg | Lifetime under hypoxic conditions (minutes) | Effect in percent |
| 1. control | - | 306,0 ± 9,8 | 100 ± 3,2 |
| Group 2 | 50 | 322,0 ± 10,9 | 105 ± 3,5 |
| Group 3 | 500 | 345,3 ± 15,9 | 113 ± 5,2* |

(continued)

| Effect of the compounds on lifetime of mice under hypoxic conditions | | | |
|---|---|---|---|
| Groups of animals | Dose in μg/kg | Lifetime under hypoxic conditions (minutes) | Effect in percent |
| Group 4 | 300,000 | 364,3 ± 13,5 | 119 ± 4,4* |
| *- significant difference relative to the control group;<br>* p<0,05. | | | |

EXAMPLE 25

ANTIISCHEMIC ACTIVITY OF COMPOUNDS

Antiischemic activity of a compound was assessed by an integral test, i.e. effect on the necrosis zone area following reproduction of myocardial infarction in mongrel rats weighing 250-300 g.

Effect of a compound on the necrosis zone area was studied in 4 hours after arterial occlusion in rats using a differential indicator method [Sernov L.N., Gatsura V.V. A differential indicator method for determinig ischemic and necrotic zone in experimental myocardial infarction in rats. Bulletin of Experimental Biology and Medicine (1989), No 5, pp. 534-535]. All the painful manipulations were performed on animals under Ethaminal anesthesia (Ethaminal - 40 mg/kg intraperitoneally). Experiment was carried out in 15 hours after the last compound administration. Each group included 8 animals.

Groups of animals:

Group 1 - intact control -administration of normal saline four times.
Groups 2 and 3 - oral administration of the compound III at doses respectively 50 and 500 μg/kg for 4 days.
Group 4 - single intraperitoneal administration of Nicorandil at a dose 1 mg/kg immediately after occlusion.

Data presented in table 5, give evidence of the fact that the compounds possess antiischemic activity.

Table 5

| The compound effect on the necrosis zone area 4 hours after coronary artery occlusion in rats | | | | |
|---|---|---|---|---|
| Group of animals | Dose, μg/kg | Ischemia zone, percent to the total myocardial mass | Necrosis zone, percent to the total myocardial mass | Necrosis zone percent to myocardial zone |
| Group 1 | - | 25,5 ± 1,7 | 17,1±1,2 | 67,6 ± 4,0 |
| Group 2 | 50 | 27,2 ± 2,8 | 15,4 ± 1,7 | 56,8 ± 2,7* |
| Group 3 | 500 | 31,1 ± 3,1 | 17,8 ± 1,0 | 54,3 ± 2,1* |
| Group 4 | 1000 | 25,0 ± 1,9 | 11,3 ± 1,6 | 44,3 ± 5,0* |
| *- significant difference relative to the control group;<br>* p<0,05. | | | | |

EXAMPLE 26

CHANGE IN FOOD ANAPHYLAXIS SEVERETY AS AFFECTED BY THE COMPOUNDS OF GENERAL FORMULA (I)

Tests were carried out on mongrel guinea-pigs with initial body mass 250-300 g given general vivarium ration. Sensibilization of animals was performed according to the published method [Shaternikov V.A., Marokko I.N., Pyatnitsky N.N., Shirina L.I., Gorgoshidze L.Sh., Kasyanenko V.V., Sugonyaeva N.P., Zhminchenko V.M., Vinokurova N.M. Experimental reproduction of food anaphylaxis. Voprosy pitaniya (Nutrition problems)(1982) No 2, pp. 27-31] with once recrystallized chicken ovalbumin (OVA), manufactured by the Olajne Scientific-Productive Amalgamation BIOCHEMREACTIV, at a dose 50 mg per an animal daily for 3 days. 14 days after completion of sensibilzation, active anaphylactic shock (AAS) was induced in animals with intravenous injection of anaphylaxis-provoking homologous protein dose 5 mg in 0,5 ml normal saline. AAS severety was assessed by the lethality level, the number of seizure signs and by the value of anaphylactic index [Weigle W., Cochrane C., Dixon F. Anaphylactogenic properties of soluble antigen-antibody

complexes in the guinea pigs and rabbits. J. Immunology. (1969), Vol. 85, pp. 469-477].

Compounds III and XLV and Claritine (groups 2, 3 - 30 guinea pigs and group 4 - 24 animals respectively) were orally administered for 3 days before provoking anaphylaxis at compound dose 50 μg/kg and Claritine dose 1000 μg/kg respectively. Animals of the control group at respective terms were administered normal saline (group 1-30 guinea pigs).

Significance of differences between groups were determined using the Fischer method of angular transformation [Gubler E.V. Computer methods of analysis and recognition of pathological processes. Moscow, Nauka publishers (1978) 365 pp.].

Table 6 shows a significant decrease in the severety of anaphylactic shock manifestations in guinea pigs which were given the compounds as compared with both the control group and the group of animals which were administered Claritine.

Table 6

| Change in food anaphylaxis severety in guinea pigs as affected by one of the compounds of general formula (I) | | | |
|---|---|---|---|
| Food anaphylaxis severety Group of animals | Lethality in percent | Seizures in percent | Anaphylactic index |
| Sensibilization with OVA | 20.0 | 40.0 | 2.13 |
| Sensibilization with OVA + compound III | 6.7** | 13,3* | 0.83* |
| Sensibilization with OVA + compound XLV | 6.7** | 15.5* | 0.90* |
| Sensibilization with OVA + Claritine | 25.0 | 53.3 | 2.03 |
| * - significant difference in relation to groups 1 and 3.<br>** - p<0.01 | | | |

EXAMPLE 27

EFFECT STUDY OF THE COMPOUNDS OF GENERAL FORMULA (I) ON BRONCHOSPASM SEVERETY

Antiasthmatic effect of the test compounds was studied using an antigen-induced bronchospasm model in actively sensitized guinea pigs according to the Andersson method [Andersson P. Antigen induced bronchial anaphylaxis in actively sensitized guinea pig. Allergy (1980), Vol. 35, pp. 65-71].

Male guinea pigs were sensitized with intramuscular injection of 0.5 ml suspension comprising 20 micrograms ovalbumin (OVA, manufactured by the Sigma company [grade III]) and 100 mg $Al(OH)_3$ per one animal. Anaphylaxis-provoking dose 150-200 μg/kg OVA in 0.1 ml normal saline was intravenously (v. jugularis) injected on day 26 after sensibilization. Bronchospasm induction and measuring external respiration parameters were carried out according to the method described in Yu-Hong L., Barnes P., Rogers D. Inhibition of neurogenic plasma exudation and bronchoconstriction by a K+ channel activator, BRL 38227, in guinea pig airways in vivo. Europ. J. Pharmacol. (?), Vol. 239, pp. 257-259]. Measurement of respiration parameters was performed using a transducer (Ugo Basel, model 7020), connected with a cannula and a recorder (Millichrome), recording breathing amplitude. Bronchospasm dynamics was observed for 30-60 minutes. Efficacy of compounds was determined by change in bronchospasm value.

A number of the compounds of general formula (I) were tested (III, IV, VI, VII, X, XXXI, XLIV, XLVIII, XLIX), wich were dissolved in normal saline and three times intragastrally infused to animals at a dose 50 μg/kg 72, 48 and 18 hours prior to bronchospasm induction and compound III intratracheally at the same dose 15 minutes prior to bronchospasm induction. Inthal was used as a reference drug that was administered to animals at a dose 5 μg/kg according to the same dosage regimen as the test substances were administered. The control group of animals received equivalent amount of normal saline.

Compounds III and XLIV were studied in greater details.

Groups of animals: group 1 - control; group 2 - animals given Inthal; group 3 - animals given oral compound XLIV; group IV - animals given oral compound III; group 5 - animals given intratracheal compound III; group 6 - animals given compound XLIX, group 7 - animals given compound XXXI, group 8 - animals given compound XLVI; group 9 - animals given compound VI, group 10 - animals given compound XLVIII.

All the tested compounds lowered bronchospasm value by 20-70% as compared with the control values. Inthal decreased bronchospasm value almost by 50%.

Table 7 summarizes the results giving evidence of the fact that the compounds of general formula (I) decrease bronchospasm value by over 50% as compared with the control values.

Table 7

| Change in bronchospasm value as affected by the test compounds | | |
|---|---|---|
| Groups of animals | Number of animals in a group | Bronchospasm (percent of the maximum value) |
| Group 1 - control | 21 | 87.5 10.7 |
| Group 2 - Inthal, 5 mg/kg | 14 | 40.7 3.4* |
| Group 3 - compound XLIV, 50 μg/kg per os | 12 | 15.6 3.2** |
| Group 4 - compound III, 50 μg/kg, per os | 12 | 12.0 4.0** |
| Group 5 - compound III, 50 μg/kg, intratracheally | 12 | 24.6 6.4* |
| Group 6 - compounds XLIX, 50 μg/kg, per os | 15 | 33.0 9.4* |
| Group 7 - compound XXXI, 50 μg/kg, per os | 12 | 39.3 7.6* |
| Group 8 - compound XLVI, 50 μg/kg, per os | 12 | 35.0 8.2* |
| Group 9 - compound VI, 50 μg/kg, per os | 12 | 35.0 10.2* |
| Group 10 - compound XLVIII, 50 μg/kg, per os | 12 | 22.0 3.4* |
| * - significant difference with the control group, * - p<0.05, ** - p<0.01 | | |

Thus, the tested compounds caused similar decrease in bronchospasm value as the reference drug Inthal. The effect was manifested in both oral and intratracheal method of administering the compounds. However, the effective dose of the tested substances was by two orders lower than that of Inthal.

EXAMPLE 28

STUDY OF CHANGE IN PASSIVE CUTANEOUS ANAPHYLAXIS REACTION AS AFFECTED BY THE COMPOUNDS OF GENERAL FORMULA (I)

Experiments were carried out on mongrel male mice with initial body mass 22-24 g. Each group included 10 animals. Sensibilization of animals was accomplished by intradermal injection of 20-30 μl of the serum of mice obtained from preliminaryly immunized animals comprising reagent antibodies against chicken ovalbumin (OVA). The test compounds III and XLIV were orally administered to animals for 3 days beginning from the dermal sensibilization day at doses 50, 150 and 500 μg/kg, and compound XLIV at a dose 50 μg/kg. Reference drugs Suprastine at a dose 10 mg/kg and Ckaritine at a dose 2 mg/kg were administered in a similar way. In 48 hours after sensibilzation an anaphylaxis-provoking OVA dose (1 mg and 0,2 ml Evans blue in 0.2 ml normal saline) were intravenously injected to mice. Passive cutaneous anaphylaxis reaction intensity (PCA) was determined in 30 minutes by the size (area) of a blue spot on the internal skin surface in the site of reagent anibodies' administration. Spot size was measured in two mutually perpendicular directions and the result was expressed in $mm^2$.

The results presented in table 8, give evidence of the fact that administration of the test compound III at a dose 50 μg/kg to mice results in a significant decrease in PCA reaction intensity by 42%, and at doses 150 and 500 μg/kg, by 34.3% and 30.0% respectively. Administration of the compound XLIV results in decrease in PCA reaction intensity by 50%. Administration of Suprastine to experimental animals did not change PCA manifestations. When Claritine was used, a pronounced decrease in PCA reaction was observed.

Table 8

| Effect of the test compounds on passive cutaneous anaphylaxis reaction | | |
|---|---|---|
| Groups of animals | Reaction intensity, $mm^2$ | Percent of the reaction inhibition |
| Control - 1 | 81.7 ± 14.5 | - |
| Compound III - 150 μg/kg | 53.7 ± 10.1*[1] | 34.3 |
| Compound III - 500 μg/kg | 57.2 ± 12.6*[1] | 30.0 |
| Control - 2 | 88.9 ± 34.6 | - |
| Compound III - 50 μg/kg | 51.6 ± 17.1*[2] | 42 |
| Compound XLIV - 50 μg/kg | 44.6 ± 11.6 | 50 |
| Suprastine - 10 μg/kg | 82.1 ± 18.1 | 7.6 |
| Claritine - 2 μg/kg | 6.4 ± 2.3**[2] | 92.8 |
| *- significant difference relative to the control groups 1 and 2, respectively; * - p0.05; **- p<0.01. | | |

Thus, the test compound possesses ability of lowering PCA manifestation to a greater extent than Suprastine. PCA reaction intensity with administering Claritine to animals was inhibited to a greater extent than with administering the test compound, however, its effective dose was by an order higher than in the test compound.

EXAMPLE 29

STUDY OF THE COMPOUNDS' OF GENERAL FORMULA (I) EFFECT ON DELAYED HYPERSENSITIVITY

A. Effect of the compound III on the dalayed type hypersensitivity reaction development induced by sheep red blood cells

Studies were cartried out on mongrel white male mice with initial body mass 22-24 g. Each group included 10 animals. Mice were sensitized with intravenous injection of the suspension of 2 x $10^5$ sheep red blood, cells in 0.05 ml normal saline. By day 5, the suspension of $10^8$ sheep red blood cells in 0.05 ml normal saline was injected into the hind paw pad. As a control, the solvent, i.e. normal saline in equivalent amount, was injected. Reaction intensity was assessed in 24 hours by difference in the paw masses of animals.

$$\text{Reaction index} = \frac{M_t - M_c}{M_c} \times 100$$

The test compound III was orally administered for three days at doses 50 and 500 μg/kg according to the dosage regimen - days 3, 4 and 5, day 5 - administration of the anaphylaxis-provoking dose.

The data presented in table 9 show that administration of the compound at doses 50 and 500 μg/kg, significantly lowers delayed hypersensitivity intensity.

Table 9

| Effect of the compound III on the manifestation of delayed hypersensitivity induced by sheep red blood cells | | | |
|---|---|---|---|
| Group of animals | Difference in paw masses (g) | Reaction index, percent | Percent to the control |
| Control | 22.4 ± 3.7 | 13.7 ± 1.25 | 100 |
| 50 μg/kg | 16.7 ± 1.9* | 10.3 ± 1.1* | 75.5 |

(continued)

| Effect of the compound III on the manifestation of delayed hypersensitivity induced by sheep red blood cells | | | |
|---|---|---|---|
| Group of animals | Difference in paw masses (g) | Reaction index, percent | Percent to the control |
| 500 μg/kg | 17.2 ± 1.3* | 10.8 ± 0.8* | 79.3 |
| * - significance of differences in relation to the control group.<br>* - $p < 0.05$ | | | |

B. Local effect study of the compounds of general formula (I) on delayed hypersensitivity induced in mice by picryl chloride

Effect study of the compounds on the delayed type hypersensitivity reaction induced in mice by 2.4.6-trichlorobenzol (TCB) was carried out according to the method of Tarayre et al. [Tarayre J.P., Barbara M., Aliaga M., Tisne-Versilles. Comparative actions of immunosuppressants, glucocorticoids and non-steroidal antiinflammatory drugs on various models of delayed hypersensitivity and on a non-immune inflammation in mice. Arzneim.-Forsch. Drug Res. (1990), Vol. 40, pip.1125-1131].

Studies were conducted on mongrel male mice with initial body mass 30-35 g. Each group included 10 animals. Mice were sensitized with 0.1 ml 3% TCB solution in acetone application on a shaved abdominal area. In 7 days, the anaphylaxis-provoking TCB dose (0.025 ml of 3% solution) was applied on both surfaces of the right ear. In 30 minutes 0.05 ml of the test ointment were rubbed into the same surfaces. 24 hours after edema induction, mice were sacrified, ears were cut off and weighed. Allergic reaction intensity was assessed by change in weight in grams of the right ear (the test one) in relation to the left ear (the control ear).

Allergic reaction inhibition expressed in percent was calculated according to the formula:

$$100 - \left[\frac{R-L(\text{exper.})}{L} : \frac{R-L(\text{control})}{L} \times 100\right] (\%)$$

where R - the right ear weight, L - the left ear weight.

Control - intact animals, experiment - animals which were administered the compounds.

The test compounds were applied on the ear in the form of 1% and 5% ointment the base of which were 10% methylcellulose solution and propylene glycol at 1:1 ratio.

Groups of animals:

Group 1 - control, ointment base without active substance was applied on the ear of animals.
Group 2 - ointment base comprising 1% compound III was applied on the ear of animals.
Group 3 - ointment base comprising 1% compound XVII was applied on the ear of animals.
Group 4 - ointment base comprising 5% compound XLVIII was applied on the ear of animals.

The data presented in table 10, give evidence of the fact that the test compounds possess the ability to inhibit delayed hypersensitivity induced by TCB.

Table 10

| Effect of the test compounds on delayed hypersensitivity induced in mice by TCB | | | | |
|---|---|---|---|---|
| Groups of animals | Weight of years (g) | | weight increment (g) | Reaction inhibition (%) |
| | Right | left | | |
| Group 1 | 22.9 ± 1.72 | 15.0 ± 1.41 | 7.90 ± 0.56 | - |
| Group 2 | 20.35 ± 1.21 | 15.4 ± 1.12 | 4.9 ± 0.5* | 38% |
| Group 3 | 21.6 ± 1.02 | 15.5 ± 1.02 | 6.1 ± 0.59* | 24% |

(continued)

| Effect of the test compounds on delayed hypersensitivity induced in mice by TCB | | | | |
|---|---|---|---|---|
| Groups of animals | Weight of years (g) | | weight increment (g) | Reaction inhibition (%) |
| | Right | left | | |
| Group 4 | 17.9 ± 0.94 | 14.2 ± 0.98 | 3.7 ± 0.81** | 54% |
| * - significance of differences in relation to the control group<br>* - p<0.05; ** - p<0.01. | | | | |

EXAMPLE 30

STUDY OF THE COMPOUNDS' OF GENERAL FORMULA (I) EFFECT ON ACUTE INFLAMMATION DEVELOPMENT

A. The model of edema induced by Freund's complete adjuvant.

Experiment was carried out on white mongrel male rats weghing 280-300 g. The model of paw edema induced by Freund's complete adjuvant (FCA), was reproduced according to the method of Ezamuzie and Umezurike [Ezamuzie Ch., Umezurike C.C. Effect of histamine H2-receptor antagonists on acute inflammatory of the rat paw oedema. J. Pharmacol. (1989), Vol. 41, pp. 261-265]. Paw volume measurement was done using a plethysmograph (Ugo Basile). Inhibition of inflammatory reaction expressed in percent, as calculated according to the formula:

$$100 - \left[\frac{R-L(\text{exper.})}{L} : \frac{R-L(\text{control})}{L} \times 100\right] (\%)$$

where R - the right paw volume, L - the left paw volume.

Control - intact animals, experiment - animals which were administered the compounds.

The test compounds were intragastrally administered to animals through a probe four times at a dose 50 μg/kg in 0.5 ml of 1% starch solution 72, 48, 24 and 1 hour before FCA administration. The reference drug Naproxene was once administered at a dose 50 μg/kg 1 hour before edema induction. Each group included 10 animals.

Group of animals:

Group 1 - control, animals were administered 0.5 ml of 1% starch solution.
Group 2 - animals were administered the compound XLVIII.
Group 3 - animals were administered the compound III.
Group 4 - animals were administered Naproxene.

The data presented in table 11, give evidence of the fact that the compounds possess ability to inhibit FCA induced inflammatory reaction. Antiinflammatory effect intensity of the compound III is comparable to the effect of Naproxene, a total dose of which was by 25 times higher than that of the test compound.

Table 11

| Effect of the test compounds on the FCA-induced edema of paws in rats | | | | |
|---|---|---|---|---|
| Groups of animals | Volume of paws (conditional units) | | Volume increment | Inflammation inhibition (%) |
| | Right | left | | |
| Group 1 | 2.22 ± 0.85 | 1.42 ± 0.45 | 0.80 ± 0.07 | - |
| Group 2 | 1.95 ± 0.48 | 1.30 ± 0.62 | 0.65 ± 0.04* | 19% |
| Group 3 | 1.85 ± 0.62 | 1.25 ± 0.34 | 0.54 ± 0.04* | 32.5% |

(continued)

| Effect of the test compounds on the FCA-induced edema of paws in rats | | | | |
|---|---|---|---|---|
| Groups of animals | Volume of paws (conditional units) | | Volume increment | Inflammation inhibition (%) |
| | Right | left | | |
| Group 4 | 1.75 ± 0.54 | 1.30 ± 0.25 | 0.45 ± 0.02** | 44% |
| * - significance of differences in relation to the control group.<br>* - p<0.05; ** - p<0.01. | | | | |

B. Effect study of the compounds on the carrageenan edema model in rats

Experiment was carried out on white mongrel male rats weighing 280-300 g. Carrageenan-induced edema was caused according to the method of Winter et al. [[inter et al. In: De Rosa M., Giroud J.P., Willoughby D.A. Studies of the mediators of the acute inflammatory reponse induced in rats in different sites by carrageenan and turpentine. J. Pharmacol. (1971), Vol. 104, pp. 15-29]. Paw volume measurement was done using a plethysmograph (Ugo Basile) 1, 3 and 4 hours after carrageenan administration. Inhibition of inflammatory reaction expressed in percent, was calculated according to the formula:

$$100 - \left[\frac{R\text{–}L(\text{exper.})}{L} : \frac{R\text{–}L(\text{control})}{L} \times 100\right] (\%)$$

where R - the right paw volume, L - the left paw volume.

Control - intact animals, experiment - animals which were administered the compounds.

Series I of experiment. The test compounds were intragastrally administered to animals through a probe four times at a dose 50 μg/kg in 0.5 ml of 1% starch solution 72, 48, 24 and 1 hour before FCA administration. The reference drug Naproxene was once administered at a dose 20 μg/kg 1 hour before edema induction. Each group included 10 animals.

Group of animals:

Group 1 - control, animals were administered 0.5 ml of 1% starch solution.
Group 2 - animals were administered the compound III.
Group 3 - animals were administered the compound II.
Group 4 - animals were administered Naproxene.

The data presented in table 12, give evidence of the fact that the compounds in oral administration possess ability to inhibit carrageenan induced inflammatory reaction. Antiinflammatory effect intensity of the compound III is comparable to the effect of Naproxene, a total dose of which was by 10 times higher than that of the test compound.

Table 12

| Effect of the test compounds on the carrageenan -induced edema of paws in rats with oral administration | | | | |
|---|---|---|---|---|
| Groups of animals | Volume of paws (conditional units) | | Volume increment | Inflammation inhibition (%) |
| | Right | left | | |
| Group 1 | 2.12 ± 0.63 | 1.35 ± 0.23 | 0.77 ± 0.04 | - |
| Group 2 | 1.72 ± 0.27 | 1.23 ± 0.41 | 0.49 ± 0.07** | 30% |
| Group 3 | 1.92 ± 0.65 | 1.28 ± 0.28 | 0.64 ±0.05*! | 12% |

(continued)

| Effect of the test compounds on the carrageenan -induced edema of paws in rats with oral administration | | | | |
|---|---|---|---|---|
| Groups of animals | Volume of paws (conditional units) | | Volume increment | Inflammation inhibition (%) |
| | Right | left | | |
| Group 4 | 1.82 ± 0.54 | 1.34 ± 0.25 | 0.48 ± 0.02* | 37% |
| * - significance of differences in relation to the control group.<br>* * - p<0.01; *! - p<0.1. | | | | |

Series 2 of experiment. 2% carrageenan solution was injected sublaterally into paws of rats. In 1 minute 0.1 ml of gel comprising the test compounds were applied on the right paw. Gel base consisted of propylene glycol dissolved in the mixture of water and ethanol. Concentration of the compounds in the gel was 1%. Volume of paws was measured 4 hours after carrageenan administration.

Groups of animals:

Group 1 - control -1, gel without compound was applied on paw.
Group 2 - the applied gel comprised the compound XL.
Group 3 - the applied gel comprised the compound III.
Group 4 - the applied gel comprised the compound XXXVI.
Group 5 - the applied gel comprised the compound XIII.
Group 6 - control -2 -1, gel without compound was applied on paw.
Group 7 - gel comprised 1% Voltaren.

The data presented in table 13, give evidence of the fact that the compounds in cutaneous application possess ability to inhibit carrageenan-induced inflammatory reaction. Antiinflammatory effect intensity of the compounds is comparable with the effect of the reference drug Voltaren.

Table 13

| Effect of the test compounds on the carrageenan -induced edema of paws in rats with cutaneous application | | | | |
|---|---|---|---|---|
| Groups of animals | Volume of paws (conditional units) | | Volume increment | Inflammation inhibition (%) |
| | right | left | | |
| Group 1 control-1 | 2.56 ± 0.83 | 1.84 ± 0.13 | 0.72 ± 0.08 | - |
| Group 2 | 2.12 ± 0.36 | 1.87 ± 0.24 | 0.29 ± 0.08** | 63% |
| Group 3 | 2.13 ± 0.64 | 1.83 ± 0.21 | 0.30 ±0.06*l | 60% |
| Group 4 | 2.23 ± 0.42 | 1.86 ± 0.16 | 0.37 ± 0.07** | 50% |
| Group 5 | 2.29 ± 0.18 | 1.82 ± 0.34 | 0.47 ± 0.08** | 35% |
| Group 6 control-2 | 2.42 ± 0.25 | 1.56 ± 0.34 | 0.74 ± 0.06 | - |
| Group 7 | 1.99 ± 0.24 | 1.63 ± 0.26 | 0.40 ± 0.03** | 48% |
| * - significance of differences in relation to the control group.<br>**- p<0.01. | | | | |

EXAMPLE 31

ASSESSMENT OF INFLAMMATORY EFFECT OF THE COMPOUNDS OF GENERAL FORMULA (I) ON THE NON-INFECTIOUS PULMONARY GRANULOMATOSIS MODEL

Antiinflammatory and immunomodulating effect of the compound III with intragastral administration was studied on the non-infectious pulmonary granulomatosis model.

20 female 4 months old Wistar rats weighing 200-220 g were used in exepriment. Selection of Wistar rats was caused by the fact that with aerosol Sephadex A-25 administration granulomatous inflammatory process develops in lungs [Makarova O.V., Kovaleva V.L., Sladkopevtsev A.S., Mikhailova L.P., Noseikina E.M. Experimental model of non-infectious pulmonary granulomatosis (1996), No 1, pp. 76-79]. Data on the number of animals in groups, are presented in table 14.

Table 14

| Experimental groups and number of animals in them. | | |
|---|---|---|
| Order No. | Group of experiments | Number of animals |
| 1. | Control (intact animals) | 12 |
| 2. | Sephadex A-25 (day 7) | 12 |
| 3. | Sephadex A-25 + compound III | 16 |

Technique of aerosol Sephadex A-25 administration

Sephadex A-25 at a dose 5 mg per 1 kg of body mass, as administered to rats under ether anesthesia using an original dosing device for inhalation administering dry powders manufactured at the Scientific-Research Institute for Medical Instrument-Making. Wistar rats quickly recovered from anesthsia following Sephadex A-25 administration and they did not show any peculiarities in their behaviour and respiration character. Compound III was administered intragastrally using a gastric probe at a dose 50 mg per 1 kg of body mass and immediately after that, single aerosol administration of Sephadex A-25 was performed according to the earlier described technique. Then compound III was administered intragastrally once daily for 6 days at a dose 50 mg/kg of body mass in the form of water solution. Rats were examined by day 7 after commencement of administering compound III and Sephadex A-25.

Bronchoalveolar lavage was obtained under Hexenal anesthesia. Cytosis, i.e. absolute number of cells in 1 ml was determined in the bronchoalveolar lavage fluid. Endopulmonal cytogram (in percent) was calculated in the smears stained according to Romanovsky-Gimza. Following bronchoalveolar lavage procedure lungs with trachea were resected from the thoracic cavity and macropreparation was placed into 2% acetic acid solution. In 18-24 hours, trachea, main and lobar bronchi were dissected under the lense. Volume density of lymphoid follicles was estimated under the lense using the point count method. Histological examination of lungs stained with hematoxylin and eosine was performed. Volume density of alveolitis and emphysema was determined using the point count method.

Histological examination of the control group rats' (intact animals) lungs did not reveal any pathological changes. Histological examination of the lungs of Wistar rats by day 7 after aerosol effect of Sephadex A-025, morphological picture of granulomatous inflammatory process was found: mature macrophage granulomas, acute bronchitis and alveolitis with neutrophilic component, focal emphysema. Granulomas were disposed along the pulmonary connective tissue and most of them were found in the connective tissue along blood vessels, pulmonary artery branches, pulmonary veins as well as in the wall of bronchi. A little portion of small macrophage granulomas was found in the interstitium of interalveolar septa "angles", alveolar passages, respiratory and terminal bronchioles. Cellular composition of granulomas was predominantly represented by macrophages as well as by occasional neutrophils and lymphocytes. In morphometric examination of Wistar rats' lungs, the parameters of alveolitis and emphysema volume density were 7.0 $\pm$ 3.0 and 15.1 $\pm$ 5.1 %, respectively (table 15).

Table 15

| Morphometric characteristics of Wistar rats' pulmonary tissue following aerosol effect of Sephadex A-25 and in administration of the compound III | | | |
|---|---|---|---|
| Order No. | Group of Animals | Volume density in percent | |
| | | Alveolitis | Emphysema |
| 1. | Control (intact animals) | - | 1.8 $\pm$ 1.0 |
| 2. | Sephadex A-25 (day 7) | 7.0 $\pm$ 3.0 | 15.1 $\pm$ 5.1 |
| 3. | Sephadex A-25 + compound III | 3.8 $\pm$ 1.0 | 11.2 $\pm$ 3.2 |
| Significance of differences P | 1-2 | - | <0.05 |
| | 2-3 | >0.05 | >0.05 |
| | 1-3 | - | <0.05 |

According to the data of bronchoalveolar lavage fluid cytological examination in Wistar rats by day 7 following

aerosol effect of Sephadex A-25, the cytosis index rised as compared with the control one (table 16). Increase in percent content of neutrophils and lymphocytes in the endopulmonary cytogram was observed, but differences were not statistically significant.

Table 16

| Bronchoalveolar lavage cytosis and cellular composition parameters in Wistar rats following exposure to aerosol Sephadex A-25 and treatment with the compound III | | | | | | |
|---|---|---|---|---|---|---|
| No | Group of animals | | Cytosis (absolute number of cells in 1 ml) | Endopulmonary cytogram in percent | | |
| | | | | Macrophages | Lymphocytes | Neutrophils |
| 1. | Control (intact animals) | | 0.168 ± 0.050 | 95.5 ±1.1 | 3.7 ± 1.1 | 0.8 ± 0.2 |
| 2. | Sephadex A-25 (day 7) | | 0.189 ± 0.042 | 54.2 ± 9.4 | 15.3 ± 1.1 | 34.0 ± 12.0 |
| 3. | Sephadex A-25 + compound III | | 0.169 ± 0.054 | 75.2 ± 4.5 | 7.6 ± 1.5 | 16.8 ± 2.6 |
| Significance of differences P | | 1-2 | >0.05 | <0.05 | <0.01 | <0.05 |
| | | 1-3 | >0.05 | <0.05 | <0.05 | <0.05 |
| | | 2-3 | >0.05 | >0.05 | <0.01 | >0.05 |

In the group of rats treated with the compound III, histological examination of lungs also revealed the picture of granulomatous inflammation. According to morphometric examination data, prevalence of alveolitis and emphysema decreased and was 3.8±0.1 and 11.2±3.2%, resepectively, but differences were statistically insignificant (see table 15). According to cytological examination data, cytosis index in the group of rats treated with the compound III, normalized (see table 16). A significant decrease in percent content of neutrophils and lymphocytes was found in endopulmonary cytogram, however, normalization of parameters was not observed.

Effect of the compound III on pulmonary immune system was assessed by its effect on the lymphoid tissue associated with bronchi. Volume density morphometric parameters of the lymphoid tissue associated with bronchi, are represented in table 17.

Table 17

| Morphometric parameters of the lymphoid tissue associated with bronchi in Wistar rats following exposure to Sephadex A-25 aerosol and treatment with the compound III | | | |
|---|---|---|---|
| No | Group of animals | | Volume density of lymphoid follicles in percent |
| 1. | Control (intact animals) | | 15.0 ± 3.9 |
| 2. | Sephadex A-25 (day 7) | | 44.8 ± 3.7 |
| 3. | Sephadex A-25 + compound III | | 34.7 ± 10.3 |
| Significance of differences P | | 1-2 | <0.05 |
| | | 1-3 | >0.05 |
| | | 2-3 | >0.05 |

The volume density index of lymphoid follicles in Wistar rats was normally low making up 15.0 ± 3.9 %. A pronounced pulmonary lymphoid apparatus hyperplasia was noted by day 7 following exposure to Sephadex A-25 aerosol, the volume density index of lymphoid follicles being 44.8 ± 3.7 %. In the group of rats treated with the compound III, a trend to decrease in volume density of lymphoid follicles was obsrved down to 34.7 ± 10.3%.

Thus, antiinflammatory activity of the compound III was found on the pulmonary non-infectious granulomatosis induced by Sephadex A-25. According to the data of pathological, morphometric and cytological examinations, the compound III induces decrease in inflammation exudative component intensity and alveolitis prevalence as well as inhibition of lymphoid tissue hyperplasia.

EXAMPLE 27

ANTIOXIDANT ACTIVITY STUDY OF ONE OF THE COMPOUNDS OF GENERAL FORMULA (I) IN AN IN VIVO EXPERIMENT

Antioxidant activity was studied in an in vivo experiment on the model of acute toxic hepatic lesion with carbon quadrichloride ($CCl_4$).

Experiment was carried out on 40 mongrel male rats with initial body mass 190-200 g fed a standard vivarium ration. Each group included 10 animals. Hepatic lesion (experimental hepatitis) was induced in animals by intragastral administration of 0.25 ml per 100 g of body mass $CCl_4$ in the form of 50% solution in vaseline oil for 3 days [Vengerovsky A.I., Chuchalin V.S., Pauls O.V., Saratikov A.S. Effect of hepatoprotectors on lipid metabolism in hepatitis induced by Ccl4. Bulletin of Experimental Biology (1987), No 4, pp. 430-432]. The test compound III was administered intragastrally to animals at doses 50 and 500 μg/kg for three days when $CCl_4$ was administered (groups 3 and 4). Animals of the control group were administered $CCl_4$ as described above (group 2). Intact animals were orally given normal saline in an equivalent amount (group 1).

Blood and liver specimens were taken for analysis 18 hours after the last $CCl_4$ administration.

The content of primary lipid peroxidation (LP) products - diene conjugates, diene ketones and trienes - were determined using the published method [Volchegorsky I.A., Nalimov A.G., Yarovinsky B.G., Lifshits R.I. Comparison of different approaches to determining lipid peroxidation products in heptane-isopropanolic blood extracts. Voprosy Meditsinskoy Chimii (Problems of Medical Chemistry) (1989),No 1, pp. 127-131]. Content calculation of LP products was done correlating the values of respective extinctions with 1 ml of test sample.

The amount of LP final product - malonic dialdehyde (MDA) - was determined by the test with thiobarbituric acid (TBA) [Korobeinikova E.N. Modification of lipid peroxidation products in the reaction with thiobarbituric acid. Laboratornoye delo (Laboratory Matter) (1989), No 7, pp. 8-10]. Concentration of TBA-active product was calculated using regression equation. MDA content in the liver of experimental animals was assessed using the modified method [Stalnaya I.D., Garishvili T.G. The method of determining malonic dialdehyde using thiobarbituric acid. In: Modem methods in biochemistry. Moscow, Meditsina publishers. (1977), pp. 66-69], performing preliminary extraction of lipids according to Folch [Cates M. Technology of Lipidology. Moscow, Mir publishers (1975), pp. 74-76].

The results presented in table 18, give evidence of the fact that $CCl_4$ administration to rats resulted in accumulation of PL products in blood serum and liver. In animals of experimental groups which were administered the test compound, a significant decrease in hepatic content of primary LP products and serum and hepatic MDA content was noted.

Table 18

| Effect of the test compounds on serum and hepatic content of lipid peroxidation products in rats with experimental hepatitis | | | | |
|---|---|---|---|---|
| Parameters Groups of animals | Blood serum, MDA, nM/ml | Liver, MDA, nM/mg of tissue | Liver, diene conjugates, $E_{232}$/mg of tissue | Liver, diene ketones, trienes $E_{278}$/mg of tissue |
| Group 1, intact rats | 3.59 ± 0.16 | 4.68 ± 0.21 | 15.26 ± 0.48 | 7.45 ± 0.34 |
| Control of Group 2 $CCl_4$ | 5.34± 0.054**1 | 7.18± 0.43***1 | 19.12 ± 1.07*1 | 12.44 ± 0.98*1 |
| Group 3 simultaneous administration of $CCl_4$ and the compound at a dose 50 μg/kg | 4.05 ± 0.198*2 | 4.98 ± 0.13**2 | 16.25 ± 0.61 | 8.42 ± 0.25**2 |

(continued)

| Effect of the test compounds on serum and hepatic content of lipid peroxidation products in rats with experimental hepatitis | | | | |
|---|---|---|---|---|
| Parameters Groups of animals | Blood serum, MDA, nM/ml | Liver, MDA, nM/mg of tissue | Liver, diene conjugates, $E_{232}$/mg of tissue | Liver, diene ketones, trienes $E_{278}$/mg of tissue |
| Group 3 simultaneous administration of $CCl_4$ and the compound at a dose 500 μg/kg | 3.89 ± 0.11**2 | 5.01 ± 0.17**2 | 15.89 ± 0.38 | 8.24 ± 0.19*2 |
| * - significant difference. * - p<0.05; ** - p<0.01; *** - p<0.001. | | | | |

Figures 1 and 2 are the numbers of groups in relation to which differences are significant.

The results showed that the compound III of general formula (I) possesses a pronounced antioxidant effect in the model of acute toxic hepatic lesion.

EXAMPLE 33

CHANGE IN HEPATIC P-450 CYTOCHROME SYSTEM AS AFFECTED BY THE COMPOUNDS OF GENERAL FORMULA (I)

A. Effect of the compounds of general formula (I) on Hexenal sleep duration in animals

Change in Hexenal sleep (HS) duration is the in vivo measurable index of hepatic P-450 cytochrome system condition.

Studies were carried out on male mice of the lines BALB/c*h75, C57B1/6, CBA, DBA/2, first generation CBF1 and BDF1 hybrides, with initial mass 18-20 g, each group including 10 mice; and on mongrel male guinea pigs with initial mass 250-300 g, each group including 24 animals. All the test compounds (from II to LII) were administered orally in drinking water and for three days at doses 50 and 500 μg/kg. Hexenal hydrochloride at a dose 36 μg/kg of animal mass was administered 24 hours after the last drug administration, except for line BALB/c*h75 mice, which were administered Hexenal at a dose 60 μg/kg at the same terms, and guinea pigs for which Hexenal dose was 30 μg/kg. HS duration was determined in minutes.

Table 19 summarizes data on change in Hexenal sleep (HS) duration in mice of different lines, CBF1 and BDF1 hybrides, as well as in guinea pigs as influenced by the compounds of general formula (I). It was shown that the compounds decrease HS duration in experimental animals at both doses 50 and 500 μg/kg.

Table 19

| Change in Hexenal sleep duration in animals as influenced by the test compounds | | | | |
|---|---|---|---|---|
| Compound | Animals | Hexenal sleep duration (minutes) | | |
| | | Control | Doses of the compound | |
| | | | 50 μg/kg | 500 μg/kg |
| XLIV | BALB/c*h75 | 29.16 ± 5.68 | 26.50 ± 3.49 | 16.72 ± 1.27*** |
| XLIV | CBA | 21.88 ± 1.34 | 17.98 ± 1.66* | 16.13 ± 1.86** |
| XLIV | BDF1 | 23.78 ± 0.78 | - | 20.33 ± 0.59* |
| XLIV | guinea pigs | 24.76 ± 3.23 | 13.48 ± 1.02* | - |
| XXXIII | CBF1 | 25.91 ± 1.57 | - | 19.82 ± 2.12* |
| XLVIII | CBF1 | 25.91 ± 1.57 | 16.27 ± 1.26** | 20-91 ± 1.48* |

(continued)

| Change in Hexenal sleep duration in animals as influenced by the test compounds | | | | |
|---|---|---|---|---|
| Compound | Animals | Hexenal sleep duration (minutes) | | |
| | | Control | Doses of the compound | |
| | | | 50 μg/kg | 500 μg/kg |
| XLIX | CBF1 | 25.91 ± 1.57 | 19.68 ± 2.04** | - |
| III | CBF1 | 25.91 ± 1.57 | 19.61 ± 2.69** | 22.02 ± 1.45 * |
| II | the same | the same | - | 21.72 ± 1.24* |
| IV | the same | the same | 20.39 ± 1.59* | - |
| XI | the same | the same | - | 17.71 ± 0.66*** |
| VII | the same | the same | 17.62 ± 0.96* | 16.99 ± 1.33* |
| IX | the same | the same | 16.71 ± 0.94** | 20.76 ± 1.32 |
| XLVI | the same | the same | 16.91 ± 1.28** | 13.21 ± 0.70*** |
| XXXI | the same | the same | 12.59 ± 0.88*** | 12.61 ± 1.45*** |
| X | the same | the same | 13.37 ± 1.78*** | 12.01 ± 1.23*** |
| XII | the same | the same | 19.74 ± 1.94* | 17.12 ± 1.90** |
| VI | the same | the same | 16.86 ± 1.38*** | 14.39 ± 1.73*** |
| LII | the same | the same | 21.14 ± 1.39 | - |
| * - significance of differences in relation to the control group.<br>* -p<0.05; ** -p<0.01; *** -p<0.001 | | | | |

In examining changes in HS duration as influenced by all the rest compounds of general formula (I) in CBF1 mice it was shown that the substances decrease HS duration by from 13% to 54% as compared with the control.

Thus, all the tested compounds decrease Hexenal sleep duration that gives evidence of hepatic P-450 cytochrome system induction by them.

B. Change in terminal oxidase and cytochrome $B_5$ content as influenced by the compounds of general formula (I)

Change in hepatic monooxygenase system was studied on mongrel male guinea pigs with initial mass 250-300 g; each group included 12 animals. The test compound III and Phenobarbital (PB) were administered three times 72, 48 and 24 hours brfore monooxygenase system examination. The content of P-450 and $B_6$ cytochromes was measured according to the published method [Omura T., Scato R. The monooxide binding pigment of liver microsomes. 11. Solubilization, purification and properties. J. Chem. (1964), Vol. 239, pp. 2379-2385] in microsomal liver fraction, isolated using differential centrifugation method [Ahokas J., Pelkonen O., Karkin N. Charcaterisation of BP-hydroxylase of Trout-liver. Cancer Res. (1977), Vol. 37, pp. 3737-3743]. The priority method {Izotov M.V., Scherbakov V.M., Devichensky V.M. et al. The method of determining the content of P-450 cytochrome isoenzymes in liver microsomes. The inventor's certificate No 1,488,738. B.I. (1989), No 3.-6.06.09, ICI, 01. - No 33/15, No 33/48]. Microsomal protein content was determined using the modified Lowry method [Hartree E. Determination of protein: a modification of the Lowry method, that gives a linear photometric responces. Ann. Biochem. (1972), Vol. 48, pp. 422-427].

The results presented in table 20, give evidence of the fact that in guinea pigs pretreated with the test compound and PB, total hepatic content of P-450 cytochromes and $B_5$ significantly rises. At the same time, animals of groups 2 and 3 showed a significant increase in P-450B cytochromes and animals administered the test compound of general formua (I) showed also significant increase in the ratio of P-450B to P450L cytochromes and in the ratio of $B_5$ to P-450 cytochromes.

The test compound was found to change the hepatic P-450 cytochrome system in a way similar to PB, however, it has its own peculiarities, such as elevation in the ratio of P-450B to P450L cytochromes and in the ratio of $B_5$ to P-450 cytochromes.

Table 20

| Change in the hepatic P-450 cytochrome system as effected by the test compound | | | |
|---|---|---|---|
| Group of animals Parameters | Control 1 | Phenobarbital 2 | Compound 3 |
| $B_5$ cytochrome nM/mg of protein | 0.61 ± 0.02 | 1.21 ± 0.10* | 1.00 ± 0.03 * |
| P-450 cytochrome nM/mg of protein | 0.82 ± 0.07 | 1.50 ± 0.09* | 1,03 ± 0.03 |
| P-450B cytochromes nM/mg of protein | 0.43 ± 0.03 | 0.76 ± 0.08* | 0.72 ± 0.02* |
| P-450B/p-450L | 1.10 | 1.02 | 2.32** |
| $B_5$ /P-450 | 0.82 | 0.81 | 0.97* |
| * - p <0.05; ** - p<0.01 - significance of differences in relation to the control group. | | | |

EXAMPLE 34

CHANGE IN HORMONAL STATE OF ANIMALS AS EFFECTED BY THE COMPOUNDS OF GENERAL FORMULA (I)

Studies were carried out on mongrel male guinea pigs with initial body mass 250-300 g; each group included 12 animals. Sensibilization of animals was done as described in example 27. Blood for hormonal level determination was sampled in animals before commencement of experiment and 18 hours after the last drug administration from 10 to 11 hours. The substance was three times orally administered to sensitized and intact animals at a dose 50 μg/kg 72, 48 and 18 hours before the rpeated blood sampling. A comparative hormonal state analysis was carried out by individual changes in each animal in a group. Mean parametric values expressed in nM/l, are presented by the groups in table 21.

Blood serum levels of the hormones progesterone, 17-oxyprogesterone, 11-desoxycortisol and cortisol were determined by radioimmunoassay using the Beloris company kits. Hormone concentration in samples was determined by the relation graph between the activity of a precipitated bound labelled hormone and hormone concentration in the calibration samples.

Experimental results are presented in table 21. Administration of the compound XLIV to intact animals resulted in a significant rise in the levels of free cortisol (F) and of its precursors oxyprogesterone (17-OH-P) and desoxycortisol as well as a trend to increase in progesterone (P) level. In sensitized guinea pigs a significant decrease in blood serum F and 17-OH-P levels and a trend to decrease in P level were found. In sensitized animals which were administered the compound, normalization in blood serum F and desoxycortisol levels and a significant increase in P and 17-OH-P levels were noted.

In administering the compound III to both intact and sensitized experimental animals, the same hormonal state changes were observed.

Table 21

| Change in blood serum hormonal levels as effected by the test compounds | | | |
|---|---|---|---|
| Groups of animals Parameters | Sensibilization | Sensibilization + compound 50 μg/kg | Compound 50 μg/kg |
| Control<br>Cortisol<br>Experiment | 3079.1<br><br>2157.7* | 1910.3<br><br>1997.0 | 1474.5<br><br>1891.5* |
| Control<br>Progesterone<br>Experiment | 3.91<br><br>3.20 | 1.73<br><br>5.08** | 2.53<br><br>2.87 |
| Control<br>Oxyprogesterone<br>Experiment | 2.95<br><br>1.93* | 1.79<br><br>2.31* | 2.83<br><br>3.88* |
| Control<br>Desoxycortisol | 21.58 | 28.9 | 20.8 |

(continued)

| Change in blood serum hormonal levels as effected by the test compounds | | | |
|---|---|---|---|
| Groups of animals Parameters | Sensibilization | Sensibilization + compound 50 μg/kg | Compound 50 μg/kg |
| Experiment | 20.91 | 33.5 | 30.95* |
| Blood levels of hormones was calculated in nM/l.<br>* - significance of differences was calculated in relation to the individual control. * - p<0.05; ** - p<0.001 | | | |

EXAMPLE 35

EFFECT OF THE COMPOUNDS OF GENERAL FORMULA (I) ON METABOLISM OF [$C^{14}$]-ARACHIDONIC ACID IN PULMONARY TISSUE HOMOGENATE

Studies were carried out on CBA male mice fed a standard vivarium ration. Animals were administered the compound at a dose 50 μg/kg and phenobarbital at a dose 80 μg/kg for three days. Then animals were sacrified, their lungs were taken out, frozen in liquid nitrogen, homogenized in a glass homogenizer manufactured by the Wheaton company (U.S.A.) at +4˚C in 10 volumes 0.05 M Tris-HCl buffer. Aliquots (0.5 ml) of supernatant were incubated in 0.5 μC [$C^{14}$]-arachidonic acid ([$C^{14}$]-AA, Amersham, Great Britain; specific activity 50-60 μCi/mM) at +37˚C for 30 minutes. Extraction of non-metabolized [$C^{14}$]-AA and its metabolism products was performed in 20 volumes of chloroform and methanol (1:1) mixture at extraction efficacy not less than 90%, assessed using [$C^{14}$]-$PGF_{2\alpha}$ . Separation and identification of [$C^{14}$]-AA and its metabolites were performed with thin-layer chromatography (the Merck company Kieselgel 60 plates, Germany) using an organic phase of the system of solvents (ethyl acetate:isooctane:acetic acid:water -- 110: 50:20:100) and labelled standards. Densitometry of the autoradiochromatograms obtained on the X-ray films X-Omat AR (Kodak, U.S.A.) and HS 11 (ORWO, Germany) were performed on the KS 3 densiscan (Kipp and Zonnen, Holland). Quantative analysis of individual eucosanoids was carried out using radiometry of the fractions obtained with high performance liquid chromatography (the Gilson company HPLC-system, France; the Du Pont company ZORBAX C8 column, U.S.A.) and with elution of spots on TLC-plates.

Experimental results are represented in table 22. The test compound was found to stimulate production of cyclooxygenase AA metabolites and namely, it increases prostaglandin $E_2$ ($PGE_2$) synthesis, 6-keto-$PGF_{1\alpha}$ , $PGF_{2\alpha}$ , and lipoxygenase AA metabolism way, i.e. it increases production of 5-HETE and HHT. At the same time, decrease in AA metabolism is obsreved in the P-450 cytochrome system - 12-HETE and 15-HETE. It should be noted that change in the profile of AA metabolites in animals pretreated with one of the compounds of general formula (I), is similar to that which was found in animals receiving Phenobarbital - a known cytochrome P-450 system inducer.

Table 22

| Effect of the compound III on arachidonic acid metabolism | | | | |
|---|---|---|---|---|
| Groups | Control 1 | Phenobarbital 2 | Control 3 | Compound 4 |
| Fractions | percent of AA conversion | | | |
| Phospholipids | 8.17 ± 0.33 | 8.18 ± 0.21 | 7.65 ± 1.65 | 7.41 ± 0.25 |
| 6-keto-$PGF_{1\alpha}$ | 4.83 ± 0.47 | 4.12 ± 0.12 | 6.80 ± 0.56 | 7.95 ± 0.28 |
| $PGF_{2\alpha}$ | 6.27 ± 0.18 | 7.91 ± 0.08 | 6.40 ± 0.50 | 8.05 ± 0.15 |
| $TXB_2$ | 4.10 ± 0.20 | 6.21 ± 0.18 | 5.10 ± 0.30 | 5.76 ± 0.24 |
| $PGE_2$ | 2.47 ± 0.20 | 3.82 ± 0.14 | 2.40 ± 0.11 | 3.07 ± 0.11 |
| 5-HETE+HHT | 10.07 ± 0.18 | 10.5 ± 0.26 | 10.80 ± 0.31 | 13.64 ± 0.26 |
| 12-, 15-HETE | 34.80 ± 0.35 | 27.1 ± 0.11 | 38.55 ± 0.78 | 23.82 ± 0.82 |
| Unmetabolized AA | 19.97 ± 0.26 | 14.5 ± 0.28 | 2.85 ± 0.15 | 15.32 ± 1.56 |

EXAMPLE 36

CHANGE IN ANTIGEN-DEPENDENT HISTAMINE SECRETION BY PERITONEAL MAST CELLS OF SENSITIZED RATS AS EFFECTED BY THE COMPOUNDS OF GENERAL FORMULA (I)

Sensibilization of Wistar male rats with initial body mass 200-250 g was carried out according to the published method [Guschin I.S., Voitenko V.G., Sviridov B.D. et al. A polyfunctional molecule produced by the conjugation of synthetic polyionimmunostimulant with specific antigen and an inhibitor of mast cell activation. Effects on histamine release. Agents and Actions (1989), Vol. 27, pp. 75-78]. By day 14 of sensibilization according to the standard method [Fredholm B.B., Gyschin I.S., Elwin K. et al. Cyclic AMP-independent inhibition by papaverine of histamine release induced by compound 48/80. Biochem. Pharmacol. (1976), Vol. 25, pp. 1583-1588], a cellular suspension was isolated from the peritoneal cavity, and spontaneous and chicken ovalbumin (OVA)-stimulated histamine secretion by mast cells (MC) was determined as well as histamine level in mast cells of the control and experimental animals. 2 ml of cellular suspension containing 0.1-0.2 x $10^6$ MC/ml were incubated in the presence of 200 $\mu$g/kg OVA. Histamine secretion was expressed in percent to its total level. Histamine amount in samples was determined using spectrofluorimetric method [Short P.A., Burkhalter A., Cohn V.N. A method for fluorimetric assay of histamine in tissues. J. Pharmacol. Exper. Ther. (1959), Vol. 127, pp. 182-186].

The test compound was intraperitoneally administered to animals according the following dosage regimen: for 3 days prior to examining MC, a test compound at a dose 50 $\mu$g/kg was administered (group 2) or Suprastine at a dose 1000 $\mu$g/kg (group 3). Animals of the control group were intraperitonelly injected normal saline (group 1). Each group included 10 animals.

Experimental results are presented in table 23. Administration of the compound XLV of general formula (I) was shown to significantly lower antigen-dependent histamine secretion by MC. At the same time, a significant rise in spontaneous histamine secretion by MC was noted. Said changes occur against the background of decrease in histamine level in MC of sensitized animals administered one of the test compounds.

Table 23

| Change in histamine secretion by mast cells of sensitized rats as effected by the compound XLV | | | |
|---|---|---|---|
| Parameters | Treatment | | |
| | Control 1 | Compound 2 | Compound 3 |
| Spontaneous secretion, percent | 5.24 | 9.63$^{*1,3}$ | 5.62 |
| Stimulated secretion, percent | 7.16 | 2.32$^{*1,3}$ | 7.02 |
| Histamine level, $\mu$g/$10^6$ MC | 25.62 | 16.90$^{*1}$ | 16.24$^{*1}$ |
| * - $p < 0.05$ - significance of differences. Statistical processing using $\chi^2$ test. | | | |

EXAMPLE 37

EFFECT OF THE COMPOUNDS OF GENERAL FORMULA (I) ON THE PRODUCTION OF ACTIVE OXYGEN FORMS IN THE MODEL SYSTEMS

Effect of the compounds of general formula (I) on change in chemiluminescence (CL) determined by hydroxyl radical ( OH) and superoxide anion-radical ($O_2$-), in the model chemical and enzymatic systems.

Active oxygen forms (AOF) of different nature were generated in the following systems:

A. Hydroxyl radical - in the mixture of $FeSO_4$ with $H_2O_2$ (the Fenton reagent) [Halliwell B. Superoxide-dependent formation of hydroxyl radicals in the presence of iron salts. FEBS Lett. (1978), Vol. 96, pp. 238-241].
Incubation medium comprised 5 mM $KH_2PO_4$ (pH 7.4); 5 mM $FeSO_4$; 2 mM luminol. $H_2O_2$ at final concentration 5 mM was introduced into a cuvette through a dispencer following background luminescence recording of the reagents' mixture. 5 to 10 ml of the test compounds dissolved in water were at needed concentration introduced into a cuvette. A total sample volume was 0.5 ml.
B. Superoxide anion-radical in the mixture of xantine and xantine oxidase [Afanas'ev I., Suslova T., Cheremisina Z. et al. Study of antioxidant properties of metal aspartates. Analyst (1995), Vol. 120, pp. 859-862].

Incubation medium comprised 5 mM $KH_2PO_4$; 0.2 IU/ml xantine oxidase. Xantine at concentration 1 mM was

introduced into sample through a dispencer. Luceginine (0.2 mM) was used in this system as luminiscence sensitizer. The test compounds were introduced in a way similar to that in hydroxyl radical study.

Preliminary studies showed that the compounds of general formula (I) has no effect on xantine oxidase activity.

CL of the described systems was measured at 25˚C under the pulse stirring (at the moment of reagents' introduction through dispencer). CL signal indication was performed by its integration every 10 seconds for 5 minutes. CL flash recording duration after mixing ingredients, depended on a particular process kinetics. For each system, CL light sum (mV) was determined in the control samples without preparations (I-) and in samples comprising respective concentrations of preparations (I+). To estimate CL inhibition (activation) degree, the I+/I- ratio (in relative units) was found in the studied systems.

AOF formation and effect of the test compounds on this process were recorded on the Luminometer-1251 instrument (LKB, Sweden).

A. Effect of the compounds of general formula (I) on formation of hydroxyl radical in the Fenton reagent

When $H_2O_2$ was introduced into phosphate buffer comprising iron sulfate, CL flash occured which was caused by formation of predominantly hydroxyl radical in the reaction mixture. The flash had a short-term character, its maximum intensity was achieved on the 10th second and during the following 10-20 seconds luminiscence became dim and CL parameters decreased down to background values.

Table 24 summarizes data on the effect of the compounds on hydroxyl radical generation in the Fenton reagent. The results show that the test compounds inhibit hydroxyl radical formation in the studied system.

Table 24

| Effect of the compounds of formula (I) on hydroxyl radical formation | | | | |
|---|---|---|---|---|
| Compound No | $\frac{\text{Chemiluminiscence intensity (mV)} + \sigma_{n-1}}{\text{concentration of the test compounds}}$ | | | |
| | 1 mM | 0.1 mM | 0.01 mM | 1 μM |
| control | 7839 ± 171 | | | |
| XLIV | 2197 ± 311<br>0.28 (*) | 4625 ± 393<br>0.59 | 6444 ± 267<br>0.83 | 7747 ± 259<br>1.0 |
| XLV | 2195 ± 18<br>0.16 | 5800 ± 247<br>0.74 | 7443 ± 267<br>0.95 | 7666 ± 101<br>1.0 |
| control | 1120 ± 163 | | | |
| III | 164 ± 14<br>0.15 | 719 ± 71<br>0.64 | 972 ± 79<br>0.87 | 1100 ± 84<br>0.98 |
| XI | 557 ± 52<br>0.50 | - | 1124 ± 113 | 1116 ± 90 |
| VII | 165 ± 7<br>0.15 | 1158 ± 102 | - | - |
| IX | 225 ± 9<br>0.20 | 664 ± 47<br>0.59 | 1069 ± 72<br>0.95 | - |
| II | 175 ± 21<br>0.16 | 693 ± 42<br>0.62 | 772 ± 44<br>0.69 | 961 ± 42<br>0.86 |
| control | 4974 ± 283 | | | |
| XII | 189 ± 13<br>0.04 | 575 ± 69<br>0.12 | 2829 ± 184<br>0.58 | 4438 ± 285<br>0.89 |
| VI | 257 ± 74 | 3110 ± 210 | 5014 ± 186 | - |

(continued)

| Effect of the compounds of formula (I) on hydroxyl radical formation | | | | |
|---|---|---|---|---|
| Compound No | $\frac{\text{Chemiluminiscence intensity (mV)} + \sigma_{n-1}}{\text{concentration of the test compounds}}$ | | | |
| | 1 mM | 0.1 mM | 0.01 mM | 1 μM |
| | 0.05 | 0.63 | | |
| XXI | 228 ± 72<br>0.05 | 3265 ± 184<br>0.66 | 4036 ± 186<br>0.81 | - |
| XIX | 626 ± 56<br>0.13 | 3377 ± 222<br>0.68 | 3846 ± 184<br>0.77 | 49089 ± 269 |
| XX | 536 ± 124<br>0.11 | 4018 ± 201<br>0.81 | 4205 ± 234<br>0.85 | - |
| control | 1332 ± 172 | | | |
| XII[a] | 615 ± 81<br>0.46 | 1246 ± 79<br>0.94 | - | - |
| (*) - relative effect: I+/I-, where I+ - CL intensity 9mV) in the presence of substance; I- - CVL intensity in the same sample without the test substance. | | | | |

B. Effect of the compounds of general formula (I) on superoxide anion-radical formation in the xantine-xantine oxidase system

Introduction of xantine into the medium containing xantine oxidase and lucegenine, resulted in CL flash appearance which achieved the maximum for 3-5 minutes and then a very slow reduction in chemiluminescence intensity begun. Addition of the test compounds into the xantine-xantine oxidase mixture did not principally change the CL response curve shape and only the maximum CL intensity values veried. Because of such "distension" of the kinetic curve, chemiluminescence light sum was recorded during the first 3-5 minutes which reflected a total amount of light quantums produced in the system till achieving the maximum CL intensity values. As seen from the presented data (table 25) the test compounds possess ability to significantly inhibit the formation of superoxide anion-radical.

Table 25

| Effect of the test compounds on fomation of superoxide anion-radical | | | | |
|---|---|---|---|---|
| Compound No | $\frac{\text{Chemiluminiscence intensity (mV)} + \sigma_{n-1}}{\text{concentration of the test compounds}}$ | | | |
| | 1 mM | 0.1 mM | 0.01 mM | 1 μM |
| control | 5616 ± 173 | | | |
| III | 4027 ± 683 0.72 | 5670 ± 379 | - | - |
| XI | 2263 ± 278 0.40 | 4621 ± 224 0.82 | - | - |
| vII | 1114 ± 51 0.20 | 4050 ± 291 0.72 | 5082 ± 278 0.91 | - |
| control | 5391 ± 195 | | | |
| II | 3143 ± 156 0.58 | 4753 ± 89 0.88 | 5229 ± 140 0.97 | - |

(continued)

| Effect of the test compounds on fomation of superoxide anion-radical | | | | |
|---|---|---|---|---|
| Compound No | Chemiluminiscence intensity (mV) $+\sigma_{n-1}$ / concentration of the test compounds | | | |
| | 1 mM | 0.1 mM | 0.01 mM | 1 μM |
| control | 7290 ± 128 | | | |
| XII | 1926 ± 33 0.26 | 4730 ± 139 0.65 | 6856 ± 209 0.94 | - |
| VI | 902 ± 15 0.12 | 5518 ± 131 0.76 | 7295 ± 106 | - |
| XXI | 4232 ± 146 0.58 | 6460 ± 166 0.89 | - | - |
| control | 6776 ± 150 | | | |
| XII[a] | 1955 ± 155 0.29 | 2188 ± 172 0.32 | 5471 ± 421 0.81 | 6168 ± 202 0.91 |
| (*) - relative effect: 1+/I-, where I+ - CL intensity 9mV) in the presence of substance; I- - CVL intensity in the same sample without the test substance. | | | | |

The results presented in examples 24-37 show that the compounds of general formula (I) possess a pronounced antihypoxic, antiallergic and antiinflammatory activity.

EXAMPLE 38

HEPATOPROTECTIVE ACTIVITY STUDY OF THE COMPOUNDS OF GEBERAL FORMULA (I)

Hepatoprotective properties of the compounds were studied on a model of subchronic liver lesion (hepatitis) with carbon quadrichloride.

70 mongrel female rats with initial body mass 190-200 g fed a standard vivarium ration, were used in experiment. Animals were divided into 7 groups (10 rats each):

Group 1 - control one; during the first four days of experiment animals were subcutaneously injected 0.2 ml vaseline oil;
Group 2 - during the first four days animals were subcutaneously injected 50% $CCl_4$ solution in vaseline oil at a rate of 0.1 ml per 100 g body mass;
Group 3 - Legalon in a starch gel was administered orally for 8 days at a dose 30 mg/kg against the background of $CCl_4$ administration;
Groups 4 and 5 - the compound III at doses 50 and 500 μg/kg, resepectively, was administered in a way similar to group III;
Groups 6 and 7 - the compound XLIV at doses 50 and 500 μg/kg, resepectively, was administered in a way similar to group III.

The test compounds and Legalon were administered to animals 1 hour prior to $CCl_4$ administration. 24 hours after the last administration of the drugs, animals were decapitated.

Hepatoprotective activity of the test compounds was estimated by the folowing parameters:

1) in blood serum:

- activity of alanine aminotransferase (ALT) and aspartate aminotransferase (AST);
- total cholesterol (total CS);
- high density lipoprotein cholesterol (HDL-CS);
- low density lipoprotein cholesterol and very low density lipoprotein cholesterol (LDL-CS and VLDL-CS, respectively);
- triglycerides (TG);
- malonic dialdehyde (MDA);

2) in the liver:

- lipid composition: phospholipids (PL), free cholesterol (FCS), - triglycerides (TG);
- MDA.

Activity of blood serum transaminases ALT and AST was assessed using the conventional Frenkel-Rightman method [Laboratory examination methods in the clinic. Ed. by Menshikov V.V. Moscow, Meditsina publishers (1969), 302 pp.].

Blood serum total-CS level was determined according to the Ilk method [Biochemical examination in the clinic. Ed. by Pokrovsky A.A. Moscow, Meditsina publishers (1969), pp. 300-302]. HDL-CS level was measured in supernatant following heparine-manganese precipitation [Titov V.N., Brener E.D., Khaltaiev N.G., Zadoya A.A., Tvorogova M.G. A method and diagnostic significance of cholesterol level study in $\alpha$-lipoproteins. Laboratomoye delo (Laboratory Matter) (1979), No 1, pp. 36-41] using the Ilk method [Biochemical examination in the clinic. Ed. by Pokrovsky A.A. Moscow, Meditsina publishers (1969), pp. 300-302]. LDL-CS and VLDL-CS levels were calculated according to the formula [Friedwald W.T., Levy R.I, Fredrickson D.S. Estimation of the concentration of low-density lipoprotein cholesterol in plasma, without use of preparative ultracentrifuge. Clin. Chem. (1972), Vol. 18, pp. 499-502]:

$$\text{LDL–CS} = \text{total–CS} - (\text{HDL–CS} + \text{TG}/5),$$

where TG/5 corresponds to blood serum VLDL-CS level.

The published method [Rodionova L.P. Modification of blood serum trigyceride level determination method. Laboratomoye delo (Laboratory Matter) (1980), No 5, pp. 297-299] was used to determine blood serum TG level.

Blood serum LP final product MDA was determined using the published method [Korobeinikova E.N. Modification of lipid peroxydation product determination in reaction with thiobarbituric acid. Laboratomoye delo (Laboratory Matter) (1989), No 7, pp. 8-10]. Concentration of TBA-active products was calculated using regression equation:

$$C = 0.21 + 0.26D,$$

where C - concentration of TBA-active products (in nM MDA per 1 ml of serum), D - index $D_{535}$ -$D_{580}$ (in optic density units).

Total hepatic lipids were extracted using the modified Folch method [Cates M. Technology of Lipidology. Moscow, Mir publishers (1975), pp. 74-76]. Quantative content of lipid fractions was assessed with thin layer chromatography method (TLC) using the system of solvents hexane:diethyl ether:acetic acid at ratio 80:20:2. Zones of individual lipid fractions were determined using 10% alcohol solution of phosphoromolyobdenic acid which following elution were spectrophotometrically analysed at 600 nm.

Hepatic MDA level was determined using the published method [Stalnaya I.D., Garishvili T.G. Malonic dialdehyde determination method using thiobarbituric acid. In: Modem methods in biochemistry. Moscow, Meditsina publishers (1977), pp. 66-69]. MDA quantity was calculated using molar extinction coefficient value of stained trimethynic complex formed by MDA with two TBA molecules:

$$E = 1.56 \text{ x } 10^5 \text{ cm}^{-1} \text{ x } M^{-1}$$

Experimental results (table 26) give evidence of the fact that in animals treated with $CCl_4$ , a significant hyperenzymemia was noted. Administration of the compounds of general formula (I) was accompanied by normalization in blood serum ALT and AST activity, the maximum hepatoprotective effect being pronounced in administering the second substance.

Table 26

| Effect of the compounds of general formula (I) on change in blood serum transaminasee activity in animals with experimental hepatitis | | | |
|---|---|---|---|
| Groups of animals | Daily dose, mg/kg | ALT, nM/dl | AST, nM/dl |
| Control, intact animals | - | 674 ± 35 | 554 ± 27 |
| $CCl_4$ - 4 days | - | 789 ± 30* | 620 ± 11* |
| Legalon + $CCl_4$ | 30.0 | 622 ± 36** | 531 ± 22** |
| Compound III + $CCl_4$ | 0.50 | 609 ± 42** | 522 ± 33** |
| Compound III + $CCl_4$ | 0.05 | 624 ± 31** | 478 ± 26*** |
| Compound XLIV + $CCl_4$ | 0.50 | 551 ± 28** | 475 ± 19*** |
| Compound XLIV + $CCl_4$ | 0.05 | 599 ± 18** | 470 ± 17** |
| * - significance of differences with the control group data,<br>* - p <0.05<br>**, *** - significance of differences with the data of the group of animals with experimental hepatitis ($CCl_4$). ** -p<0.01; *** -p<0.001. | | | |

Obtained data analysis of blood serum lipid composition in experimental animals showed (table 27) that toxic liver lesion is accompanied by hypertriglyceridemia. Some peculiarities of cholesterol distribution among lipoprotein fractions under the effect of $CCl_4$ were noted, namely, along with rise in total cholesterol and VLDL-CS levels, HDL-CS level rised and LDL-CS level dropped. Against the background of administering Legalon and the test compounds, there was observed a trend to lowering in total cholesterol level, normalozation of LDL-CS, VLDL-CS and HDL-CS levels.

Table 27

| Effect of the compounds of general formula (I) on change in blood serum levels of cholesterol and triglycerides in animals with experimental hepatitis | | | | | |
|---|---|---|---|---|---|
| Groups of animals | Cholesterol level, mg/100 ml | | | | TG level, mg/ 100ml |
| | total-CS | HDL-CS | LDL-CS | VLDL-CS | |
| Control, intact animals | 77.6 ± 2.9 | 47.3 ± 2.1 | 18.4 ± 0.7 | 11.7 ± 0.4 | 59.5 ± 2.5 |
| $CCl_4$ - 4 days | 93.5 ± 6.0* | 60.8 ± 4.6* | 12.1 ± 1.1 | 20.4 ± 1.6* | 102.9 ± 8.1* |
| Legalon + $CCl_4$ | 90.9 ± 3.4 | 54.8 ± 2.5 | 20.1 ± 1.1** p<0.01 | 15.7 ± 1.1** p<0.05 | 78.0 ± 6.2** p<0.05 |
| Compound III, 0.5 μg/kg + $CCl_4$ | 87.0 ± 2.6 | 59.3 ± 2.6 | 12.0 ± 0.6 | 15.8 ± 1.0** p<0.05 | 78.6 ± 5.4** p<0.05 |
| Compound III, 0.05 μg/kg + $CCl_4$ | 89.5 ± 3.8 | 52.3 ± 2.9 | 19.6 ± 1.4** p<0.01 | 17.7 ± 2.0 | 88.0 ± 10.2 |
| Compound XLIV, 0.5 μg/kg +$CCl_4$ | 87.2 ± 6.5 | 57.5 ± 5.1 | 17.1 ± 1.0** p<0.01 | 12.4 ± 0.7** p<0.001 | 62.4 ± 3.9** p<0.001 |
| Compound XLIV, 0.05 μg/kg + $CCl_4$ | 85.3 ± 3.9 | 49.6 ± 2.5 | 21.9 ± 1.1** p<0.001 | 13.9 ± 1.1** p<0.01 | 68.3 ± 5.7 p<0.001 |
| * - significance of differences with the control group data,<br>** - significance of differences with the data of the group of animals with experimental hepatitis. | | | | | |

The results presented in table 28 show that $CCl_4$ administration resulted in lowering cholesterol level. The test compounds normalized FCS level and lowered TG level. At the same time, rise in phospholipid fraction was observed when the test substances were administered.

Table 28

| Effect of the compounds of general formula (I) on change in hepatic lipid composition in rats with experimental hepatitis | | | |
|---|---|---|---|
| Groups of animals | Lipid composition (in percent of total lipids) | | |
| | Phospholipids | FCS | Triglycerides |
| $CCl_4$ - 4 days | 21.3 ± 0.9 | 23.6 ± 1.2 | 23.9 ± 1.2 |
| Legalon + $CCl_4$ | 19.7 ± 1.1 | 15.0 ± 0.4** p<0.01 | 44.1 ± 1.9 |
| Compound III, 0.5 μg/kg + $CCl_4$ | 23.8 ± 1.0** p<0.05 | 18.9 ± 0.7 | 35.3 ± 0.7 |
| Compound III, 0.05 μg/kg + $CCl_4$ | 24.4 ± 0.7** p<0.05 | 20.3 ± 0.8** p<0.05 | 35.5 ±1.1 |
| Compound XLIV, 0.5 μg/kg + $CCl_4$ | 26.1 ± 0.6** p<0.001 | 20.6 ± 0.8** p<0.05 | 33.9 ± 1.0** p<0.05 |
| Compound XLIV, 0.05 μg/kg + $CCl_4$ | 22.8 ± 0.9 | 18.0 ± 0.8 | 39.9 ± 1.7 |
| * - significance of differences in relation to the control group;<br>** - significance of differences in relation to the group of animals with experimental hepatitis ($CCl_4$). | | | |

Table 29 summarized data on change in blood serum and hepatic final LP product MDA level in injury caused by $CCl_4$ and administering the test compounds. Toxic liver injury was accompanied by rise in MDA levels in both serum and liver. Treatment of animals with Legalon and the compounds of general formula (I) resulted in normalization of MDA level in the examined tissues.

Table 29

| Effect of the compounds of general formula (I) on change in blood serum and hepatic final lipid peroxidation product MDA level in rats with experimental hepatitis | | | |
|---|---|---|---|
| Groups of animals | Dose of compounds, mg/kg | Serum MDA, nM/l | Hepatic MDA, nM/l |
| Control, intact animals | - | 5.60 ± 0.60 | 3.49 ± 0.16 |
| $CCl_4$ - 4 days | - | 7.03 ± 0.38 p<0.05* | 5.98 ± 0.29* p<0.001 |
| Legalon + $CCl_4$ | 30.0 | 5.58 ± 0.15** | 4.17 ± 0.31** p<0.001 |
| Compound III + $CCl_4$ | 0.50 | 5.77 ± 0.15** p<0.05 | 4.60 ± 0.52** p<0.05 |
| Compound III + $CCl_4$ | 0.05 | 5.86 ± 0.70 | 5.00 ± 0.15** p<0.05 |
| Compound XLIV + $CCl_4$ | 0.50 | 4.72 ± 0.57** p<0.01 | 3.51 ± 0.30** p<0.001 |
| Compound XLIV + $CCl_4$ | 0.05 | 5.82 ± 0.38** p<0.05 | 4.47 ± 0.45** p<0.05 |
| * - significance of differences with the control group results;<br>** - significance of differences with the results obtained in the group of animals with experimenta; hepatitis ($CCl_4$). | | | |

Thus, the test compounds of general formula (I) possessed a pronounced antioxidant and lipoid-regulating effect which is comparable with the effect of reference drug Legalon, and by a number of parameters, they exceeded the latter. At the same time it should be noted that the test compounds were administered at doses that were by two to three orders lower than that of Legalon.

EXAMPLE 39

HYPOLIPIDEMIC ACTIVITY OF THE COMPOUNDS OF GENERAL FORMULA (I)

Hypolipidemic activity of the compounds of general formula (I) was studied on the model of experimental hyperlipidemia [Arichi H., Kumura H.O. Effects of stibens compounds of roots of polygonum cuspidatium on the lipid metabolism. Chem Pharm. Bull. (1982), Vol. 30, No 5, pp. 1766-1767] in mongrel male rats with initial body mass 220-250 g which at the background of a standard ration for 10 days were intragastarally administered oil suspension comprising 10% cholesterol and 1% cholic acid (at a rate 1 ml suspension per 100 g body mass). Each group of animals included 10 rats. The test compounds III, V, VI, VII, VIII, IX, X, XIII, XXVIII, XLVII, L, LI were orally administered to animals at doses 50 and 500 μg/kg during the last four days of experiment. As a reference drug, nicotinic acid was used which was administered at a dose 10 μg/kg to animals for 10 days at the background of atherogenic load. Blood samples were taken for analysis 18 hours after the last administration of the test substances during which period food was taken away from rats.

The following parameters were determined: total cholesterol (total CS); high density lipoprotein cholesterol (HDL-CS); low density lipoprotein cholesterol and very low density lipoprotein cholesterol (LDL-CS and VLDL-CS); triglycerides (TG). Blood serum CS level was determined according to the Ilk method [Biochemical examination in the clinic. Ed. by Pokrovsky A.A. Moscow, Meditsina publishers (1969), pp. 300-302]. HDL-CS level was measured in supernatant following heparine-manganese precipitation of LDL+VLDL [Titov V.N., Brener E.D., Khaltaiev N.G., Zadoya A.A., Tvorogova M.G. A method and diagnostic significance of cholesterol level study in α-lipoproteins. Laboratomoye delo (Laboratory Matter) (1979), No 1, pp. 36-41] LDL-CS level determination was carried out by calculation according to the formula presented in the work Friedwald W.T. [Friedwald W.T., Levy K.J., Leus R. Fat transport in lipoproteins: an integrated approach to mechanism and disorders. New Engl. J. Med. (1967), Vol. 276, p. 32].

To assess the effect of the test compounds on the ratio of atherogenic and antiatherogenic blood serum lipoproteins, cholesterol index ($K_{cs}$) was calculated according to the formula presented in the work [Klimov A.N., Nikulcheva N.G. Lipoproteins, dylipoproteinemias and atherosclerosis. Moscow, Meditsina publishers (1984), 165 pp.].

Blood serum TG level was determined using the conventional method [Friedwald W.T., Levy R.I., Fredrickson D.S. Estimation of concentration of low-density lipoprotein cholesterol in plasma, without use of preparative ultracentrifuge. Clin. Chem. (1972), Vol. 18, pp. 499-502].

Administration to animals of fat suspension was accompanied by a significant rise in blood serum CS level at the expense of atherogenic fractions of lipoproteins (LDL and VLDL) at the background of decrease in the level of antiatherogenic fraction (HDL). Three-fold rise in atherogeneity index was observed. A significant rise in blood serum TG was also noted (table 30). When the test compounds were administered to animals (table 31) at both doses, drop in total cholesterol level by 10-26 percent was noted as compared with the animals with atherogenic load, at the expense of LDL-CS and VLDL-CS lowering at significant rise in HDL-CS by 20-100 percent. Drop in cholesterol atherogeneity index $K_{cs}$ practically down to the control values, was found with administration of all the compounds. With administration of the drugs, blood TG level in animals dropped to the control values.

Table 30

Effect of nicotinic acid on the level of cholesterol and triglycerides in blood serum of experimental animals

| Groups of animals<br>Parameters | Control<br>1 | Atherogenic diet<br>2 | Atherogenic diet<br>+ nicotinic acid<br>3 |
|---|---|---|---|
| Total-CS | 68.8 ± 2.3 | 96.9 ± 10.8*1 | 65.7 ± 3.5 |
| HDL-CS | 37.5 ± 1.0 | 27.1 ± 1.3 | 42.1 ± 4.2***1.2 |
| LDL-CS + VLDL-CS | 30.9 ± 0.9 | 68.7 ± 4.8***1 | 25.7 ± 2.8***2 |
| Triglycerids | 84.9 ± 3.2 | 146.5 ± 12.9***1 | 89.4 ± 6.2***2 |
| $K_{cs}$ | 0.835 | 2.58***1 | 0.56***2 |

* - significance of differences, * - p<0.05; ** - p<0.01; *** - p<0.001. Figures signify the groups in relation to which differences are significant.

As an example of the effect of the test compounds on change in blood lipid composition, table 31 summarizes the effect results of one of the compounds of general formula (I) on blood lipid composition in animals which were given atherogenic load. Administration of these compounds were shown to normalize all the examined parameters practically

to the control values.

Table 31

| Effect of the compounds of general formula (I) on blood levels of cholesterol and triglycerids in experimental animals | | | | | |
|---|---|---|---|---|---|
| Parameters Groups | Total CS | HDL-CS | LDL-CS+ VLDL-CS | Triglycerides | $K_{cs}$ |
| Control-1 | 72,2±2,9 | 29,6±2,6 | 42,6±2,7 | 104,7±8,0 | 1,44±0,1 |
| Atherogenic diet | 93,3±3,7*** | 21,3±1,5* | 72,0±4,0*** | 261,7±11,6*** | 3,38±0,2*** |
| Compound VII, 50 μg/kg | 72,3±4,9**A | 26,0±1,9 | 46,3±3,2**A | 250,6±12,0 | 1,78±0,12***A |
| Compound VII, 500 μg/kg | 78,1±3,3* | 22,3±2,2 | 55,8±3,9**A | 251,4±12,71 | 2,5±0,17**A |
| Compound IX, 50 μg/kg | 78,4±4,4*A | 29,7±3,6*A | 48,7±4,4**A | 24,5±20,2 | 1,64±0,16***A |
| Compound IX, 500 μg/kg | 72,1±3,9**A | 20,1±1,6 | 52,0±3,4**A | 201.8±8,1***A | 2,59±0,17***A |
| | | | | | |
| Compound XIII, 50 μg/kg | 76,3±5,5*A | 23,9±1,9 | 52,4±3,9**A | 201,2±8,7***A | 2,19±0,16***A |
| Compound XIII, 500 μg/kg | 69,3±3,7***A | 27,8±2,3*A | 41,5±2,9***A | 190,0±11,7***A | 1,49±0,1***A |
| Compound XXVIII, 50 μg/kg | 85,4±4,0 | 34,6±4,6*A | 50,8±4,6**A | 157,5±12,4***A | 1,47±0,13***A |
| Compound XXVIII, 500 μg/kg | 80,7±4,8 | 47,0±6,9*A | 33,7±3,4***A | 204,1±22,1*A | 0,72±0,07***A |
| Compound X, 50 μg/kg | 87,9±7,0 | 31,8±3,3*A | 56,1±5,0*A | 218,7±17,9 | 1,76±0,16***A |
| Compound X, 500 μg/kg | 71,3±2,3***A | 38,4±4,5**A | 32,9±2,5***A | 179,2±18,3**A | 0,86±0,06***A |
| Compound VI, 50 μg/kg | 86,0±5,2 | 35,7±5,6*A | 50,3±5,5**A | 141,2±16,1***A | 1,41±0,16***A |
| Compound VI, 500 μg/kg | 70,8±3,7***A | 45,8±3,5***A | 25,0±1,6***A | 127,2+7,1***A | 0,55±0,04***A |
| Control-2 | 82,9±5,1 | 49.3±3,7 | 33,9±2,9 | 84,3±5,7 | 0,68±0,05 |
| Atherogenic diet | 100,6±4,3* | 33,8±2,5** | 66,8±3,3*** | 157,9±9,0*** | 1,98±0,1*** |
| Compound XLVII 50 μg/kg | 86,5±3,9*A | 44,1±1,9**A | 42,4±1,7***A | 90, 0±7,7***A | 0,96±0,04***A |
| Compound XLVII 500 μg/kg | 90,0±2,4 | 40,8±4,8 | 50,8±3,6**A | 116,7±10,8*A | 1,27±0,09 |
| Compound III, 50 μg/kg | 87,8±2,8*A | 46,1±2,8**A | 41,7±3,1***A | 136,4±9,7 | 0,9±0,04***A |
| Compound III, 500 μg/kg | 86,2±5,6 | 53,1±6,2*A | 33,1±2,9***A | 90,0±6,7***A | 0,62±0,05***A |
| Compound XLIV, 50μg/kg | 84,7±3,0**A | 47,3±1,1***A | 37,4±1,1***A | 85,0±6,3***A | 0,79±0,02***A |
| Control-3 | 94,1±1,2 | 45,6±2,5 | 48,5±2,4 | 112,1±10,6 | 1,06±0,03 |
| Atherogenic diet | 107,7±6,1* | 27,6±1,5*** | 80,1±3,1*** | 207,1±16,0 | 2,90±0,16***A |
| Compound V, 50 μg/kg | 103,4±2,8 | 40,0±3,1**A | 63,3±1,9*A | 181,2±12,2 | 1,58±0,09***A |
| Compound LI, 50 μg/kg | 99,1±4,5 | 39,8±1,6**A | 59,3±3,5*A | 132,1±17,4**A | 1,49±0,07***A |
| Compound VIII, 50 μg/kg | 98,8±7,2 | 31,7±2,4 | 67,1±3,1*A | 136,8±18,2*A | 2,12±0,16**A |
| Compound L, 50 μg/kg | 94,1±2,1 | 31,0±2,1 | 63,1±2,4*A | 141,6±12,6**A | 2,03±0,09***A |
| * - differences are significant in relation to the control groups,<br>*A - differences are significant in relation to the groups of animals which were given atherogenic ration.<br>* - p<O.05; ** - p<0.01; *** - p<0.001. | | | | | |

Thus, changes in blood serum lipid composition in administering the test substance to experimetnal animals

are comparable with hypolipidemic effect of the reference drug nicotinic acid (table 30).

EXAMPLE 40

EXPERIMENTAL STUDY OF HYPOGLYCEMIC ACTIVITY OF THE COMPOUNDS OF GENERAL FORMULA (I)

Experiments were carried out on Wistar male rats weighing 250-300 g. Experimental diabetes was induced with single intravenous injection of streptozotocine (the Synthez cooperative enterprise at the IMBG of the Academy of Sciences of Ukraine) at a dose 42 mg/kg to rats which were preliminarily fasting for 24 hours with access to food immediately following injection. Rats were selected for experiment 2 weeks after diabetes induction with glycemia 120-180 mg/dl. Each group included 12 animals.

The compounds were intragastrally administered to intact animals and to rats with streptozotocine diabetes for four days at daily doses 50 μg/kg and 500 μg/kg in water solution at a rate of 1 ml per 200 g of body mass. Intact animals were given the compounds III and XLIV, and rats with induced diabetes were given the compound XLIV. Check blood glucose level determination was done at the day of experiment, then animals were administered the test compounds and were deprived of food. The control animals received a respective water volume. Effect was estimated by change in blood glucose level in 2 and 5 hours. Then animals received forage and 24 hours after the last drug administration blood was again sampled for examination. 0.1 ml of blood were collected from the tail vein. Glucose level was determined using the o-toluidine method.

Glucose level was calculated in mg/dl using standard glucose solutions. Then the degree of change in glucose level in relation to the initial level was determined for each animal with its expression in percent from the initial level. Final calculation was performed for each group of animals. Each group included 10 animals.

Table 32

| Effect of two test compounds on blood glucose level in intact rats | | | | |
|---|---|---|---|---|
| Conditions of experiment | Blood glucose level (mg/dl) | | | |
| Groups | 2 hours before | 2 hours after | 5 hours after | 24 hours after |
| Control - 1 | 82.6 ± 4.1 | 58.6 ± 3.2 | 60.4 ± 2.3 | 83.8 ± 5.2 |
| Compound XLIV, 50 μg/kg | 81.2 ± 3.8 | 60.8 ± 4.0 | 64.0 ± 3.1 | 79.9 ± 5.2 |
| Compound XLIV, 500 μg/kg | 80.8 ± 2.7 | 64.8 ± 4.2 | 65.9 ± 3.9 | 83.7 ± 4.0 |
| Control - 2 | 72.6 ± 4.6 | 56.8 ± 3.8 | 65.4 ± 3.3 | 75.0 ± 3.2 |
| Compound III, 50 μg/kg | 84.2 ± 6.1 | 58.5 ± 5.9 | 70.8 ± 5.5 | 66.5 ± 3.3 |
| Compound III, 500 μg/kg | 71.3 ± 4.0 | 69.9 ± 2.8*$^2$ | 72.6 ± 1.5*$^2$ | 73.1 ± 3.4 |
| * - significance of differences in relation to the control - 2<br>* - p<0.05 | | | | |

The results presented in table 32, give evidence of the fact that compound XLIV has no effect on the curve of blood glucose level changes in intact animals following a short-term fasting period, while compound III administered at a dose 500 μg/kg, prevented drop in blood glucose level in intact rats two hours after food deprivation.

Table 33

| Effect of one of the compounds of general formula (I) on blood glucose level in animals with experimental streptozotocine diabetes | | | |
|---|---|---|---|
| Groups of animals<br>Blood glucose level | Control<br>1 | Compound III 50 μg/kg<br>2 | Compound III 500 μg/kg<br>3 |
| Initial level, mg/dl | 142.1 ± 9.9 | 146.9 ± 8.2 | 145.2 ± 10.6 |
| mg/dl in 2 hours percent from the initial level | 114.9 ± 9.3<br>80.9 ± 5.1 | 94.4 ± 5.4<br>65.4 ± 4.3* | 98.8 ± 5.6<br>67.6 ± 3.6* |
| mg/dl in 5 hours percent from the initial level | 80.5 ± 6.1<br>56.7 ± 5.0 | 64.1 ± 5.0<br>43.9 ± 4.9 | 65.1 ± 1.1<br>44.9 ± 4.5 |

(continued)

| Effect of one of the compounds of general formula (I) on blood glucose level in animals with experimental streptozotocine diabetes | | | |
|---|---|---|---|
| Groups of animals | Control | Compound III 50 $\mu$g/kg | Compound III 500 $\mu$g/kg |
| mg/dl in 24 hours percent from the initial level | 153.9 ± 3.8<br>108.4 ± 7.1 | 122.2 ± 9.2<br>83.7 ± 6.6 | 143.4 ± 10.9<br>98.9 ± 10.2 |
| * - a significant difference from the control group data, $p<0.05$. | | | |

Data presented in table 33 show that intragastral administration of compound III for four days at daily doses 50 and 500 $\mu$g/kg, results in a significant drop in blood glucose level two hours after the last drug administration to animals. Hypoglycemic antidiabetic effect was maintained 5 hours post administration, however, it was less pronounced.

Thus, the compounds of general formula (I) either have no effect or stabilize blood glucose level in intact animals and possess a glucose-lowering activity in rats with streptozotocine diabetes, which is equally pronounced in administration at both doses 50 $\mu$g/kg and 500 $\mu$g/kg.

The results presented in examples 38-40, give evidence of the fact that the compounds of general formula (I) possess a pronounced hepatoprotective effect. This effect of the compounds is explained by the fact that with their administering into the body, functional rearrangement occurs of many central body systems which participate in mainaining homeostasis.

EXAMPLE 41

EFFECT OF THE COMPOUNDS OF GENERAL FORMULA (I) ON LEWIS PULMONARY CARCINOMA GROWTH

Experiments were carried out on male mice with initial body mass 18-20 g. Each group included 10 animals. Tumor transplantation was performed according to the standard technique [Experimental estrimation of antitumor drugs in the U.S.S.R. and the U.S.A. edited by E.P.Sof ina, A.B.Syrkin, A.Goldin, A.Kline. Moscow, Meditsina publishers (1980), 296 pp.]. Administration of compounds II, III, IX, X, XII, XIV, XV, XVI, XVII, XIX, XXII, XLVII in drinking water was commenced 24 hours following transplantation and continued till the end of experiment. Effect of the compounds on Lewis rat pulmonary carcinoma (LLC) was estimated by day 12 of experiment by change in tumor volume expressed in $mm^3$.

Data presented in table 34 show that all the tested compounds inhibit LLC growth though to a different extent.

Table 34

| Effect of the compounds of general formula (I) on Lewis pulmonary carcinoma growth | | |
|---|---|---|
| Administered compound | Tumor volume ($mm^3$) | Growth inhibition percent |
| Control, intact animals | 1492 ± 230 | - |
| XXII | 985 ± 179 | 34 |
| XLVII | 826 ± 119* | 45 |
| Control, intact animals | 2633 ± 275 | - |
| III | 1249 ± 168** | 53 |
| II | 1025 ± 150** | 61 |
| IX | 2372 ± 323 | 10 |
| X | 1221 ± 209** | 54 |
| XII | 1299 ± 145** | 51 |
| XVII | 1367 ± 233** | 48 |
| Control, intact animals | 1373 ± 114 | - |
| XXI | 1023 ± 207 | 25 |
| XIX | 994 ± 173 | 28 |

(continued)

| Effect of the compounds of general formula (I) on Lewis pulmonary carcinoma growth | | |
|---|---|---|
| Administered compound | Tumor volume ($mm^3$) | Growth inhibition percent |
| XX | 1148 ± 112 | 16 |
| Control, intact animals | 2436 ± 260 | - |
| XVI | 1076 ± 69** | 56 |
| XIV | 779 ± 94** | 68 |
| XV | 889 ± 67** | 63 |
| * - significance of differences in relation to the respective control, * - p<0.02; ** - p<0.01. | | |

EXAMPLE 42

EFFECT OF THE COMPOUNDS OF GENERAL FORMULA (I) ON LEWIS PULMONARY CARCINOMA METASTASIZING

A. Antimetastatic activity study of the compounds of general formula (I)

The study was carried out on $BDF_1$ female mice with initial body mass 25-30 g. Each group included 15 animals. 0.3 ml of LLC tumor tissue suspension was intramuscularly injected to mice. Henks solution was used as a solvent. The test compounds II, III, IV, IX, X, XII, XXIII and XLIV were dissolved in water and orally administered to animals for 24 days at doses 50 and 500 μg/kg in 0.2 ml of solvent beginning from day two following tumor transplantation. By day 25 of experiment animals were sacrified, lungs were resected and fixed in Bouen solution using the MBS-9 microscope at magnification 8 x 2. 24 hours following fixation pulmonary metastatic colonies were calculated. Metastasizing index calculation was carried out in accordance with the methodological recommendations [Methodological recommendations on preclinical study of agents possessing ability to inhibit metastasizing process and to enhance efficacy of cytostatic therapy of malignant tumors. Moscow (1992) 13 pp.].

The test compounds were found to inhibit spontaneous metastasizing process of the transplantable LLC tumor in the presence of primary tumor node of different degree (by from 20 to 70 percent). Table 35 summarizes the results demonstrating a high antimetastatic activity of the substances being tested.

Activity criterion for antimetastatic drugs is their ability to inhibit metastasizing by 35 percent.

Table 35

| Antimetastatic properties of the compounds of general formula (I) in oral administration to $BDF_1$ female mice with LLC | | |
|---|---|---|
| Parameters Groups | Mean number of metastases | Metastasizing inhibition percent |
| Control - 1 | 52.2 ± 5.1 | - |
| Compound III, 500 μg/kg | 30.1 ± 2.7**[1] | 42.0 |
| Compound III, 50 μg/kg | 38.5 ±6.3**[1] | 26.0 |
| Control - 2 | 46.9 ± 8.5 | - |
| Compound XLIV, 50 μg/kg | 42.4 ± 4.5 | 10.0 |
| Compound XLIV, 500 μg/kg | 31.9 ± 3.8*[2] | 32.0 |
| Compound II, 50 μg/kg | 44.3 ± 8.0 | 5.5 |
| Compound II, 500 μg/kg | 32.9 ± 4.8*[2] | 30.0 |
| Compound VI, 50 μg/kg | 35.5 ± 4.5*[2] | 24.0 |
| Compound VI, 500 μg/kg | 39.0 ± 14.0 | 17.0 |
| Control - 3 | 50.8 ± 6.9 | - |

(continued)

| Antimetastatic properties of the compounds of general formula (I) in oral administration to $BDF_1$ female mice with LLC | | |
|---|---|---|
| Parameters Groups | Mean number of metastases | Metastasizing inhibition percent |
| Compound IX, 500 µg/kg | 38.5 ± 5.7*3 | 24.0 |
| Compound X, 500 µg/kg | 34.6 ± 7.2*3 | 30.8 |
| Compound XII, 500 µg/kg | 35.5 ± 4.6*3 | 30.1 |
| Compound XXIII, 500µg/kg | 31.5 ± 5.8*3 | 37.0 |
| * - significance of differences in relation to the respective control; * - p<0.05; ** - p<0.01 | | |

B. Compound antimetastatic activity estimation under the conditions of surgical resection of the primary tumor node

An important property of the compounds is their ability to manifest pharmacological activity on the model with resection of the primary tumor node and not to lower therapeutic efficacy of respective antitumor drugs. This fact was demonstrated in experiment on the model of spontaneously metastasizing LLC tumor transplanted into paw pad of C57BL/6 female mice weighing 20-25 g. Surgical resection of the primary tumor node was performed by day 13 following tumor transplantation. The test anitumor compounds were administered according to two dosage regimens:

Dosage regimen 1 - animals were administered compound III from day 1 till day 13 of experiment at a dose 500 µg/kg (group 1); Cyclophosphane was administered intraperitoneally two times before operation with 96 hour interval at a dose 100 µg/kg (group 2); combined administration of compound III and Cyclophosphane was similar to administration of substances in groups 1 and 2 (group 3). Number of metastases was calculated by day 29 of experiment;

Dosage regimen 2 - animals were administered compound III from day 14 of experiment (commencement of administration 24 hours post operation) till day 28 (group 4); Cyclophosphane was administered twice post operation with 96 hour interval at a dose 100 µg/kg (group 5); combined administration of compound III and Cyclophosphane similar to administration of substances in groups 4 and 5 (group 6). Antimetastatic efficacy of the drugs was considered by day 29 of experiment.

Experimental results are presented in table 36. As seen from the presented results, compound III manifested antimetastatic activity on the model with surgical resection of the primary tumor node, and in combined administration with Cyclophosphane following tumor resection potentiation of pharmacological effect was observed and summation of the tested phenomenon in administering both drugs prior to surgical resection of tumor.

Table 36

| Antimetastatic activity parameters of compound III under the conditions of surgical resection of the primary tumor node and in combined administration with Cyclophosphane | | | | |
|---|---|---|---|---|
| Experimental groups | Drugs | Metastasizing rate (%) | Mean number of metastases | Metastasizing inhibition percent |
| Administration of drugs prior to operation according to dosage regimen 1 | | | | |
| Control | Normal saline | 100 | 34.8 ± 4.9 | |
| Group 1 | compound | 100 | 32.0 ± 11.8 | 8.0 |
| Group 2 | Cyclophosphane | 100 | 19.5 ± 5.1* | 43.9 |
| Group 3 | Compound + Cyclophosphane | 100 | 16 ± 2.4*** | 52.8 |
| Administration of drugs post to operation according to dosage regimen 2 | | | | |
| Control | Normal saline | 100 | 37.1 ± 13.2 | |
| Group 4 | compound | 100 | 28.6 ± 8.5 | 22.9 |
| Group 5 | Cyclophosphane | 100 | 18.0 ± 4.3* | 51.5 |

(continued)

| Administration of drugs post to operation according to dosage regimen 2 | | | | |
|---|---|---|---|---|
| Group 6 | Compound + Cyclophosphane | 100 | 6.7 ± 0.9* | 81.9 |

B. A comparative estimation of antimetastatic sactivity of the compound III substance and its tabletted dosage form

To estimate therapeutic efficacy of compound III tabletted dosage form (0.2 g tablets 6 mm in diameter comprising 17 mg substance) studies were carried out on the model of spontaneous LLC carcinoma metastasizing.

Experiments were carried out on C57BL/6 female mice weighing 20-25 g. Experimental groups included 8 animals and the control group 11 mice. Substance and dosage form of compound III were administered orally at a dose 500 μg/kg (at a rate per an active basis) daily from day 1 till day 10 of experiment. Experiment lasted for 24 days.

Experimental results are presented in table 37. They give evidence of the fact that the parameters of antimetastatic activity of the substance and tabletted dosage form, are paractically similar.

Table 37

| Comparative therapeutic efficacy study of compound III substance and dosage form | | | | |
|---|---|---|---|---|
| Groups | Antimetastatic activity parameters | | | |
| | Metastasizing rate in percent | Tumor mass (g) | Mean number of metastases | Metastasizing inhibition percent |
| Control | 100 | 13.5 ± 0.9 | 42.0 ± 7.5 | |
| Substance | 100 | 12.9 ± 0.9 | 22.5 ± 0.9* | 46.4 |
| Dosage form | 100 | 12.9 ± 0.6 | 18.8 ± 2.7** | 55.2 |
| *,***- significance of differences in relation to the control,<br>* - p<0.05; ***- p<0.001. | | | | |

EXAMPLE 43

STUDY OF THE EFFECT OF THE COMPOUNDS OF GENERAL FORMULA (I) ON RESISTANCE OF ANIMALS TO MICROBIAL INFECTIONS

Effect of the compounds of general formula (I) on change in average lifetime of experimental animals infected with Salmonella spp.

Experiments were carried out on mongrel white mice of both sexes weighing 18-22 g. Compounds III, XLIV and the reference drug sodium nucleinate were administered orally for 3 days (a total of 6 times) prior and post infection (dosage regimen -3, -2, -1, 0, 1, 2, 3, where 0 is infection day). A 24-hour Salmonella spp. culture grown on Hottinger agar, was used for infecting. Normal saline solution was used to prepare cellular suspension. Suspension of Salmonella spp. cells was administered subcutaneously at a dose 5 x $10^8$ cells per a mouse in 0.5 ml.

The test compounds were administered at a dose 500 μg/kg and the reference drug sodium nucleinate at a dose 50 μg/kg in 0.5 ml of normal saline solution.

The test compounds at a dose 500 μg/kg significantly increased average lifetime of mice infected with Salmonella spp. (table 38) exerting a protective effect under strict experimental conditions (a hundred percent lethality in the control group). Significant rise in survival of mice administered compound III was observed by day 2 of experiment as compared with the control group, and by day one of animals which were administered compound XLIV. At the same time it was established that distribution character of lethal outcomes in animals of the control group, was abnormal, while as affected by compounds III and XLIV it became normal that confirms efficacy of the tested compounds.

Protective effect of the test compounds in relation to bacterial infection was comparable with the effect of sodium nucleinate which is a known promoter of immune responces including phagocytosis [Lazareva D.L., Alekhin E.K. Immunity stimulants (1985), 286 pp.], efficient dose of the test compounds being by two orders lower than the reference drug.

Thus, the compounds of general formula (I) possess a protective effect against microbial infection.

Table 38

| Effect pf the test compounds on resistance of mice to Salmonella spp. | | | | | |
|---|---|---|---|---|---|
| Groups of animals Days folloing infection | Number of dead mice | | | | |
| | Control 1 | Compound III | Sodium nucleinate | Control 2 | Compound XLIV |
| Day 1 | 1 | 1 | - | 6 | 3*2 |
| Day 2 | 4 | 1*1 | - | - | - |
| Day 3 | 1 | 1 | 3 | - | 1 |
| Day 4 | 1 | 2 | 3 | 2 | - |
| Day 5 | 1 | 1 | 1 | 1 | 5 |
| Day 6 | 2 | 2 | 3 | - | 2 |
| Day 7 | - | 2 | - | 1 | 1 |
| Day 8 | - | - | 1 | - | - |
| MLT (days) | 3.3 ± 0.58 | 4.5 ± 0.65* | 4.7 ± 0.48* | 2.6 ± 0.71 | 4.0 ± 0.73* |
| MLT - mean lifetime.<br>* - significance of differneces in relatrion to the control group of animals; * - p<0.05. | | | | | |

Figures signify control groups in relation to which differences are significant.

EXAMPLE 44

ANTIVIRAL ACTIVITY OF THE COMPOUNDS OF GENERAL FORMULA (I)

A. Protective effect of compound XLIV in infection caused by encephalomyocarditis virus

Studies ere carried out on mongrel mice of both sexes with initial body mass 10-11 g. The control and experimental groups included 30 animals each. Compound XLIV and the reference drug interferon inducer Ridostine were administered once intraperitoneally 24 hours after infection of mice with encephalomyocarditis virus at doses 30 μg/kg and 5.0 mg/kg, respectively. Encephalomyocarditis virus was administered at a dose 100 $LD_{50}$. Antiviral activity was determined by change in mean lifetime (MLT) of mice and by the degree of protection against lethal viral infection.

The results obtained are presented in table 39. It is shown that the test compound XLIV possesses a pronounced antiviral activity at a dose which is by two orders lower as compared with the reference drug dose.

Table 39

| Antiviral activity of the test compound XLIV | | | |
|---|---|---|---|
| Groups of animals | MLT, days | Survival rate, percent | Protection degree, percent |
| Control | 7.2 | 13.3 | 0 |
| Compound XLIV | 12.5* | 30.3* | 17.0 |
| Ridostine | 13.3* | 82.0* | 68.7 |
| * - significance of differences in relation to the control group;<br>* - p<0.05; ** - p<0.01 | | | |

B. Effect of the compounds of general formula (I) on intensity of influenzal infection in mice

Studies ere carried out on white mongrel mice of both sexes weghing 8-10 g. Strain Aichi (allantois fluid) of type A human influenza virus was intranasally administered to mice at a dose $LD_{50}$. The test compounds III, VI, VIII, XXXIII, XLVII, XLVIII were orally administered to animals for three days before infection and for ten days following infection. As the reference drugs, there were used Arbidol (a specific drug) at a dose 100 mg/kg which was administered

24 and 2 hours prior and 3 days post infection, and Tymogen (non-specific drug) at a dose 10 μg/kg administered to animals according to the same dosage regimen as the test compound. Antiviral activity was determined by MLT of mice and protection degree against lethal dose of viral infection.

The study results are presented in table 40. It was shown that increase in MLT, survival rate and protection degree are most manifested in administering compound VII at a dose 500 μg/kg. Administration of the other tested compound at doses 50 and 500 μg/kg and Arbidol was accompanied by equal change in the parameters. Effect of Tymogen as an antiviral drug, was less expressed.

Thus, the presented data give evidence of a pronounced antiviral effect of the compounds of general formula (I) in experimental viral infection in mice caused by type A human influenza virus.

Table 40

| Antiviral effect of the compounds of general formula (I) on experimental influenzal infection in mice | | | |
|---|---|---|---|
| Groups of animals, order Nos | MLT, days | Survival rate in percent | Protection degree in percent |
| 1. control - 1 | 5.76 | 16.7 | - |
| 2. Arbidol | 9.22* | 43.4* | 26.7 |
| 3. Thymogene | 8.02 | 30.0 | 13.3 |
| 4. Compound VIII, 50 μg/kg | 9.88*$^{1}$ | 40.0*$^{1}$ | 23.3 |
| 4. Compound VIII, 500 μg/kg | 14.37**$^{1}$ | 56.7*$^{1}$ | 40.0 |
| 5. Compound XLVIII, 50 μg/kg | 9.58*$^{1}$ | 34.5*$^{1}$ | 17.8 |
| 6. Control - 2 | 9.1 | 34.5 | - |
| 7. Arbidol | 13.88*$^{2}$ | 94.7*$^{2}$ | 60.2 |
| 8. Compound VI, 50 μg/kg | 11.24*$^{2}$ | 73.3*$^{2}$ | 38.8 |
| 8. Compound VI, 500 μg/kg | 11.62*$^{2}$ | 79.3*$^{2}$ | 44.8 |
| 9. Compound III, 50 μg/kg | 10.1 | 86.6*$^{2}$ | 52.1 |
| 9. Compound III, 500 μg/kg | 11.5*$^{2}$ | 76.6*$^{2}$ | 42.2 |
| 10. Compound XLVII, 50 μg/kg | 13.0*$^{2}$ | 83.3*$^{2}$ | 48.8 |
| 10. Compound XLVII, 500 μg/kg | 10.0 | 79.3*$^{2}$ | 44.8 |
| 11. Compound XXXIII, 50 μg/kg | 9.09 | 63.3 | 28.8 |
| 11. Compound XXXIII, 500 μg/kg | 11.9 | 76.6*$^{2}$ | 76.6 |
| * - significance of differences in relation to the control group;<br>* - p<0.05; ** - p<0.01. | | | |

B. Effect of the compounds of general formula (I) on human immunodeficiency virus reproduction in acute infection of lymphoblastoid cells

Study was carried out on the culture of human lymphoblastoid cells MT-4. Isolate HIV-1/IV17 of type 1 human immunodeficiency virus from the collection of the D.I.Ivanovsky Institute for Virology was used in experiment. The test compounds of general formula (I): III, VI, VII were introduced into cellular culture at concentrations 1.0; 0.1 and 0.01 μg/ml. The reference drug Azidothymidine was introduced at a dose 0.05 μM/ml. The compounds were introduced into the culture of MT-4 cells at a dose 1000 $TCD_{50}$ one hour prior to virus introduction. Viability of MT-4 cells was estimated by day 7 of experiment.

Viability of cells was the main efficacy parameter of the test compounds.

Preliminary experiments showed that the test compounds at the used concentrations are non-toxic for lymphoblastoid cells at concentrations from 1.0 μg/ml and less.

Table 41

| Effect of the test compounds on viability of MT-4 cells infected with HIV-1 | | |
|---|---|---|
| Groups | Compound dose | Viability of cells in percent |
| Non-infected cells - 1 | - | 90 |
| Infected cells - 1 | - | 10-17 |
| Azidothymidine | 0.05 μM/ml | 75*1 |
| Compound III | 0.01 μg/ml | 18 |
| Compound III | 0.1 μg/ml | 39*1 |
| Compound III | 1.0 μg/ml | 68*1 |
| Non-infected cells - 2 | - | 88 |
| Infected cells - 2 | - | 24 |
| Azidothymidine | 0.01 μg/ml | 72*2 |
| Compound VI | 0.01 μg/ml | 45*2 |
| Compound VI | 0.1 μg/ml | 49*2 |
| Compound VI | 1.0 μg/ml | 54*2 |
| Compound VII | 0.01 μg/ml | 34 |
| Compound VII | 0.1 μg/ml | 49*2 |
| Compound VII | 1.0 μg/ml | 51*2 |
| * - significance of differences in relation to the respective group of infected cells, * - p<0.01. | | |

The results presented in table 41, show that the test compounds at concentrations 1.0 and 0.1 μg/ml possess a protective effect against cytodestructive HIV-1 effect which is pronounced to a different extent, the effect of compound III being comparable with the effect of the reference drug Azidothymidine.

EXAMPLE 45

EFFECT OF THE COMPOUNDS OF GENERAL FORMULA (I) ON THE ACTIVITY OF PERIPHERAL BLOOD NEUTROPHILS

Experiments were carried out on C57BL/6 female mice with intramuscularly transplanted LLC carcinoma. Activity of neutrophils was measured by days 11 and 14.

Experimental animals were divided into four groups:

control - animals with LLC tumor, 10 animals;
animals with LLC tumor + compound III at a dose 500.0 μg/kg for 10 days. 8 animals;
animals with LLC tumor + Cyclophosphane 5 times at a dose 20.0 mg/kg (in 48 hours) for 10 days, 8 animals;
compound III and Cyclophosphane according to the above dosage regimen, 8 animals.

Experiment duration was 14 days.
Table 42 summarizes the results of neutrophils' activity study in animals of experimental groups.

Table 42

| Change in the activity of neutrophils in C57/BL/6 mice with LLC in combined and individual administration of compound III and Cyclophosphane | | | |
|---|---|---|---|
| Experimental Groups | Percent of active neutrophils | | |
| | Background | day 11 | day 14 |
| Control | 22.4 ± 3.5 | 13.0 ± 3.0 | 16.9 ± 1.8 |
| Compound III | 20.1 ± 2.7 | 9.3 ± 1.7 | 15.3 ± 4.3 |
| Cyclophosphane | 21.5 ± 3.4 | 7.6 ± 2.0 | 20.6 ± 6.0 |
| Compound III + Cyclophosphane | 21.9 ± 4.2 | 20.8 ± 5.4 | 26.0 ± 3.4* |
| * - significance of differences in relation to the control, $p<0.05$. | | | |

Development of LLC carcinomas was shown to be accompanied by decrease in the number of active forms of neutrophils in peripheral blood of experimental animals. Groups of animals which were administered combination of compound III and Cyclophosphane, were characterized by a significantly higher activity level of neutrophils as compared with the control animals.

EXAMPLE 46

EFFECT OF THE COMPOUNDS OF GENERAL FORMULA (I) ON THE ACTIVITY OF PERITONEAL MACROPHAGES

Change in the activity of peritoneal macrophages (PM) as effected by the test compounds was studied.

Experiments were carried out on mongrel white male mice with initial body mass 20-22 g. Each group included 10 animals. Compounds III, VI, XLIV were dissolved in normal saline (NS). The test compounds were administered to animals orally for three days at doses 50 and 500 μg/kg. Animals of the control group were given equal volume of NS. Mice were sacrified 20 hours after the last compound administration, peritoneal cavity was washed with 1.5 ml Henks solution comprising 5 U/ml heparine. 0.1 ml of wash solution were incubated for 30 minutes at 37˚C with 0.1 ml 0.2% nitroblue tetrasolium (NBT) solution, then smears were prepared. Nuclei were additionaly stained with o.1% neutral red solution. Results were expressed in percent of active cells [Klimov V.V., Kolovkina T.V. Nitroblue tetrasolium reduction test stimulated with pyrogenal. Laborotornoye delo (Laboratory matter) (1982), No 10, pp. 624-625]. PM activity degree as estimated by the mount of reduced NBT inclusions (formazane) in scores:

0 - no formazane inclusions, inactive PM;
1 - single formazane inclusions;
2 - inclusions fill up to 1/3 of PM cytoplasm;
3 - inclusions fill up to 1/2 of PM cytoplasm;
4 - inclusions fill up all cytoplasm.

Table 43

| Change in the activity of peritoneal macrophages as effected by the compounds of general formula (I) | | | | |
|---|---|---|---|---|
| Groups of Animals | Activity of macrophages, percent | | | |
| | 0 | 1 | 2 | 3 |
| Control | 25.3 ± 4.2 | 35.7 ± 3.6 | 32.6 ± 3.9 | 6.4 ± 1.4 |
| Compound XLIV, 50 μg/kg | 23.9 ± 4.4 | 37.3 ± 3.4 | 34.9 ± 5.9 | 5.4 ± 2.0 |
| Compound XLIV, 500 μg/kg | 17.4 ± 2.5* | 37.3 ± 3.9 | 35.8 ± 4.4 | 9.4 ± 2.9* |
| Compound III, 50 μg/kg | 11.6 ± 2.8* | 27.5 ± 2.5* | 44.6 ± 1.9* | 15.8 ± 3.3* |
| Compound III, 500 μg/kg | 12.8 ± 1.3* | 31.3 ± 2.3 | 46.0 ± 2.6* | 10.9 ± 2.1* |
| Compound VI, 50 μg/kg | 17.9 ± 1.4* | 30.9 ± 3.0 | 40.3 ± 2.1* | 10.4 ± 1.9* |

(continued)

| Change in the activity of peritoneal macrophages as effected by the compounds of general formula (I) | | | | |
|---|---|---|---|---|
| Groups of Animals | Activity of macrophages, percent | | | |
| | 0 | 1 | 2 | 3 |
| Compound VI 500 μg/kg | 20.3 ± 3.4 | 37.9 ± 3.1 | 34.0 ± 4.0 | 7.1 ± 2.0 |
| * - significance of differences in relation to the control group; * - p<0.01. | | | | |

Data presented in table 43 show that the tested compounds increase the portion of active macrophages at the expense of decrease in amount of inactive forms, the effect of compound III being pronounced in study of the both substance doses, while compound XLIV effect is more pronounced at a dose 500 μg/kg and that of compound VI - at a dose 50 μg/kg.

Thus, the tested compounds posses ability to increase the portion of active forms of macrophages but at different doses.

EXAMPLE 47

EFFECT OF THE COMPOUNDS OF GENERAL FORMULA (I) ON CHANGE IN PROSTACYCLINE TO THROMBOXANE RATIO IN MICE WITH PULMONARY CARCINOMA

Experiments were carried out on C57B1 male mice weighing 18-20 g. Lewis pulmonary carcinoma (generation 3) transplantation was performed according to a conventional technique [Experimental estrimation of antitumor drugs in the U.S.S.R. and the U.S.A. edited by E.P.Sof'ina, A.B.Syrkin, A.Goldin, A.Kline. Moscow, Meditsina publishers (1980), 296 pp.]. Each group included 8 animals. Administration of compound III at a dose 500 μg/kg per day in drinking water was commenced since 24 hours after tumor transplantation.

Determination of thromboxane $A_2$ and prostacycline was done according to the technique described in example 35. By day 24 of experiments animals were sacrified and the test parameters were determined.

Groups of animals:

Group 1 - intact control.
Group 2 - mice with transplanted Lewis carcinoma.
Group 3 - mice with transplanted Lewis carcinoma + compound III.

Table 44

| Change in arachidonic acid metabolism in mice with transplanted Lewis carcinoma as effected by compound III | | | |
|---|---|---|---|
| Groups | Prostacycline | Thromboxane $A_2$ | Ratio 6-keto-$PGF_{1\alpha}$ :$TXB_2$ |
| Group 1 | 8.9 ± 0.42 | 5.98 ± 0.30 | 1.5 ± 0.05 |
| Group 2 | 4.57 ± 0.07$^{**C}$ | 7.6 ± 0.31$^{*C}$ | 0.60 ± 0.03$^{**C}$ |
| Group 3 | 5.80 ± 0.75$^{*2}$ | 7.23 ± 0.18$^{*C}$ | 0.80 ± 0.11$^{*2}$ |
| * - significance of differences between groups: C - cjntrol group; 2 - group 2; * - p < 0.05; ** - p < 0.01. | | | |

Data presented in table 44, give evidence of the fact that in mice with transplanted Lewis carcinoma rise in thromboxane $A_2$ level by 1.4 times is found, twofold drop in prostacycline formation and prostacycline to thromboxane $A_2$ ratio are detected. Administration of compound III to animals results in rise in prostacycline formation and prostacycline to thromboxane $A_2$ ratio, as well as in drop in thromboxane $A_2$ synthesis.

EXAMPLE 48

EFFECT OF THE COMPOUNDS OF GENERAL FORMULA (I) ON THE DEVELOPMENT OF POSTSRESS CONDITIONS IN MICE

Experiments were carried out on inbred Balb male mice with initial body mass 16-18 g. Each group included

12 animals. The model of swimming in warm water for 20 minutes was used to develop emotional-locomotory stress in animals. Stress development was estimated by the activity of natural killer lymphocytes (NK cells) and by functioning of the interferon system [Sukhikh T.G., Nossik N.N., Parshina O.V., Van'ko L.V., Meerson F.Z., Yershov F.I. Relationship between the natural cellular cytotoxicity system and the interferon system in immobilization stress. Bulletin of Experimental Biology (1984), № , pp.593-595].

Activity of NK-cells was determined in the test of $H^3$-uridine release from the labelled target cells YAC-1 (mouse cellular lymphoma maintained by passages in vitro in suspension in the RPMI-1640 medium with 10% fetal serum). $H^3$-uridine (3-5 μC/cells/ml) was introduced into cellular suspension at concentration 1 miilion cells/ml on the 60th minute while shaking with 15 minute inerval and subsequent washing with the medium.

Splenocytes of mice which were obtained using the published method [Nossik N.N., Bopegamage S.A., Yershov F.I. Properties of interferons produced by different cell populations. Acta microbiologica Hungarica (1988), Vol. 35, Iss. 4, pp. 397-403] served as the source of NK-cells.

In studying interferon reaction of mouse splenocytes spleen cells were placed into incubation medium (the RPMI-1640 medium + 10% fetal calf serum), their concentration was brought to 1-3 x $10^8$ cells/ml and they were incubated at 37°C for 24 hours. Newcasle disease virus (α-interferon induction) and FGA mitogen (γ-interferon induction) were used as intereferon inducers [Nossik N.N., Bopegamage S.A., Yershov F.I. Properties of interferons produced by different cell populations. Acta microbiologica Hungarica (1988), Vol. 35, Iss. 4, pp. 397-403].

Animals were divided into four groups, each including 36 mice.

Group 1 - intact animals not subjected to stress and given normal saline (NS).
Group 2 - animals which ere subjected to stress effect and given NS.
Group 3 - animals which were subjected to stress effect and administered compound III.
Group 4 - animals which were subjected to stress effect and administered compound XLIV.

The test compounds at a dose 50 μg/kg and NS were orally administered to animals four times according to the following dosage regimen: for two days before exposure to stress (first and second administrations), two hours prior to stress (third administration) and 24 hours after third administration (fourth administration).

Activity of NK-cells and interferon state were dynamically determined in 4 hours following stress, in 24 hours and then by days 5, 7 and 10.

Table 45

| Change in activity of NK-cells as effected by the compounds of general formula (I) | | | | | |
|---|---|---|---|---|---|
| Groups of animals | Time after stress, days | | | | |
| | 0.16 | 1 | 5 | 7 | 10 |
| 1. Intact control | 81 ± 0.82 | 79 ± 1.32 | 79 ± 0.99 | 76 ± 0.66 | 80 ± 0.99 |
| 2. Stress | 57.3 ± 0.82***[1] | 39 ± 48***[1,3] | 68.3 ± 1.65 | 76.5 ± 0.49 | 71.7 ± 2.46*[1] |
| 3. Stress + compound III | 68.5 ± 0.99*[1,2] | 69.3 ± 1.81***[2] | 79 ± 1.46 | 69.3 ± 4.85 | 77 ± 1.98 |
| 3. Stress + compound XLIV | 69.5 ± 1.32**[2] | 61.5 ± 2.64*[1,2] | 71 ± 0.82 | 67.7 ± 3.95 | 77.5 ± 1.65 |
| *, ** - significance of differences; figures signify groups in relation to which differences are significant; * - p < 0.05; ** - p<0.01 | | | | | |

Results presented in table 45 show that administration of the test compounds prevents drop in activity of NK-cells caused by stress. In addition, administration of the compounds to animals results in normalization of α-interferon production and rises γ-interferon production (table 46).

Table 46

| Effect of the test compounds of general formula (I) on α-interferon and γ-interferon production by splenocytes of mice under poststress conditions | | | | | | |
|---|---|---|---|---|---|---|
| Groups of animals | Time after stress, days | | | | | |
| | 0 | 0.16 | 1 | 5 | 7 | 10 |
| α-interferon, U/ml | | | | | | |
| 1. Intact control | 160 | 160 | 80 | 160 | 160 | 160 |
| 2. Stress | 160 | 10 | 10 | 16 | 32 | 16 |
| 3. Stress + compound III | 160 | 10 | 10 | 160 | 160 | 160 |
| 4. Stress + compound XLIV | 160 | 10 | 80 | 80 | 100 | 140 |
| γ-interferon, U/ml | | | | | | |
| 1. Intact control | 40 | 40 | 32 | 40 | 40 | 40 |
| 2. Stress | 80 | 20 | 40 | 40 | 10 | 10 |
| 3. Stress + compound III | 80 | 80 | 32 | 32 | 320 | 80 |
| 4. Stress + compound XLIV | 80 | 20 | 320 | 40 | 32 | 32 |

Interferon production by splenocytes of 8 mice per every measurement point.

Thus, in animals which were administered the test compounds, consequences of stress reaction were less pronounced than in mice which were not administered them.

EXAMPLE 49

EFFECT OF THE COMPOUNDS OF GENERAL FORMULA (I) ON MITOGEN-INDUCED BLASTTRANSFORMATION OF LYMPHOCYTES

Human lymphocytes were used for blasttransformation reaction [Kiseleva E.P., Tsveibakh A.S., Goldman E.I., Pigareva N.V. Application of a micromethod to study blasttransformation of lymphocytes in humans and animals. Immunology (1985), No 1, pp. 76-78]. 10 ml blood were collected into a sterile syringe containing heparine (to final concentration 25 U/ml) and incubated to sediment red blood cells (60-90 minutes at 37˚C). Leukocytic suspension layer was selected and the number of nucleated cells was calculated. The cells obtained in such a way were incubated for 72 hours in a humid atmosphere with 5% $CO_2$ at 37˚C in the cells of plates for immune reactions in RPMI-1640 medium comprising 15% fetal calf serum, 50 μg/kg Neomycine (incubation mixture volume 0.15 ml). 5 x $10^5$ cells were introduced into each plate cell. Incubation was carried out in the presence or absence of mitogen phytohemagglutinin (PHA) (Sigma) (13.3μg/kg) or concanavalin A (ConA) (Sigma) (13.3μg/kg) with or without addition of the test compounds at different concentrations. 2 mCi $H^3$ -thymidine in RPMI-1640 medium were introduced into each cell 16 hours before incubation termination. Following culturing period termination, 0.15 ml cellular suspension from each plate cell were transferred on the FN-8 paper filters. Filters were dried, washed two times with normal saline for 5 minutes then twice with TCA for 60 minutes. After drying, radioactivity of samples was calculated on a counter in the ZhS-8 scintillator. Stimulation index was calculated as ratio of sample radioactivity with mitogen to sample radioactivity without mitogen.

The results presented in tables 47-49, give evidence of a stimulating effect of compounds XLIV and III on blasttranformation reaction of human lymphocytes, enhancement of the reaction induced by both PHA and ConA being

observed.

Table 47

| Effect of compound XLIV on PHA- induced blasttranformation of human lymphocytes | | | | |
|---|---|---|---|---|
| No | Compound XLIV concentration, M/l | Mitogen | Radioactivity, number of pulses for 20 seconds | Stimulation index |
| 1 | Control | - | 139 ± 51 | |
| | | +PHA | 3085 ± 166 | 22.2 |
| 2 | $10^{-8}$ | - | 261 ± 100 | |
| | | +PHA | 3830 ± 286* | 14.7 |
| 3 | $10^{-7}$ | - | 300 ± 107 | |
| | | +PHA | 2960 ± 210 | 9.9 |
| 4 | $10^{-6}$ | - | 150 ± 33 | |
| | | +PHA | 4414 ± 570* | 29.6 |
| 5 | $10^{-5}$ | - | 132 ± 13 | |
| | | +PHA | 2999 ± 213 | 23.2 |
| 6 | $10^{-4}$ | - | 301 ± 103 | |
| | | +PHA | 3276 ± 225 | 10.9 |

Table 48

| Effect of compound III on concanavalin A - induced blasttranformation of human lymphocytes | | | | |
|---|---|---|---|---|
| No | Compound III concentration, M/l | Mitogen | Radioactivity, number of pulses for 20 seconds | Stimulation index |
| 1 | Control | - | 317 ± 88 | |
| | | +ConA | 941± 113 | 2.97 |
| 2 | $10^{-8}$ | - | 234 ± 36 | |
| | | +ConA | 849 ± 133 | 3.63 |
| 3 | $10^{-7}$ | - | 478 ± 35 | |
| | | +ConA | 1483 ± 67** | 3.10 |
| 4 | $10^{-6}$ | - | 408 ± 75 | |
| | | +ConA | 1922 ± 248** | 4.71 |
| 5 | $10^{-5}$ | - | 467 ± 109 | |
| | | +ConA | 1562 ± 134** | 3.34 |
| 6 | $10^{-4}$ | - | 274 ± 28 | |
| | | +ConA | 1197±97 | 4.37 |
| 7 | $10^{-3}$ | - | 330 ± 105 | |
| | | +ConA | 680 ± 82 | 2.06 |

Table 49

| Effect of compound XLIV on concanavalin A - induced blasttranformation of human lymphocytes | | | | |
|---|---|---|---|---|
| No | Compound XLIV concentration, M/1 | Mitogen | Radioactivity, number of pulses for 20 seconds | Stimulation index |
| 1 | Control | - | 361 ± 43 | |
| | | +ConA | 1061 ± 243 | 2.94 |
| 2 | $10^{-8}$ | - | 233 ± 54 | |
| | | +ConA | 971 ± 44 | 4.17 |
| 3 | $10^{-7}$ | - | 242 ± 19 | |
| | | +ConA | 1786 ± 241* | 7.38 |
| 4 | $10^{-6}$ | - | 289 ± 52 | |
| | | +ConA | 1963 ± 205* | 6.96 |
| 5 | $10^{-5}$ | - | 339 ± 32 | |
| | | +ConA | 1695 ± 139* | 5.00 |
| 6 | $10^{-4}$ | - | 322 ± 38 | |
| | | +ConA | 1259 ± 90 | 3.91 |
| 7 | $10^{-3}$ | - | 356±51 | |
| | | +ConA | 1072 ± 135 | 3.01 |

The data presented in examples 41-49, give evidence of the fact that the test compounds possess immunomodulating activity, significantly inhibit the growth of the transplantable tumor and the process of its metastasizing as well as increase resistivity of animals to microbial and viral infections.

All the above mentioned studies show that the compounds of the present invention show the above mentioned biological efficacy at doses which are by 2 to 3 orders lower than the doses of the known drugs used for comparison in practically similar efficacy.

Further, examples of dosage forms are presented in which the compounds of general formula (I) can be used.

EXAMPLE 50

EXAMPLES OF DOSAGE FORMS

A. Gelatine capsules.

Composition of the powder introduced into a capsule:

| | |
|---|---|
| A Compound of general formula (I) | 1-35 mg |
| Magnesium oxide | 50 mg |
| Starch | 100-200 mg, |

The above indicated ingridients are mixed and the mixture is introduced into solid gelatine capsules at amount 151-285 mg.

B. Tabletted dosage form.

Tabletted dosage form is prepeared using the above mentioned ingredients:

| | |
|---|---|
| Compound of general formula (I) | 1-35 mg, |
| Potato starch | 100 mg |
| Polyvinyl pyrrolidone | 10 mg |

(continued)

| | |
|---|---|
| Magnesium stearate | 2 mg |
| Lactose | 48-82 mg |
| Aerosyl | 5 mg |

Ingredents are stirred and pressed for production of tablets weighing 200 mg each.

B. Aerosolic dosage form.

Composition of aerosolic mixture calculated per 10 administrations:

| | |
|---|---|
| Compound of general formula (I) | 10-40 mg |
| Magnesium sulphate | 150 mg |
| Lactose | 110-140 mg |

The compound is mixed with excipients and placed into a special device for spraying.

D. Suppositoria.

As the suppositorial bases the following bases can be used: water-unsoluble bases - cocoa oil;

water-soluble bases or bases mixable with water - gelatine-glycerinic or polyethylene oxide bases;
combined bases - soapy-glycerinic.

Example of suppositorium composition:

| | |
|---|---|
| Compound of general formula (I) - | 1-35 mg |
| Cocoa oil - | amount needed to prepare a suppositorium. |

If needed, rectal, vaginal and urethral suppositoria with respective excipients can be produced.

E. Ointments.

The following compounds can be used as ointment base:

carbohydrate ointment bases - white and yellow vaseline (Vaselinum album, Vaselinum flavum), vaseline oil (Oleum Vaselini), white and yellow ointment (Unguentum album, Unguentum flavum), and as supplements to confer a firmer consistence, hard paraffine and wax can be used;
absorbtive oinment bases - hydrophylic vaseline (Vaselinum hydrophylicum), lanoline (Lanolinum), coldcreme (Unguentum leniens);
ointment bases washable with water - hydrophylic ointment (Unguentum hydrophylum); water-soluble ointment bases - polyethylene glycol ointment (Unguentum Glycolis Polyaethyleni), bentonite bases and others.

Example of ointment composition:

| | |
|---|---|
| A compound of general formula (I) | 0.1-0.5 g |
| Vaseline | 10 g |

Ointments are produced according to the respective technology.

F. Solution for injections.

The following substances can be used as solvents to prepare solution for injections: 0.9% sodium chloride

solution, distilled water, novocaine solution. Dosage forms: ampules, vials, syringes-tubes, inserts.

Composition of solution for injections:

| | |
|---|---|
| A compound of general formula (I) - | 1-5 mg |
| Distilled water - | 1-2 ml |

Production of different dosage forms for injections is possible: sterile solutions, sterile powders and tablets.

EXAMPLE 51

VERSIONS OF APPLYING THE COMPOUNDS OF GENERAL FORMULA (I) AS COMPONENTS OF COSMETIC AGENTS

A. Lotions.

Composition of a therapeutic-cosmetic lotion:

| | |
|---|---|
| A compound of general formula (I) | 0.1-1 g |
| Flavor - | 0.5-1.5 g |
| Ethyl alcohol $60^0$ - $70^0$ - | 100-150 ml |

Lotions are produced according to conventionasl technology with pH parameters 5.5-6.0.

G. Creams.

Composition of a therapeutic-cosmetic cream:

| | |
|---|---|
| A compound of general formula (I) - | 0.1-1 g |
| Olive oil - | 8-10 g |
| Triethanol amine - | 1 g |
| Glycerine - | 3-5 g |
| Sodium benzoate - | 1-2 g |
| Flavor - | 1-1.5 g |
| Lanoline - | 20-25 g |
| Water - | up to 100 g |

Creams are produced according to the respective technology.

## Claims

1. Derivatives of peptides of general formula I

$$R_1\text{-}(CH_2)_n\text{-}CONH\text{-}\underset{\displaystyle R_2}{\underset{|}{CH}}\text{-}(CH_2)_m\text{-}R_3 \text{ (I)},$$

or pharmaceutically acceptable salts thereof, where $R_1$ is a hydrogen atom or $C_1$ - $C_3$ - hydrocarbon radical substituted for a functional group selected from amino-, $C_1$ - $C_5$ - amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; or $C_1$ - $C_3$ - hydrocarbon radical substituted for indole residue or 5-6 membered saturated or unsaturated cyclic or heterocyclic group, hydrocarbon radical possibly simultaneously comprising amino group, free or substituted for an acyl substitute or ether of carbonic acid; $R_2$ is hydrogen atom or a functional group selected from carboxyl which can be etherified; $R_3$ is indole or methyl and/or hydroxyl derivative thereof, hydroxyl group possibly being acylated, alcylated or aralcylated; 5-6 membered saturated or unsaturated cyclic or heterocyclic groups comprising oxygen, sulfur and/or 1-3 nitrogen atoms or methyl derivatives thereof; hydrogen atom or $C_1$ - $C_3$ - hydrocarbon radical substituted for a functional group selected from amino-, $C_1$ - $C_5$ - amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; n=0-4, m=1-5 provided that when $R_1$ = $-NH_2$, n = 2-3, m=1, $R_2$ =H, then $R_3$ does not signify -4-imidazolyl, -3-(5-Ome-indolyl). -3-(5-OH-indolyl); when $R_1$ =$-NH_2$, n=4-5. $R_2$ =-H, then $R_3$ does not signify -4-imidazolyl: when $R_1$ = $-NH_2$, n = 4-5, m=1, $R_2$ =-H. then $R_3$ does not signify -4-imidazolyl; when $R_1$ = $-NH_2$, n = 2-3, m=1, $R_2$ =-COOH, then $R_3$ does not signify -4-imidazolyl; when $R_1$ = - $NHCOCH_3$, n = 2, m=1, $R_2$ =H, -COOH, then $R_3$ does not signify -4-imidazolyl; when $R_1$ = -HOOC-CH ($NH_2$) -, n = 2, m=1, $R_2$ =H, -COOH, then $R_3$ does not signify -4-imidazolyl, -3-indolyl, -3-(5-OH-indolyl); when $R_1$ = HOOC-CH ($NH_2$)-, n = 1, m=1, $R_2$ =-COOH, then $R_3$ does not signify -4-imidazolyl; when $R_1$ = $-NH_2$-CH ($[CH_2]_k$ COOH)- n = 0, k=1-2, m=1, $R_2$ =COOH, then $R_3$ does not signify -4-imidazolyl; when $R_1$ = $-NH_2$-CH ($[CH_2]_2$ COOH)-, n = 0, m=1, $R_2$ =H, then $R_3$ does not signify -4-imidazolyl; when $R_1$ = $-CH_3$-CONH-CH (COOH)-, n = 1, m=1, $R_2$ =H, then $R_3$ does not signify -4-imidazolyl, -3-indolyl, -3-(5-OH-indolyl); when $R_1$ = $-CH_3$-CONH-CH (COOH)-, n = 2, m=1, $R_2$ =-COOH, then $R_3$ does not signify -4-imidazolyl; when $R_1$ = - $CH_3CONH$-CH ($CH_2COOH$)-, n = 0. m=1, $R_2$ =H, then $R_3$ does not signify -4-imidazolyl, 3-indolyl, -3-(5-OH-indolyl); when $R_1$ =Ry-NH-CH (Rx-$CH_2$-)-, n = o, m=1, $R_2$ =-COOH, where Rx = -4-imidazolyl, -3-indolyl, Ry= Boc-, Z-, H-, then $R_3$ does not signify -4-imidazolyl, -3-indolyl; when $R_1$ = o-, m, -$\pi$-$C_5H_4N$-, n = 0, m=1, $R_2$ =H, then $R_3$ does not signify -3-indolyl, -3-(5-Ome-indolyl); when $R_1$ = - COOH, n = 1-2, m=1, $R_2$ =-COOH, then $R_3$ does not signify -3-indolyl; when $R_1$ -CO- = pGlu-, n = 0, m=1, $R_2$ =H, -COOH, -$COOCH_3$, then $R_3$ does not signify -4-imidazolyl; when $R_1$ -CO-= pGlu-, n = 0, m=1, $R_2$ =COOH, then $R_3$ does not signify -3-indolyl; when $R_1$ -CO- = Pro-, n = 0, m=1, $R_2$ =H, then $R_3$ does not signify -4-imidazolyl; when $R_1$ -CO- = Pro-, n = 0, m=1, $R_2$ =COOH, then $R_3$ does not signify -4-imidazolyl, -3-indolyl;

**2.** Derivatives of peptides according to claim 1,
where $R_1$ =$NH_2$ -, n=2÷5; $R_1$ =HOOC -, n=1÷4; $R_1$ =Rz-OCO -, n=1÷4; Rz= -H or $C_1$ - $C_3$ - hydrocarbon radical; $R_1$ =HOOC-$CH_2$ - ($CH_3$) C (Rv) -, Rv= H, OH, $CH_3$; $R_1$ =$C_6H_5CH_2$-OCO-NH-, n=2÷3; $R_1$ =Rx-CONH-, n=2÷5, Rx= $C_1$ - $C_3$ - hydrocarbon radical;; $R_1$ = $CH_3CONH$-CH(COOH) -, n=1÷2; $R_1$ = $CH_3CONH$-CH($[CH_2]_k$ COOH)-, n=0, k=1÷2; $R_1$ = $NH_2$ -CH($[CH_2]_k$ COOH)-, n=0, k=1÷2; $R_1$ = HOOC-CH($NH_2$) -, n=0, k=1÷2; $R_1$ = HOOC-CH ($NH_2$)-, n=1÷2; $R_1$ = $CH_3$ OOC-CH ($NH_2$)-, n=1÷2; $R_1$ = $(CH_3)_3C$-OCONH-CH ($COOCH_2$-$C_6H_5$)-, n=1÷2; $R_1$ = -4-imidazolyl, -3-indolyl, n=1÷2; $R_1$ = Rb-$CH_2$-CH (NHRy)-, Rb= -4-imidazolyl, -3-indolyl, Ry= Boc-, Z-, H-, n=0; $R_1$ = $-CH_3$ , n=3-5; $R_1$ = cyclo-$C_6H_{11}$, n=0; $R_1$ = o,m,$\pi$-$C_5H_4N$-, n=0; $R_1$ -CO-= pGlu-, n=0; $R_1$ -CO-= Pro-, homo-Pro-, n=0;
$R_2$ =-H, -COOH, -COORz, Rz= -H or $C_1$ - $C_3$ - hydrocarbon radical,

$R_3$= 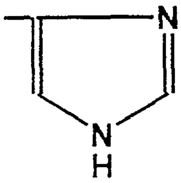, 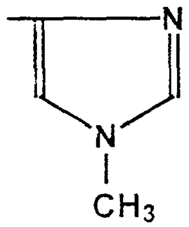, 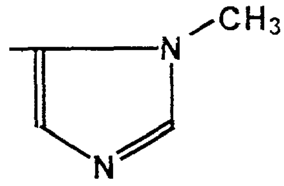, 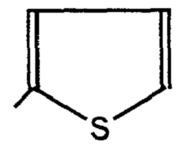

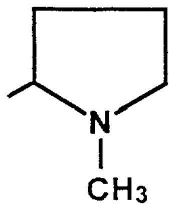, 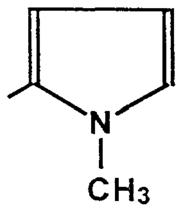, 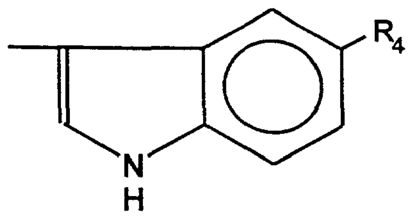, 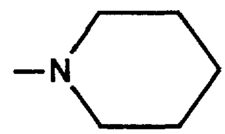,

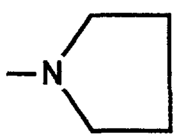, 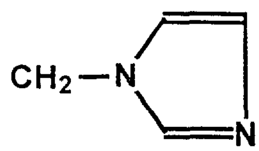, 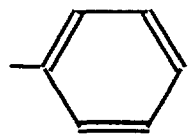, 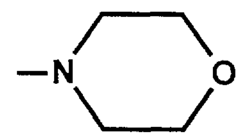, 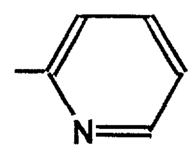

m=1; $R_3$ = $-CH_3$, m=1-5; $R_3$ = $-NH_2$, m=1-3; $R_3$ = -COOH, m=0-3; $R_3$ = $-CH(NH_2)-COOH$, m=0-2, where $R_4$ = -H, -OH, $-OCH_3$, $-OCH_2\ C_6H_5$.

3. A method to produce derivatives of peptides of general formula I,

$$R_1\text{-}(CH_2)_n\text{-CONH-}\underset{\displaystyle R_2}{\underset{|}{CH}}\text{-}(CH_2)_m\text{-}R_3 \text{ (I)},$$

or pharmaceutically acceptable salts thereof, where $R_1$ is a hydrogen atom or $C_1$ - $C_3$ - hydrocarbon radical substituted for a functional group selected from amino-, $C_1$ - $C_5$ - amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; or $C_1$ - $C_3$ - hydrocarbon radical substituted for indole residue or 5-6 membered saturated or unsaturated cyclic or heterocyclic group, hydrocarbon radical possibly simultaneously comprising amino group, free or substituted for an acyl substitute or ether of carbonic acid; $R_2$ is hydrogen atom or a functional group selected from carboxyl which can be etherified; $R_3$ is indole or methyl and/or hydroxyl derivative thereof, hydroxyl group possibly being acylated, alcylated or aralcylated: 5-6 membered saturated or unsaturated cyclic or heterocyclic groups comprising oxygen, sulfur and/or 1-3 nitrogen atoms or methyl derivatives thereof: hydrogen atom or $C_1$ - $C_3$ - hydrocarbon radical substituted for a functional group selected from amino-, $C_1$ - $C_5$ - amido-. $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; n=0-4, m=1-5 including

amino group acylation of the amino compound of general formula $NH_2$ -CH ($R_2$) - $(CH_2)_m$ - $R_3$, activated by carboxylic group with the compound of general formula $R_1$ - $(CH_2)_n$ -COX, where X is the activating group.

**4.** A method to produce derivatives of peptides of general formula (I) according to claim 3, **characterized in that** when $R_1$ = HOOC-, n=1÷4; $R_1$=HOOC-$CH_2(CH_3)$ C (Rv)-, n=1, Rv= H, $CH_3$; $R_2$=-H or -$COOCH_3$, as an activated aminocompound anhydrides of dicarbonic acids are used and the process is carried out in an organic solvent.

**5.** A method to produce derivatives of peptides of general formula (I) according to claim 3, **characterized in that** when $R_1$ = -$NH_2$, n=2-3; $R_2$=-H,

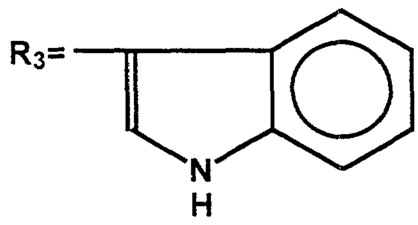
,

m=1,
when $R_1$ =$NH_2$ -, n=2-3 or $R_1$ =HOOC -CH ($NH_2$)-, n=1-2; $R_2$ =-$COOCH_3$, -H,

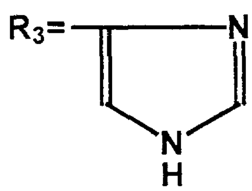
,

m=1,
as an acylating agent, there are used pentafluor phenyl ethers of N-benzyl oxycarbonyl-α-alanine or α-benzyl ether of N-tert-butyl oxycarbonyl-L-glutamic or aspargic acid and the process is carried out in an organic solvent with subsequent cleaving off a protective group andα-benzyl ether from the protected derevatives of dipeptides using catalytic hydrogenolysis with subsequent cleaving off N-tert-butyl oxycarbonylic group in unhydrous acidic medium.

**6.** A method to produce derivatives of peptides of general formula (I) according to claim 3, **characterized in that** when $R_1$ =$CH_3CONH$-CH (COOH)-, n=1-2, $R_1$=$CH_3CONH$-CH($[CH_2]_k$ COOH)-, n=0, k=1÷2, $R_1$=$CH_3CONH$-, n=3-5, $R_2$=H,

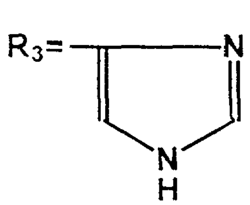
,
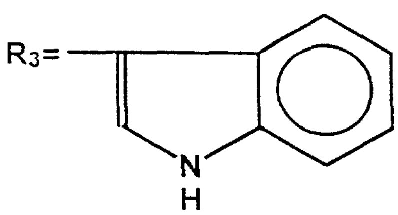
,

m=1, p-nitrophenyl acetate is used as an acylating agent.

**7.** Derivatives of peptides according to claim 1, possessing antioxidant, antiradical, lipid regulating, hypoglycemic, antiinflammatory, antiaggregate, immunomodulating effects as well as ability to induce the P-450 cytochrome system, to modulate the metabolism of arachidonic acid and adrenocortical hormones, to lower the level and antigen-dependent histamine secretion by peritoneal mast cells, to modulate the activity of macrophages, natural killers and the interferon system (of cytokines).

**8.** Derivatives of peptides according to claim 1, possessing antiallergic activity as well as activity related to controlling the signs and preventing asthma and pulmonary emphysema.

**9.** Derivatives of peptides according to claim 1, possessing wound healing properties and activity related to controlling the signs of cutaneous lesions and skin diseases, for instance, of psoriasis, eczema as well as varicose veins.

**10.** Derivatives of peptides according to claim 1, possessing activity related to preventing dysfunctional disorders including imminent abortion, dysfunctional uterine bleedings, amenorrhea etc.

**11.** Derivatives of peptides according to claim 1, possessing antihypoxic and antiatherosclerotic activity as well as activity related to controlling the signs of atheroselerosis, ischemic disease, obesety and diabetes mellitus.

**12.** Derivatives of peptides according to claim 1, possessing hepatoprotective properties and activity related to controlling radiation lesions, hepatic lesions including toxic lesions, hepatitis, cirrhosis and alcohol abuse.

**13.** Derivatives ot peptides according to claim is possessing ability to present the development and to control the signs of herontological diseases including cataract, changes in cutaneous teguments, senile psychoses. Alzheimer and Parkinson diseases.

**14.** Derivatives of peptides according to claim 1, possessing antibacterial and antiviral activity including activity against HIV infection.

**15.** Derivatives of peptides according to claim 1, possessing antitumor and antimetastatic activity including their combined use with cytostatic agents and radiotherapy.

**16.** Derivatives of peptides according to claim 1 as an adaptogens to overcome stress conditions including a hard physical load.

**17.** A pharmaceutical composition comprising a peptide derivative of general formula I,

$$R_1\text{-}(CH_2)_n\text{-}CONH\text{-}\underset{\displaystyle R_2}{\underset{|}{CH}}\text{-}(CH_2)_m\text{-}R_3 \ (I),$$

or pharmaceutically acceptable salts thereof, where $R_1$ is a hydrogen atom or $C_1$ - $C_3$ - hydrocarbon radical substituted for a functional group selected from amino-, $C_1$ - $C_5$ - amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; or $C_1$ - $C_3$ - hydrocarbon radical substituted for indole residue or 5-6 membered saturated or unsaturated cyclic or heterocyclic group, hydrocarbon radical possibly simultaneously comprising amino group, free or substituted for an acyl substitute or ether of carbonic acid; $R_2$ is hydrogen atom or a functional group selected from carboxyl which can be etherified; $R_3$ is indole or methyl and/or hydroxyl derivative thereof, hydroxyl group possibly being acylated, alcylated or aralcylated; 5-6 membered saturated or unsaturated cyclic or heterocyclic groups comprising oxygen, sulfur and/or 1-3 nitrogen atoms or methyl derivatives thereof; hydrogen atom or $C_1$ - $C_3$ - hydrocarbon radical substituted for a functional group selected from amino-. $C_1$ - $C_5$ - amido-, $C_1$ - $C_7$ -urethano- or carboxylic group, carboxylic group possibly being etherified, and amino group can be substituted for an acyl substitute or ether of carbonic acid: or $C_1$ - $C_3$ - hydrocarbon radical simultaneously substituted for ammo- or carboxylic group, carboxylic group possibly being etherified and amino group can be substituted for an acyl substitute or ether of carbonic acid; n=0-4, m=1-5, in an efficient amount and pharmaceutically acceptable supplements.

**18.** A pharmaceutical composition according to claim 17, possessing antihypoxic, antioxidant, antiradical, lipid regulating, hypoglycemic, antiaggregate, immunomodulating, wound healing, antiallergic, antiasthmatic, antiviral, antibacterial, antitumor, antimetastatic, adaptogenic effects, the ability to induce the hepatic P-450 cytochrome system, to modulate the metabolism of arachidonic acid and adrenocortical hormones, to lower the level and antigen-dependent histamine secretion by mast cells, to modulate the activity of macrophages, natural killers, the interferon system (of cytokines), as well as to prevent abortions and dysfunctional uterine bleedings, the signs of diabetes mellitus, obesety, ischemic heart disease, stress conditions, hepatitis, cirrhosis, toxic liver lesions, alcohol abuse, radiation injuries, herontological changes.

**19.** A cosmetic agent comprising a peptide derivative of general formula I,

$$R_1\text{-}(CH_2)_n\text{-}CONH\text{-}\underset{\displaystyle R_2}{\underset{|}{CH}}\text{-}(CH_2)_m\text{-}R_3 \text{ (I)},$$

or cosmetically acceptable salts thereof, where $R_1$ is a hydrogen atom or $C_1$ - $C_3$ - hydrocarbon radical substituted for a functional group selected from amino-, $C_1$ - $C_5$ - amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; or $C_1$ - $C_3$ - hydrocarbon radical substituted for indole residue or 5-6 membered saturated or unsaturated cyclic or heterocyclic group, hydrocarbon radical possibly simultaneously comprising amino group, free or substituted for an acyl substitute or ether of carbonic acid; $R_2$ is hydrogen atom or a functional group selected from carboxyl which can be etherified; $R_3$ is indole or methyl and/or hydroxyl derivative thereof, hydroxyl group possibly being acylated, alcylated or aralcylated; 5-6 membered saturated or unsaturated cyclic or heterocyclic groups comprising oxygen, sulfur and/or 1-3 nitrogen atoms or methyl derivatives thereof; hydrogen atom or $C_1$ - $C_3$ - hydrocarbon radical substituted for a functional group selected from amino-, $C_1$ - $C_5$ - amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; n=0-4, m=1-5, in an efficient amount and cosmetically acceptable supplements.

**20.** A cosmetic agent according to claim 19, possessing antihypoxic, antioxidant, antiradical, lipid-regulating, hypoglycemic, antiaggregate, immunomodulating, wound healing, antiallergic, antiasthmatic, antiviral, antibacterial, antitumor, antimetastatic, adaptogenic effects, the ability to induce the hepatic P-450 cytochrome system, to modulate the metabolism of arachidonic acid and adrenocortical hormones, to lower the level and antigen-dependent histamine secretion by mast cells, to modulate the activity of macrophages, natural killers, the interferon system (of cytokines), as well as to prevent abortions and dysfunctional uterine bleedings, the signs of diabetes mellitus, obesety, ischemic heart disease, stress conditions, hepatitis, cirrhosis, toxic liver lesions, alcohol abuse, radiation injuries, herontological changes.

**21.** Use of derivatives of peptides of general formula I,

$$R_1\text{-}(CH_2)_n\text{-}CONH\text{-}\underset{\displaystyle R_2}{\underset{|}{CH}}\text{-}(CH_2)_m\text{-}R_3 \text{ (I)},$$

where when $R_1$ = $NH_2$ -, n=2-3, $R_1$ = $CH_3CONH$-CH (COOH)-, n=1, $R_1$ = $CH_3CONH$-CH ($CH_2$ COOH)-, n=0, $R_1$ -CO =pGlu-, n=0, $R_2$ = -H, when $R_1$ = $NH_2$-CH (COOH)-, n=2, $R_2$ = -COOH,

$R_3$= 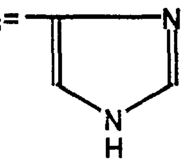 ,

m=1, when $R_1$ = $NH_2$ -, n=2-3, $R_1$ = $NH_2$-CH (COOH)-, n=2, $R_2$ =H,

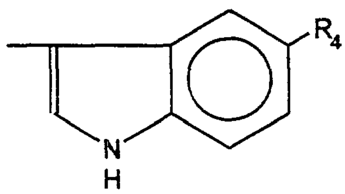 ,

m=1, where $R_4$= -H. -OH, $-OCH_3$, $-OCH_2C_6H_5$ as the agents possessing immunomodulating and antiradical effect (except for the compounds β-alanyihistamine and γ- aminobutyrylhistamine), antihypoxic, in vivo antioxidant, lipid regulating, hypoglycemic, antiinflammatory, antiaggregate effects as well as the ability to induce the P-450 cytochrome system, to modulate the metabolism of arachidonic acid and adrenocortical hormones, to lower the level and antigen-dependent histamine secretion by peritoneal mast cells, to modulate the activity of macrophages, natural killers, the interferon system (cytokines).

**22.** Use of derivatives of peptides according to claim 21 as the agents possessing antiallergic activity (except for the compound γ- L-glutamylhistamine) and the activity related to controlling the signs and preventing asthma and pulmonary emphysema.

**23.** Use of derivatives of peptides according to claim 21 as the agents possessing wound healing properties (except for the compounds β-alanylhistamine and γ-L-glutamylhistamine) and the activity related to controlling the signs of cutaneous lesion, for instance psoriasis, eczema as well as varicose veins.

**24.** Use of derivatives of peptides according to claim 21 as the agents possessing activity related to preventing dysfunctional disorders including imminent abortion, dysfunctional uterine bleedings, amenorrhea etc.

**25.** Use of derivatives of peptides according to claim 21 as the agents possessing antihypoxic and antiatherosclerotic activity as well as activity related to controlling the signs of atherosclerosis, ischemic disease, obesety and diabetes mellitus.

**26.** Use of derivatives of peptides according to claim 21 as the agents possessing hepatoprotective properties and activity related to controlling radiation lesions, hepatic lesions including toxic lesions, hepatitis, cirrhosis and alcohol abuse.

**27.** Use of derivatives of peptides according to claim 21 as the agents possessing ability to prevent the development and to control the signs of herontological diseases including cataract, changes in cutaneous teguments, senile psychoses, Alzheimer and Parkinson diseases.

**28.** Use of derivatives of peptides according to claim 21 as the agents possessing antibacterial and antiviral activity including activity against HIV infection.

**29.** Use of derivatives of peptides according to claim 21 as the agents possessing antitumor and antimetastatic activity including their combined use with cytostatic agents and radiotherapy.

**30.** Derivatives of peptides according to claim 1 as an adaptogens to overcome stress conditions including a hard physical load.

**31.** A method of therapy or prevention of diseases comprising administration to warm-blooded animals or humans in need of such therapy, a peptide derivative of general formula I.

$$R_1\text{-}(CH_2)_n\text{-}CONH\text{-}\underset{\underset{\displaystyle R_2}{|}}{CH}\text{-}(CH_2)_m\text{-}R_3 \text{ (I)},$$

or pharmaceutically acceptable salts thereof, where $R_1$ is a hydrogen atom or $C_1$ - $C_3$ - hydrocarbon radical substituted for a functional group selected from amino-, $C_1$ - $C_5$ - amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; or $C_1$ - $C_3$ - hydrocarbon radical substituted for indole residue or 5-6 membered saturated or unsaturated cyclic or heterocyclic group, hydrocarbon radical possibly simultaneously comprising amino group, free or substituted for an acyl substitute or ether of carbonic acid; $R_2$ is hydrogen atom or a functional group selected from carboxyl which can be etherified; $R_3$ is indole or methyl and/or hydroxyl derivative thereof, hydroxyl group possibly being acylated, alcylated or aralcylated; 5-6 membered saturated or unsaturated cyclic or heterocyclic groups comprising oxygen, sulfur and/or 1-3 nitrogen atoms or methyl derivatives thereof; hydrogen atom or $C_1$ - $C_3$ - hydrocarbon radical substituted for a functional group selected from amino-, $C_1$ - $C_5$ - amido-, $C_1$ - $C_7$ -urethano- or carboxylic group, carboxylic group possibly being etherified, and amino group can be substituted for an acyl substitute or ether of carbonic acid; or $C_1$ - $C_3$ - hydrocarbon radical simultaneously substituted for amino- or carboxylic group, carboxylic group possibly being etherified and amino group can be substituted for an acyl substitute or ether of carbonic acid; n=0-4, m=1-5, in an efficient amount and pharmaceutically acceptable supplements.

**32.** A method of therapy or prevention according to claim 31 in which diseases are selected from the group including: allergic, inflammatory diseases, bronchial asthma, pulmonary emphysema, psoriasis, eczema, varicose veins, dysfunctional disorders, imminent abortion, uterine bleedings, atherosclerosis, ischemic disease, obesety, diabetes mellitus, infectious diseases (viral and bacterial), cancer diseases, hepatitis, hepatic cirrhosis, alcohol abuse, as well as radiation lesions, hepatic lesions including toxic lesions, and herontological changes.

Europäisches Patentamt
European Patent Office
Office européen des brevets

## PARTIAL EUROPEAN SEARCH REPORT

under Rule 62a and/or 63 of the European Patent Convention. This report shall be considered, for the purposes of subsequent proceedings, as the European search report

Application Number
EP 08 00 6502

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SCIORTINO T ET AL: "POTENTIAL ANTI VIRAL AGENTS IX HIGHER ACYL DERIVATIVES OF SYMPATHOMIMETIC AMINES OF BIOLOGICAL SIGNIFICANCE AMPHETAMINE AMINO HEPTANE TYRAMINE TRYPTAMINE"<br>BOLLETTINO CHIMICO FARMACEUTICO, SOCIETA EDITORIALE FARMACEUTICA, MILANO, IT,<br>vol. 107, no. 8,<br>1 January 1968 (1968-01-01), pages 506-511, XP009079217<br>ISSN: 0006-6648<br>* compound with RN 90579-19-4 *<br>-----<br>-/-- | 1 | INV.<br>A61K9/00<br>C07D233/24<br>C07D233/54<br>C07D209/16<br>C07D211/88<br>C07D401/06<br>C07D209/26<br>C07D209/18<br>C07D403/06 |

TECHNICAL FIELDS SEARCHED (IPC)

C07D
A61K
A61P

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

3

| Place of search | Date of completion of the search | Examiner |
|---|---|---|
| Munich | 29 July 2010 | Villa Riva, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

Europäisches Patentamt
European Patent Office
Office européen des brevets

**PARTIAL EUROPEAN SEARCH REPORT**

Application Number
EP 08 00 6502

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | BENG T. HO ET AL.: "Hydroxyindole-o-methyltransferase IV: Inhibitory activities of some N-acryltryptamines and N-crotonyltryptamines" JOURNAL OF PHARMACEUTICAL SCIENCES., vol. 59, no. 4, April 1970 (1970-04), - April 1970 (1970-04) pages 573-574, XP002225150 AMERICAN PHARMACEUTICAL ASSOCIATION. WASHINGTON., US ISSN: 0022-3549 * compound III * ----- | 1 | |
| X | BLASKO G ET AL: "INDOLE ALKALOID BETA CARBOLINE-1-PROPIONIC-ACID" PLANTA MEDICA, THIEME VERLAG, DE, no. 1, 1 January 1986 (1986-01-01), pages 41-43, XP009130889 ISSN: 0032-0943 * compound with RN 92256-33-2 * ----- | 1 | TECHNICAL FIELDS SEARCHED (IPC) |
| X | MITA H ET AL: "AN ATTEMPT TO PRODUCE AN ANTIBODY OF HISTAMINE AND HISTAMINE DERIVATIVES" AGENTS AND ACTIONS, BIRKHAEUSER VERLAG, BASEL, CH, vol. 14, no. 5-6, 1 January 1984 (1984-01-01), pages 574-579, XP009130884 ISSN: 0065-4299 * compound with RN 90579-19-4 * ----- -/-- | 1 | |

3

EPO FORM 1503 03.82 (P04C10)

Europäisches Patentamt
European Patent Office
Office européen des brevets

# PARTIAL EUROPEAN SEARCH REPORT

Application Number
EP 08 00 6502

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DELAAGE M A ET AL: "RADIO IMMUNOASSAYS FOR SEROTONIN AND 5 HYDROXY IAA" JOURNAL DE PHYSIOLOGIE, MASSON, PARIS, FR, vol. 77, no. 2-3, 1 January 1981 (1981-01-01), pages 339-347, XP009130890 ISSN: 0021-7948 * compound with RN 74010-65-4 * | 1 | |
| A | US 4 297 346 A (RICHARD RIPS ET AL) 27 October 1981 (1981-10-27) * column 1 - column 6 * | 1,9 | |
| X | EP 0 412 595 A1 (MERCK & CO INC [US]) 13 February 1991 (1991-02-13) * example 1 , starting product * | 1 | TECHNICAL FIELDS SEARCHED (IPC) |
| X | DE 22 38 545 A1 (SHIONOGI & CO) 14 February 1974 (1974-02-14) * compound histidylalanine * | 1-32 | |
| X | WO 95/12581 A1 (EXSYMOL SA [MC]; BABIZHAYEV MARC [RU]; SEGUIN MARIE CHRISTINE [FR]) 11 May 1995 (1995-05-11) * page 4-page 7 : formulae of the specific pseudopeptides; pages 4-7 * | 1-32 | |
| X | WO 94/25435 A1 (CELLTECH LTD [GB]; MORPHY RICHARD JOHN [GB]; MILLICAN ANDREW THOMAS [G) 10 November 1994 (1994-11-10) * abstract * * formula (I) * | 1-32 | |

3

EPO FORM 1503 03.82 (P04C10)

Europäisches Patentamt
European Patent Office
Office européen des brevets

**INCOMPLETE SEARCH**
**SHEET C**

Application Number
EP 08 00 6502

Claim(s) completely searchable:
12-32

Claim(s) searched incompletely:
1-11

Reason for the limitation of the search:

In the present set of claims, it is almost impossible to determine which "derivatives" of the peptides of formula (I) are intended to be included in the claims, and only some of the compounds themselves are exemplified. In particular the definition of R3 is complicated by so many provisos and exclusions that it is almost impossible to determine what substituents are actually meant to be included into the claimed set of compounds. Moreover, like in the parent application, the initial phase of the search revealed a very large number of documents relevant to the issue of novelty of compounds of general formula I of claim 1.
In summary, it is impossible to determine which parts of the claim 1 may be said to define subject-matter for which protection might legitimately be sought (Article 84 EPC). For these reasons, a meaningful search over the whole breadth of the claims 1-32 is impossible.
Consequently, the search and the search report can only be considered comprehensive for the compounds of the specific examples of the present application and for the general formula (I).

**ANNEX TO THE EUROPEAN SEARCH REPORT ON EUROPEAN PATENT APPLICATION NO.** EP 08 00 6502

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2010

| Patent document cited in search report | Publication date | Patent family member(s) | Publication date |
|---|---|---|---|
| US 4297346 A | 27-10-1981 | CH 628324 A5 | 26-02-1982 |
| | | DE 2755036 A1 | 15-06-1978 |
| | | FR 2373517 A1 | 07-07-1978 |
| | | GB 1592552 A | 08-07-1981 |
| EP 0412595 A1 | 13-02-1991 | CA 2021944 A1 | 29-01-1991 |
| | | JP 3148295 A | 25-06-1991 |
| | | US 5110799 A | 05-05-1992 |
| DE 2238545 A1 | 14-02-1974 | NONE | |
| WO 9512581 A1 | 11-05-1995 | BR 9407971 A | 03-12-1996 |
| | | CA 2174526 A1 | 11-05-1995 |
| | | CN 1136808 A | 27-11-1996 |
| | | CZ 9601155 A3 | 11-12-1996 |
| | | DE 69425153 D1 | 10-08-2000 |
| | | DE 69425153 T2 | 15-03-2001 |
| | | EP 0726895 A1 | 21-08-1996 |
| | | ES 2149951 T3 | 16-11-2000 |
| | | FR 2711990 A1 | 12-05-1995 |
| | | GR 3034543 T3 | 31-01-2001 |
| | | HU 74387 A2 | 30-12-1996 |
| | | JP 3503899 B2 | 08-03-2004 |
| | | JP 9504540 T | 06-05-1997 |
| | | PT 726895 E | 29-12-2000 |
| | | RU 2159775 C2 | 27-11-2000 |
| | | US 6046340 A | 04-04-2000 |
| WO 9425435 A1 | 10-11-1994 | AU 6575494 A | 21-11-1994 |
| | | CA 2139129 A1 | 10-11-1994 |
| | | EP 0648206 A1 | 19-04-1995 |
| | | JP 8500610 T | 23-01-1996 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2701947 A
- US 4568489 A
- WO 9512581 A
- EP 95107298950513 A, Koyama, Masayoshi, Takahashi, Mikiko, Yanagawa, Masayoshi
- JP 94115161940527 B
- US 1488738 B1


### Non-patent literature cited in the description


- **Marone G. ; Columbo N. ; Soppeisa L. et al.** *J. Immunol.,* 1984, vol. 133 (3), 1542-1546
- **Wise J. ; Wojtecka-Lukasik E. ; Maslinski S.** *Agents Actions,* 1986, vol. 18 (1-2), 262-265
- **Duchateau J. ; Bolla K.** *Med. Oncol. and Tumor Pharmacoter.,* 1989, vol. 6 (6), 19-23
- **Muller E. ; Sonnenschein B.** *Naturamed.,* 1989, vol. 4 (4), 34-38
- **Proulx M. ; Dusouich P.** *J. Pharm. Pharmacol.,* 1995, vol. 47 (5), 392-397
- **Barakat M. ; du Souich P.** *J. Pharm. Pharmacol.,* 1996, vol. 48, 906-910
- **Sokolov E.I.** Diabetes mellitus and atherosclerosis. Nauka publishers, 1996, 405 pp
- **Bestervelt L.L. ; Vaz A.D.N. ; Coon M.J.** *Proc. Natl. Acad. Sci. U.S.A.,* 1995, vol. 92 (9), 3764-3768
- **Krzhechkovskaya V.V. ; Meltsev G.Yu. ; Marokko I.N.** *The 4th All-Union Symposium on Medical Enzymology,* 1983, 137
- **Marokko I.N. ; Krzhechkovskaya V.V. ; Malikova N.A. ; Izotov M.V. ; Benediktova S.A. ; Spiridonova S.M.** *Bulletin of Experimental Biology and Medicine,* 1991, vol. 8, 200-202
- **Marokko I.N. ; Krzhechkovskaya V.V. ; Malikova N.A. ; Izotov M.V. ; Benediktova S.A. ; Spiridonova S.M.** *Bulletin of Experimental Biology and Medicine,* 1991, (8), 200-202
- **Fornhem C. ; Kumlin M. ; Lundberg J.M. ; Alving K.** *Eur. Resp. J.,* 1995, vol. 8 (7), 1100-1109
- **Fornhem C. ; Lundberg J.M. ; Alving K.** *Eur. Resp. J.,* 1995, vol. 8 (6), 928-937
- **Marokko I.N. ; Krzhechkovskaya V.V. ; Malikova N.A.** Proceedings of the All-Union Conference ''P-450 Cytochrome and Modification of Macromolecules''. *Yalta,* 1989, 339
- **Macharadze D.Sh ; Marokko I.N. ; Balabolkin I.I. ; Yukhtina N.V. ; Malikova N.A.** *Pediatry,* 1994, 9-12
- **Knickle L.C. ; Bend J.R.** *J. Moll. Pharmacol,* 1994, vol. 45 (6), 1273-1280
- **Quiroga J. ; Prieto J.** *Phamacol. Therap.,* 1993, vol. 58 (1), 67-91
- **Ma Y.H. ; Gebremedich D. ; Schwartzman M.L. et al.** *Circulation Research,* 1993, vol. 72 (1), 126-136
- **Quiroga J. ; Prieto J.** *Pharmacol. Therap.,* 1993, vol. 58 (1), 67-91
- **Caroll M.A. ; Balazy M. ; Margiotta P. ; Falck J.R. ; Mcgiff J.C.** *J. Biol. Chem.,* 1993, vol. 268 (17), 12260-12266
- **Sakairi Y. ; Jacobson H.R. ; Noland T.D. ; Capdevilla J.H. ; Falck J.R. ; Breyer M.D.** *Amer. J. Physiol-Renal. Fl. Elect.,* 1995, vol. 37 (5), F931-F939
- **Oyekan A.O.** *Eur. J. Pharmacol.,* 1995, vol. 277 (2-3), 123-132
- **Sakairi Y. ; Jacobson H.R. ; Noland T.D. ; Capdevila J.H. ; Falck J.R. ; Breyer M.D.** *Amer. J. Physiol-Renal. Fl. Elect.,* 1995, vol. 37 (5), F931-F939
- Mediators: Release and Functions. **Parker Ch.V.** Immunology. Mir publishers, 1989, vol. 3, 170-247
- **Chakravarty N.K.** The mast cell: Its role in health and disease. 1979, 38-46
- **Fesus L. ; Szucs E. ; Barrett K. et al.** *J. Biol. C. Chem.,* 1995, 13771-13778
- **Imaoka S. ; Sugiyama T. ; Taniguchi N. ; Funae Y.** *Carcinogenesis,* 1993, vol. 14 (1), 117-121
- **Debinski H.S. ; Lee C.S. ; Danks J.A. ; Mackenzie P.I. ; Desmond P.V.** *Gastroenterology,* 1995, vol. 108 (5), 1464-1469
- **Kapil A. ; Koul I.B. ; Suri O.P.** *Phytother. Res.,* 1995, vol. 9 (3), 189-193
- **Wolfgang GHI ; Robertson DG ; Welty DF ; Metz AL.** *Fund. Appl. Toxicol.,* 1995, vol. 26 (2), 272-281
- **Remaley A.T. ; Schumacher U.K. ; Amouzadeh H.R. ; Brever H.B. ; Hoeg J.M.** *J. Lipid Res.,* 1995, vol. 36 (2), 308-314

- **Stegeman J.J. ; Hahn M.E. ; Weisbrod R. ; Woodin B.R. ; Joy J.S. ; Najibi S. ; Cohen R.A.** *Mol. Pharmacol.,* vol. 47 (2), 296-306
- **Wade A. ; Harred W.** *Feder. Proct.,* 1976, vol. 55, 2475-2479
- **Buters J.T.M. ; Zysset T. ; Reichen J.** *Biochem. Pharmacol.,* 1993, vol. 46 (6), 983-991
- **Reichen J. ; Buters J.T.M. ; Sojcic Z. ; Roos F.J.** *Experentia,* 1992, vol. 48 (5), 482-486
- **Tatony Ya.N.** Obesety. Pathophysiology, diagnostics, therapy. Polish Medical publishers, 1981, 364
- **Shimojo N. ; Ishizaki T., Imaoka S. ; Funae Y. ; Fujii S. ; Okuda K.** *Biochem. Pharmacol.,* 1993, vol. 46 (4), 621-627
- **Sokolov E.I.** Diabetes mellitus and atherosclerosis. Nauka publishers, 1996, 450
- Immunology. Mir publishers, 1989, vol. 3, 202-228
- **Paltsev M.A. ; Ivanov A.A.** Intercellular ineractions. Meditsina publishers, 1995, 224
- **Mayansky A.N. ; Pikuza O.I.** Clinical aspects of phagocytosis. MAGARIF publishers, 1993, 192
- **Henderson W.R. ; Jorg A. ; Klebanoff S.J.** *J. Immunol.,* 1982, vol. 128 (6), 2609-2613
- **Sokolov E.I.** Diabetes mellitus and atherosclerosis. Nauka publishers, 1996, 405
- **Burov Yu.V. ; Syrkin A.B. ; Kinzirsky A.S. ; Koroleva A.M.** Chemical-pharmaceutical production. *Review information,* 1992, vol. 11, 42
- **Evstigneeva R.P. ; Zheltukhina G.A. ; Ogrel' S.A. ; Nebol'sin V.E.** Synthesis of pseudopeptides based on biogenic amines. *Reports of the Russian Sciences Academy,* 1995, vol. 345 (4), 493-495
- **Mc Caman M.W. ; Stetzler J. ; Clark B.** Synthesis of γ-Glutamyl dopamine and Other Peptidoamines in the Nervous System of Aplysia californica. *J. Neurochem.,* 1985, vol. 45 (6), 1828-1835
- **Evstigneeva R.P. ; Zheltukhina G.A. ; Ageeva E.A. ; Babizhaev M.A.** Lipoperoxidase activity of carnozine and carcinine. *Reports of the Academy of Sciences of the USSR,* 1993, vol. 333 (1), 104-106
- **Schreder E. ; Lubke K.** Peptides. Mir publishers, 1967, 249
- **Evstigneeva R.P. ; Zheltukhina G.A. ; Ogrel' S.A. ; Nebol'sin V.E.** Synthesis of pseudopeptides based on biogenic amines. *Reports of the Academy of Sciences of the USSR,* 1995, vol. 345 (4), 493-495
- **Konishi H. ; Kakimoto Y.** Formation of γ-Glutamylhistamine from histamine in rat brain. *J.Neurochem.,* 1976, vol. 27, 1461-1463
- **Mc Caman M.W. ; Stetzler J. ; Clark B.** Synthesis of γ-Glutamyldopamine and Other Peptidoamines in the Nervous System of Aplysia california. *J. Neurochem.,* 1985, vol. 45 (6), 1828-1835
- **Parker K.L. ; Schimmer B.P.** The Role of Nuclear Receptors in Steroid-Hormones Production. *Seminars in Cancer Biology,* 1994, (5), 317-325
- **Emanuel N.M. ; Obukhova L.K. ; Hajdich V.I. ; Murza L.I. ; Bunto T.V.** Aging Inhibition through Activation of the System of Microsomal Oxidases with Phenobarbital. *Reports of the USSR Academy of,* 1977, vol. 235 (4), 957-960
- **Honn K.V. ; Tang D.G. ; Gao X. ; Butovich I.A. ; Liu B. ; Timar J. ; Hagmann W.** *Cancer and Metastasis Reviews,* 1994, vol. 13 (3-4), 265-396
- **Gubler E.V.** Computer analysis methods and recognition of pathological processes. Nauka publishers, 1978, 365
- **Sernov L.N. ; Gatsura V.V.** A differential indicator method for determinig ischemic and necrotic zone in experimental myocardial infarction in rats. *Bulletin of Experimental Biology and Medicine,* 1989, 534-535
- **Shaternikov V.A. ; Marokko I.N. ; Pyatnitsky N.N. ; Shirina L.I. ; Gorgoshidze L.Sh. ; Kasyanenko V.V. ; Sugonyaeva N.P. ; Zhminchenko V.M. ; Vinokurova N.M.** Experimental reproduction of food anaphylaxis. *Voprosy pitaniya,* 1982, 27-31
- **Weigle W. ; Cochrane C. ; Dixon F.** Anaphylactogenic properties of soluble antigen-antibody complexes in the guinea pigs and rabbits. *J. Immunology,* 1969, vol. 85, 469-477
- **Gubler E.V.** Computer methods of analysis and recognition of pathological processes. Nauka publishers, 1978, 365
- **Andersson P.** Antigen induced bronchial anaphylaxis in actively sensitized guinea pig. *Allergy,* 1980, vol. 35, 65-71
- **Yu-Hong L. ; Barnes P. ; Rogers D.** Inhibition of neurogenic plasma exudation and bronchoconstriction by a K+ channel activator, BRL 38227, in guinea pig airways in vivo. Europ. *J. Pharmacol.,* vol. 239, 257-259
- **Tarayre J.P. ; Barbara M. ; Aliaga M.** Tisne-Versilles. Comparative actions of immunosuppressants, glucocorticoids and non-steroidal antiinflammatory drugs on various models of delayed hypersensitivity and on a non-immune inflammation in mice. *Arzneim.-Forsch. Drug Res.,* 1990, vol. 40, 1125-1131
- **Ezamuzie Ch. ; Umezurike C.C.** Effect of histamine H2-receptor antagonists on acute inflammatory of the rat paw oedema. *J. Pharmacol.,* 1989, vol. 41, 261-265

- **De Rosa M. ; Giroud J.P. ; Willoughby D.A.** Studies of the mediators of the acute inflammatory reponse induced in rats in different sites by carrageenan and turpentine. *J. Pharmacol.,* 1971, vol. 104, 15-29
- **Makarova O.V. ; Kovaleva V.L. ; Sladkopevtsev A.S. ; Mikhailova L.P. ; Noseikina E.M.** *Experimental model of non-infectious pulmonary granulomatosis,* 1996, 76-79
- **Vengerovsky A.I. ; Chuchalin V.S. ; Pauls O.V. ; Saratikov A.S.** Effect of hepatoprotectors on lipid metabolism in hepatitis induced by Ccl. *Bulletin of Experimental Biology,* 1987, 430-432
- **Volchegorsky I.A. ; Nalimov A.G. ; Yarovinsky B.G. ; Lifshits R.I.** Comparison of different approaches to determining lipid peroxidation products in heptane-isopropanolic blood extracts. *Voprosy Meditsinskoy Chimii,* 1989, 127-131
- **Korobeinikova E.N.** Modification of lipid peroxidation products in the reaction with thiobarbituric acid. *Laboratornoye delo,* 1989, 8-10
- The method of determining malonic dialdehyde using thiobarbituric acid. **Stalnaya I.D. ; Garishvili T.G.** Modem methods in biochemistry. Meditsina publishers, 1977, 66-69
- **Cates M.** Technology of Lipidology. Mir publishers, 1975, 74-76
- **Omura T. ; Scato R.** The monooxide binding pigment of liver microsomes. 11. Solubilization, purification and properties. *J. Chem.,* 1964, vol. 239, 2379-2385
- **Ahokas J. ; Pelkonen O. ; Karkin N.** Charcaterisation of BP-hydroxylase of Trout-liver. *Cancer Res.,* 1977, vol. 37, 3737-3743
- **Hartree E.** Determination of protein: a modification of the Lowry method, that gives a linear photometric responces. *Ann. Biochem.,* 1972, vol. 48, 422-427
- **Guschin I.S. ; Voitenko V.G. ; Sviridov B.D. et al.** A polyfunctional molecule produced by the conjugation of synthetic polyionimmunostimulant with specific antigen and an inhibitor of mast cell activation. *Effects on histamine release. Agents and Actions,* 1989, vol. 27, 75-78
- **Fredholm B.B. ; Gyschin I.S. ; Elwin K. et al.** Cyclic AMP-independent inhibition by papaverine of histamine release induced by compound 48/80. *Biochem. Pharmacol.,* 1976, vol. 25, 1583-1588
- **Short P.A. ; Burkhalter A. ; Cohn V.N.** A method for fluorimetric assay of histamine in tissues. *J. Pharmacol. Exper. Ther.,* 1959, vol. 127, 182-186
- **Halliwell B.** Superoxide-dependent formation of hydroxyl radicals in the presence of iron salts. *FEBS Lett.,* 1978, vol. 96, 238-241
- **Afanas'ev I. ; Suslova T. ; Cheremisina Z. et al.** Study of antioxidant properties of metal aspartates. *Analyst,* 1995, vol. 120, 859-862
- Laboratory examination methods in the clinic. Meditsina publishers, 1969, 302
- Biochemical examination in the clinic. Meditsina publishers, 1969, 300-302
- **Titov V.N. ; Brener E.D. ; Khaltaiev N.G. ; Zadoya A.A. ; Tvorogova M.G.** A method and diagnostic significance of cholesterol level study in α-lipoproteins. *Laboratomoye delo,* 1979, 36-41
- Biochemical examination in the clinic. Meditsina publishers, 1969, 300-302
- **Friedwald W.T. ; Levy R.I.** Fredrickson D.S. Estimation of the concentration of low-density lipoprotein cholesterol in plasma, without use of preparative ultracentrifuge. *Clin. Chem.,* 1972, vol. 18, 499-502
- **Rodionova L.P.** Modification of blood serum trigyceride level determination method. *Laboratomoye delo,* 1980, 297-299
- **Korobeinikova E.N.** Modification of lipid peroxydation product determination in reaction with thiobarbituric acid. *Laboratomoye delo,* 1989, 8-10
- Malonic dialdehyde determination method using thiobarbituric acid. **Stalnaya I.D. ; Garishvili T.G.** Modem methods in biochemistry. Meditsina publishers, 1977, 66-69
- **Arichi H. ; Kumura H.O.** Effects of stibens compounds of roots of polygonum cuspidatium on the lipid metabolism. *Chem Pharm. Bull.,* 1982, vol. 30 (5), 1766-1767
- **Titov V.N. ; Brener E.D. ; Khaltaiev N.G. ; Zadoya A.A.** Tvorogova M.G. A method and diagnostic significance of cholesterol level study in α-lipoproteins. *Laboratomoye delo,* 1979, 36-41
- **Friedwald W.T. ; Levy K.J. ; Leus R.** Fat transport in lipoproteins: an integrated approach to mechanism and disorders. *New Engl. J. Med.,* 1967, vol. 276, 32
- **Klimov A.N. ; Nikulcheva N.G.** *Lipoproteins, dylipoproteinemias and atherosclerosis,* 1984, 165
- **Friedwald W.T. ; Levy R.I. ; Fredrickson D.S.** Estimation of concentration of low-density lipoprotein cholesterol in plasma, without use of preparative ultracentrifuge. *Clin. Chem.,* 1972, vol. 18, 499-502
- Experimental estrimation of antitumor drugs in the U.S.S.R. and the U.S.A. Meditsina publishers, 1980, 296
- *Methodological recommendations on preclinical study of agents possessing ability to inhibit metastasizing process and to enhance efficacy of cytostatic therapy of malignant tumors,* 1992, 13
- **Lazareva D.L. ; Alekhin E.K.** *Immunity stimulants,* 1985, 286
- **Klimov V.V. ; Kolovkina T.V.** Nitroblue tetrasolium reduction test stimulated with pyrogenal. *Laborotornoye delo,* 1982, 624-625

- **Sukhikh T.G. ; Nossik N.N. ; Parshina O.V. ; Van'ko L.V. ; Meerson F.Z. ; Yershov F.I.** Relationship between the natural cellular cytotoxicity system and the interferon system in immobilization stress. *Bulletin of Experimental Biology,* 1984, 593-595
- **Nossik N.N. ; Bopegamage S.A. ; Yershov F.I.** Properties of interferons produced by different cell populations. *Acta microbiologica Hungarica,* 1988, vol. 35 (4), 397-403
- **Nossik N.N. ; Bopegamage S.A. ; Yershov F.I.** Properties of interferons produced by different cell populations. *Acta microbiologica Hungarica,* vol. 35 (4), 397-403
- **Kiseleva E.P. ; Tsveibakh A.S. ; Goldman E.I. ; Pigareva N.V.** Application of a micromethod to study blasttransformation of lymphocytes in humans and animals. *Immunology,* 1985, 76-78